# EUROPÄISCHE PATENTSCHRIFT

(11) **EP 1 497 269 B1**
(45) Veröffentlichungstag und Bekanntmachung des Hinweises auf die Patenterteilung: **23.04.2008**
(21) Anmeldenummer: 03720434.4
(22) Anmeldetag: 08.04.2003
(51) Int. Cl.: C07D 237/22, C07D 401/04, C07D 401/12, C07D 403/04, A61K 31/501, A61P 1/16

(54) **SUBSTITUIERTE 2-PHENYL-3(2H)-PYRIDAZINONE**
SUBSTITUTED 2-PHENYL-3(2H)-PYRIDAZINONES
2-PHENYL-3(2H)-PYRIDAZINONES SUBSTITUEES

(30) Priorität: 12.04.2002 DE 10216144
(43) Veröffentlichungstag der Anmeldung: 19.01.2005
(73) Patentinhaber: Bayer HealthCare AG, 51368 Leverkusen (DE)
(72) Erfinder: SCHOHE-LOOP, Rudolf, 42327 Wuppertal (DE); BURCHARDT, Elmar, 42113 Wuppertal (DE); FAESTE, Christiane, N-0270 Oslo (NO); HIRTH-DIETRICH, Claudia, 42115 Wuppertal (DE); KELDENICH, Jörg, 42113 Wuppertal (DE); KNORR, Andreas, 40699 Erkrath (DE); LAMPE, Thomas, 40223 Düsseldorf (DE); NAAB, Paul, 42287 Wuppertal (DE); SCHMIDT, Delf, 42213 Wuppertal (DE); SCHMIDT, Gunter, 42115 Wuppertal (DE)
(86) Internationale Anmeldenummer: PCT/EP2003/003628
(87) Internationale Veröffentlichungsnummer: WO 2003/097612

(56) Entgegenhaltungen:
- PATENT ABSTRACTS OF JAPAN vol. 014, no. 485 (C-0772), 23. Oktober 1990 (1990-10-23) & JP 02 200634 A (MORISHITA PHARMACEUT CO LTD), 8. August 1990 (1990-08-08) in der Anmeldung erwähnt

## Beschreibung

Die Erfindung betrifft neue substituierte 2-Phenyl-3(2H)-Pyridazinon-Derivate, Verfahren zu ihrer Herstellung und ihre Verwendung zur Herstellung von Arzneimitteln, insbesondere zur Bekämpfung fibrotischer Erkrank-ungen.

Lysyloxidase ist eine Kupfer-abhängige Aminoxidase (EC 1.4.3.13), durch die Peptidyllysin-Reste in Collagen- und Elastinmolekülen oxidativ desaminiert werden. [H.M. Kagan, Lysyloxidase: Mechanism, regulation and relationship to liver fibrosis, Path. Res. Pract. 190, 910-919 (1994)]. Dies hat die Ausbildung von stabilen kovalenten Verknüpfungen von Tropocollagen oder Tropoelastin zur Folge, die die Zusammenlagerung von Tropocollagen zu stabilen Collagenfasern ermöglicht.

Die Lysyloxidase spielt bei Erkrankungen, bei denen es zu einer verstärkten Collagenablagerung im interstitiellen Raum kommt, eine Schlüsselrolle. Die Aktivität der Lysyloxidase ist bei Patienten mit verstärkter interstitieller Collagenablagerung gegenüber einem gesunden Normalkollektiv mehrfach erhöht [R.C. Siegel, K.H. Chen, J.S. Greenspan, J.M. Aguiar, Biochemical and immunochemical study of lysyl oxidase experimental hepatic fibrosis in the rat, Proc. Natl. Acad. Sci. USA 75, 2945-2949 (1978); A. Konishi, H. Iguchi, J. Ochi, R. Kinoshita, K. Miura, H. Uchino, Increased lysyl oxidase in culture-medium of non-parenchymal cells from fibrotic livers, Gastroenterol. 89, 709-715 (1985)]. Ein Anstieg der Lysyloxidase-Konzentration im Serum von solchen Patienten läßt sich messen [Y. Murawaki, Y. Kusakabe, C. Hirayama, Serum lysyl oxidase activity in chronic liver disease in comparison with serum levels of prolyl hydroxylase and laminin, Hepatol. 14, 1167-1173 (1991)]. Des weiteren konnte in Tiermodellen [S. Ricard-Blum, G. Ville, J.A. Grimaud, Hepatic pyridoline level in murine schistosomiasis, in: Molecular and cell biology of liver fibrogenesis, edited by A.M. Gressner and G. Ramadori, Kluwer Academic Publishers, Dordrecht, 1992] und in Patienten [S. Ricard-Blum, S. Bresson-Hadni, D.A. Vuitton, G. Ville, J.A. Grimaud, Hydroxypyridinium collagen cross-links in human liver fibrosis: a study of alveolar echinococcosis, Hepatol. 15, 599-602 (1992); A. Hayasaka, S. Iida, N. Suzuki, F. Kondo, M. Miyazaki, H. Yonemitsu, Pyridinolone collagen cross-links in patients with chronic viral hepatitis and cirrhosis, J. Hepatol. 24, 692-698 (1996)] gezeigt werden, dass Reaktionsprodukte der Lysyloxidase, die Dipyridinium-crosslinks, in fibrotischen Geweben in stark erhöhten Konzentrationen nachweisbar sind. Es wurde gezeigt, dass die Abbaubarkeit von abgelagertem Collagen vom Grad der Collagen-Quervernetzung abhängt: Weniger stark quervernetztes Collagen wird schneller von Collagenase abgebaut als hochgradig quervernetztes Collagen [C.A. Vater, E.D. Harris, R.C. Siegel, Native cross-links in collagen fibrils induce resistance to human human synovial collagenase, Biochem. J. 181, 639-645 (1979)].

Der Lysyloxidase kommt somit bei der Ausbildung von pathologischen Collagen-Ablagerungen eine Schlüsselrolle durch Verminderung der Abbaubarkeit von Collagenfasern zu. Die Inhibition der Lysyloxidase-Aktivität führt somit zu einem verstärkten Collagenabbau, so dass der typische fibrotische Gewebeumbau durch Inhibitoren der Lysyloxidase verhindert werden kann.

Aufgabe der vorliegenden Erfindung ist die Bereitstellung von Arzneimitteln für die Prophylaxe und/oder Behandlung von fibrotischen Erkrankungen.

Die Aufgabe der vorliegenden Erfindung wird durch Verbindungen der Formel (I) gelöst, die als Lysyloxidase-Inhibitoren wirken.

Strukturell ähnliche Verbindungen sind in anderen Indikationen bzw. für andere Wirkmechanismen bekannt. So ist 4-Ethoxy-2-methyl-5-morpholino-3(2H)-Pyridazinon als nicht-steroidales Analgetikum und als antiinflammatorischer Wirkstoff beschrieben [J. Med. Chem. 22, 53 (1979); Jpn. Kokai Tokkyo Koho JP 02200634]. Von 5-(4-Methylsulfonylphenyl)pyridazinonen ist eine Wirkung als selektive COX-2-Inhibitoren beschrieben; sie sind zur Bekämpfung inflammatorischer oder Cyclooxygenase-mediierter Prozesse wie Asthma oder Arthritis geeignet (WO-A-98/41511).

Gegenstand der vorliegenden Erfindung sind Verbindungen Formel (I) 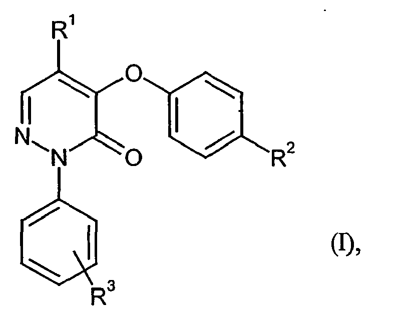 in welcher
- R¹: für 5- bis 7-gliedriges, gesättigtes oder partiell ungesättigtes, über ein Ring-Stickstoffatom verknüpftes Heterocyclyl mit gegebenenfalls einem weiteren Heteroatom oder Heterokettenglied aus der Reihe N, O, S, SO oder SO₂ steht, das ein- oder zweifach, gleich oder verschieden, durch Substituenten ausgewählt aus der Gruppe Halogen, (C₁-C₆)-Alkyl, (C₂-C₆)-Allcenyl, (C₃-C₈)-Cycloalkyl, Hydroxy, Oxo, Carboxyl, (C₁-C₆)-Alkoxycarbonyl, (C₁-C₆)-Alkanoyl, (C₃-C₈)-Cycloalkylcarbonyl, (C₁-C₆)-Alkylsulfonyl, Aminocarbonyl, 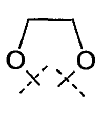 und (C₁-C₆)-Alkylaminocarbonyl substituiert sein kann,
wobei (C₁-C₆)-Alkyl und (C₁-C₆)-Alkanoyl ihrerseits jeweils durch Halogen, Hydroxy, (C₁-C₄)-Alkoxy, (C₁-C₄)-Alkoxycarbonyl, Amino, Mono- oder Di-(C₁-C₄)-alkzylamino, (C₁-C₄)-Alkoxycarbonylamino oder 5- oder 6-gliedriges Heterocyclyl mit bis zu zwei Heteroatomen aus der Reihe N, O und/oder S substituiert sein können,
oder
- R¹: für 5-gliedriges, über ein Ring-Stickstoffatom verknüpftes Heteroaryl mit bis zu zwei weiteren Ring-Stickstoffatomen steht, das ein- bis dreifach, gleich oder verschieden, durch Halogen, (C₁-C₆)-Alkoxycarbonyl oder (C₁-C₆)-Alkyl, welches seinerseits gegebenenfalls durch Hydroxy oder Halogen substituiert ist, substituiert sein kann,
- R²: für (C₆-C₁₀)-Aryl steht, das ein- oder zweifach, gleich oder verschieden, durch Substituenten ausgewählt aus der Gruppe Halogen, Nitro, Cyano, (C₁-C₆)-Alkyl, Trifluormethyl, (C₁-C₆)-Alkanoyl, (C₁-C₆)-Alkoxy, Hydroxy, (C₁-C₆)-Acyloxy, Amino, (C₁-C₆)-Acylamino, Mono- und Di-[(C₁-C₆)-alkylsulfonyl]-amino substituiert sein kann,
wobei (C₁-C₆)-Alkyl und (C₁-C₆)-Alkoxy ihrerseits jeweils durch Hydroxy, Amino, (C₁-C₄)-Alkoxy oder (C₁-C₄)-Acylamino substituiert sein können,
oder
- R²: für 5- oder 6-gliedriges Heteroaryl mit bis zu zwei Ring-Stickstoffatomen steht, das durch Amino, Hydroxy, Halogen, (C₁-C₆)-Alkyl oder (C₁-C₆)-Alkoxy substituiert sein kann,
und
- R³: für Wasserstoff, Halogen, (C₁-C₆)-Alkyl, Trifluormethyl, Nitro, Cyano, Carboxyl oder (C₁-C₆)-Alkoxycarbonyl steht.

Die erfindungsgemäßen Verbindungen können auch in Form ihrer Salze, Solvate oder Solvate der Salze vorliegen.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methyhnorpholin, Dihydroabiethylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Reste, soweit nicht anders spezifiziert, die folgende Bedeutung:
(C₁ -C₆)-Alkanoyl steht für einen geradkettigen oder verzweigten Alkanoyl-Rest mit 1 bis 6 Kohlenstoffatomen. Beispielhaft und vorzugsweise seien genannt: Formyl, Acetyl, Propanoyl, Butanoyl, Isobutanoyl, Pentanoyl, Isopentanoyl und Hexanoyl. Bevorzugt ist ein geradkettiger oder verzweigter Alkanoylrest mit 1 bis 4 Kohlenstoffatomen. Besonders bevorzugt sind Acetyl und Propanoyl.
C₁-C₆)- und (C₁-C₄)-Alkyl stehen für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4, besonders bevorzugt mit 1 bis 3 Kohlenstoffatomen. Beispielhaft und vorzugsweise seien genannt: Methyl, Ethyl, n-Propyl, Isopropyl, tert.-Butyl, n-Pentyl und n-Hexyl.
(C₂-C₆) - und (C₂-C₄)-Alkenyl stehen für einen geradkettigen oder verzweigten Alkenylrest mit 2 bis 6 bzw. 2 bis 4 Kohlenstoffatomen. Bevorzugt ist ein geradkettiger oder verzweigter Alkenylrest mit 2 bis 4, besonders bevorzugt mit 2 bis 3 Kohlenstoffatomen. Beispielhaft und vorzugsweise seien genannt: Vinyl, Allyl, n-Prop-1-en-1-yl und n-But-2-en-1-yl.
(C₁ -C₆)- und (C₁-C₄)-Alkoxy stehen für einen geradkettigen oder verzweigten AlkoxyRest mit 1 bis 6 bzw. 1 bis 4 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielhaft und vorzugsweise seien genannt: Methoxy, Ethoxy, n-Propoxy, Isopropoxy, tert.-Butoxy, n-Pentoxy und n-Hexoxy.
Mono-(C₁-C₆)- und Mono-(C₁-C₄)-alkylamino stehen für einen geradkettigen oder verzweigten Monoalkylamino-Rest mit 1 bis 6 bzw. 1 bis 4 Kohlenstoffatomen. Bevorzugt ist ein geradkettiger oder verzweigter Alkylaminorest mit 1 bis 4, besonders bevorzugt mit 1 bis 3 Kohlenstoffatomen. Beispielhaft und vorzugsweise seien genannt: Methylamino, Ethylamino, n-Propylamino, Isopropylamino, ten.-Butylamino, n-Pentylamino und n-Hexylamino.
Di-(C₁-C₆)- und Di-(C₁-C₄)-alkylamino steht für einen geradkettigen oder verzweigten Dialkylamino-Rest, wobei die Alkylreste gleich oder verschieden sein können und jeweils 1 bis 6 bzw. 1 bis 4 Kohlenstoffatome enthalten. Bevorzugt ist ein geradkettiger oder verzweigter Dialkylaminorest, in dem die Alkylreste jeweils 1 bis 4, besonders bevorzugt 1 bis 3 Kohlenstoffatome enthalten. Beispielhaft und vorzugsweise seien genannt: Dimethylamino, Diethylamino, Di-n-propylamino, Diisopropylamino, Di-tert.-butylamino, Di-n-pentylamino, Di-n-hexylamino, Ethyhnethylamino, Isopropyhnethylamino, n-Butylethylamino, tert.-Butylmethylamino, n-Hexyl-isopentylamino.
(C₁ -C₆)- und (C₁ -C₄)-Alkoxycarbonyl steht für einen geradkettigen oder verzweigten Alkoxycarbonyl-Rest mit 1 bis 6 bzw. 1 bis 4 Kohlenstoffatomen in der AlkoxyGruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
(C₁-C₆)- und (C₁ -C₄)-Alkylsulfonyl steht für einen geradkettigen oder verzweigten Alkylsulfonyl-Rest mit 1 bis 6 bzw. 1 bis 4 Kohlenstoffatomen in der Alkylgruppe. Beispielhaft und vorzugsweise seien genannt: Methylsulfonyl, Ethylsulfonyl, n-Propylsulfonyl, Isopropylsulfonyl und tert.-Butylsulfonyl.
(C₁-C₆)-Apyloxy steht für einen geradkettigen oder verzweigten Alkyl-Rest mit 1 bis 6 Kohlenstoffatomen, der in der 1-Position ein doppelt gebundenes Sauerstoffatom trägt und in der 1-Position über ein weiteres Sauerstoffatom verknüpft ist. Beispielhaft und vorzugsweise seien genannt: Acetoxy, Propionoxy, n-Butyroxy, i-Butyroxy, Pivaloyloxy, n-Hexanoyloxy.
(C₁-C₆)- und (C₁-C₄)-Acylamino steht für eine Amino-Gruppe mit einem geradkettigen oder verzweigten Alkanoyl-Substituenten, der 1 bis 6 bzw. 1 bis 4 Kohlenstoffatome aufweist und über die Carbonylgruppe verknüpft ist. Beispielhaft und vorzugsweise seien genannt: Formamido, Acetamido, Propionamido, n-Butyramido und Pivaloylamido.
(C₁-C₆)-Alkylaminocarbonyl steht für eine Aminogruppe, die über eine Carbonylgruppe verknüpft ist und die einen geradkettigen oder verzweigten Alkylsubstituenten mit 1 bis 6 Kohlenstoffatomen aufweist. Bevorzugt ist ein Alkylaminocarbonyl-Rest mit 1 bis 4 Kohlenstoffatomen. Beispielhaft und vorzugsweise seien genannt: Methylaminocarbonyl, Ethylaminocarbonyl, n-Propylaminocarbonyl, Isopropylaminocarbonyl und t-Butylaminocarbonyl.
(C₁-C₄)-Alkoxycarbonylamino steht für eine Amino-Gruppe mit einem geradkettigen oder verzweigten Alkoxycarbonyl-Substituenten, der im Alkoxyrest 1 bis 4 Kohlenstoffatome aufweist und über die Carbonylgruppe mit der Amino-Gruppe verknüpft ist. Beispielhaft und vorzugsweise seien genannt: Methoxycarbonylamino, Ethoxycarbonylamino, n-Propoxycarbonylamino, Isopropoxycarbonylamino und t-Butoxycarbonylamino.
Mono- und Di-[(C₁-C₆)-alkylsulfonyl]amino steht für eine Amino-Gruppe mit einem bzw. zwei gleichen oder verschiedenen, geradkettigen oder verzweigten Alkylsulfonyl-Substituenten mit jeweils 1 bis 6 Kohlenstoffatomen in der Alkylgruppe. Beispielhaft und vorzugsweise seien genannt: Methylsulfonylamino, Bis-(methylsulfonyl)amino, Ethylsulfonylamino, n-Propylsulfonylamino, Isopropylsulfonylamino und tert.-Butylsulfonylamino.
(C₆-C₁₀)-Aryl steht für einen aromatischen Rest mit 6 bis 10 Kohlenstoffatomen. Bevorzugte Arylreste sind Phenyl und Naphthyl.
(C₃-C₈)-Cycloalkyl steht für eine Cycloalkylgruppe mit 3 bis 8 Kohlenstoffatomen. Bevorzugt ist eine Cycloalkylgruppe mit 3 bis 6 Kohlenstoffatomen. Beispielhaft und vorzugsweise seien genannt: Cyclopropyl, Cyclobutyl, Cyclopentyl, Cyclohexyl, Cycloheptyl und Cyclooctyl. Besonders bevorzugt sind Cyclopropyl, Cyclopentyl und Cyclohexyl.
(C₃-C₈)-Cycloalkylcarbonyl steht für eine wie oben definierte Cycloalkylgruppe, die über eine Carbonylgruppe verknüpft ist. Bevorzugt ist Cyclopropylcarbonyl.
5- oder 6-gliedriges Heteroaryl steht für einen heteroaromatischen Rest mit 1 bis 3 bzw. 1 bis 2 Ring-Stickstoffatomen, der über ein Ring-Kohlenstoffatom oder gegebenenfalls über ein Ring-Stickstoffatom gebunden ist. Beispielhaft und vorzugsweise seien genannt: Pyrrolyl, Imidazolyl, Pyrazolyl, Triazolyl, Pyridyl, Pyrimidinyl und Pyridazinyl.
5- oder 6-gliedriges Heterocyclyl mit bis zu zwei Heteroatomen aus der Reihe N, O und/oder S steht für einen gesättigten Heterocyclus. Beispielhaft und vorzugsweise seien genannt: Pyrrolidinyl, Piperazinyl, Morpholinyl, Thiomorpholinyl und Piperidinyl. Bevorzugt ist Piperazinyl.
5- bis 7-gliedriges, gesättigtes oder partiell ungesättigtes, über ein Ring-Stickstoffatom verknüpftes Heterocyclyl steht für einen nicht-aromatischen Heterocyclus, der neben dem Stickstoffatom ein weiteres Heteroatom aus der Reihe N, O, S, SO oder SO₂ und gegebenenfalls eine oder zwei Doppelbindungen enthalten kann. Bevorzugt ist ein 5- bis 6-gliedriger gesättigter Heterocyclus, der neben dem Stickstoffatom ein weiteres Heteroatom aus der Reihe N, O oder S enthalten kann. Beispielhaft und vorzugsweise seien genannt: Pyrrolidinyl, Pyrrolinyl, Piperidinyl, 1,2-Dihydropyridinyl, 1,4-Dihydropyridinyl, Piperazinyl, Morpholinyl, Thiomorpholinyl, Hexahydroazepinyl und Hexahydro-1,4-diazepinyl. Bevorzugt sind Piperidinyl, Piperazinyl, Morpholinyl und Pyrrolidinyl.
Halogen steht für Fluor, Chlor, Brom und Jod. Bevorzugt sind Fluor, Chlor und Brom. Besonders bevorzugt sind Fluor und Chlor.

Bevorzugt sind Verbindungen der Formel (I), in welcher
- R¹: für eine Gruppe der Formel 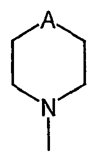 steht, worin
A für CR⁴R⁵, O, S, NR⁶ oder -CH₂NR⁶- steht, wobei
R⁴ und R⁵ unabhängig voneinander für Wasserstoff, (C₁-C₄)-Alkyl, das durch Hydroxy substituiert sein kann, Hydroxy, Fluor, Carboxyl oder (C₁-C₄)-Alkoxycarbonyl stehen oder gemeinsam mit dem Kohlenstoffatom, an das sie gebunden sind, eine Carbonylgruppe bilden, und
R⁶ für Wasserstoff, (C₂-C₄)-Alkenyl, (C₃-C₆)-Cycloalkyl, (C₁-C₄)-Alkoxycarbonyl, Formyl, Acetyl, (C₃-C₆)-Cycloalkylcarbonyl, (C₁-C₄)-Alkylsulfonyl, Aminocarbonyl, (C₁-C₄)-Alkylaminocarbonyl oder für (C₁-C₄)-Alkyl steht, welches seinerseits durch Hydroxy, Methoxy, Ethoxy, (C₁-C₄)-Alkoxycarbonyl, Amino, Dimethylamino, Diethylamino, Pyrrolidino, Piperidino oder Morpholino substituiert sein kann,
oder
- R¹: für 5-gliedriges, über ein Ring-Stickstoffatom verknüpftes Heteroaryl mit bis zu zwei weiteren Ring-Stickstoffatomen steht, das ein- oder zweifach, gleich oder verschieden, durch Fluor, Chlor, (C₁-C₄)-Alkoxycarbonyl oder (C₁-C₄)-Alkyl, welches seinerseits gegebenenfalls durch Hydroxy substituiert ist, substituiert sein kann,
- R²: für Phenyl steht, das ein- oder zweifach, gleich oder verschieden, durch Substituenten ausgewählt aus der Gruppe Fluor, Chlor, Cyano, (C₁-C₄)-Alkyl, Trifluormethyl, Formyl, Acetyl, (C₁-C₄)-Alkoxy, Hydroxy, Acetoxy, Pivaloyloxy, Amino, Formylamino, Acetylamino und Methylsulfonylamino substituiert sein kann,
wobei (C₁-C₄)-Alkyl und (C₁-C₄)-Alkoxy ihrerseits jeweils durch Hydroxy, Amino, Methoxy, Ethoxy oder Acetylamino substituiert sein können,
oder
- R²: für Pyrrolyl, Pyridyl oder Pyrimidinyl steht, die jeweils durch Amino, Fluor, Chlor, Methyl, Ethyl, Methoxy oder Ethoxy substituiert sein können,
und
- R³: für Wasserstoff, Fluor, Chlor, Brom, Methyl, Ethyl, Trifluormethyl, Nitro oder Cyano steht.

Von besonderer Bedeutung sind Verbindungen der Formel (I), in welcher
- R¹: für über ein Ring-Stickstoffatom angebundenes Imidazolyl oder für über ein Ring-Stickstoffatom angebundenes Piperazinyl, das am zweiten Ring-Stickstoffatom durch Methyl, Ethyl, 2-Hydroxyethyl, 2-Methoxyethyl, Acetyl, tert.-Butoxycarbonyl, Cyclopropylcarbonyl, Aminocarbonyl oder Methylsulfonyl substituiert sein kann, steht.

Gleichfalls von besonderer Bedeutung sind Verbindungen der Formel (I), in welcher
- R²: für Phenyl, das durch Hydroxy oder Fluor substituiert sein kann, oder für Pyridyl steht.

Ganz besonders bevorzugt sind Verbindungen der Formel (I), in welcher
- R¹: für über ein Ring-Stickstoffatom angebundenes Imidazolyl oder für über ein Ring-Stickstoffatom angebundenes Piperazinyl, das am zweiten Ring-Stickstoffatom durch Methyl, Ethyl, 2-Hydroxyethyl, 2-Methoxyethyl, Acetyl, tert.-Butoxycarbonyl oder Methylsulfonyl substituiert sein kann, steht,
- R²: für Phenyl, das in 4-Position zur Anknüpfstelle an den Phenylring durch Fluor oder Hydroxy substituiert sein kann, steht,
und
- R³: sich in 4-Position zur Anknüpfstelle desPyridazinonrings befindet und für Wasserstoff, Fluor, Chlor, Methyl oder Trifluormethyl steht.

Insbesondere bevorzugt sind Verbindungen der Formel (I), die ausgewählt sind aus der Gruppe der folgenden Verbindungen:

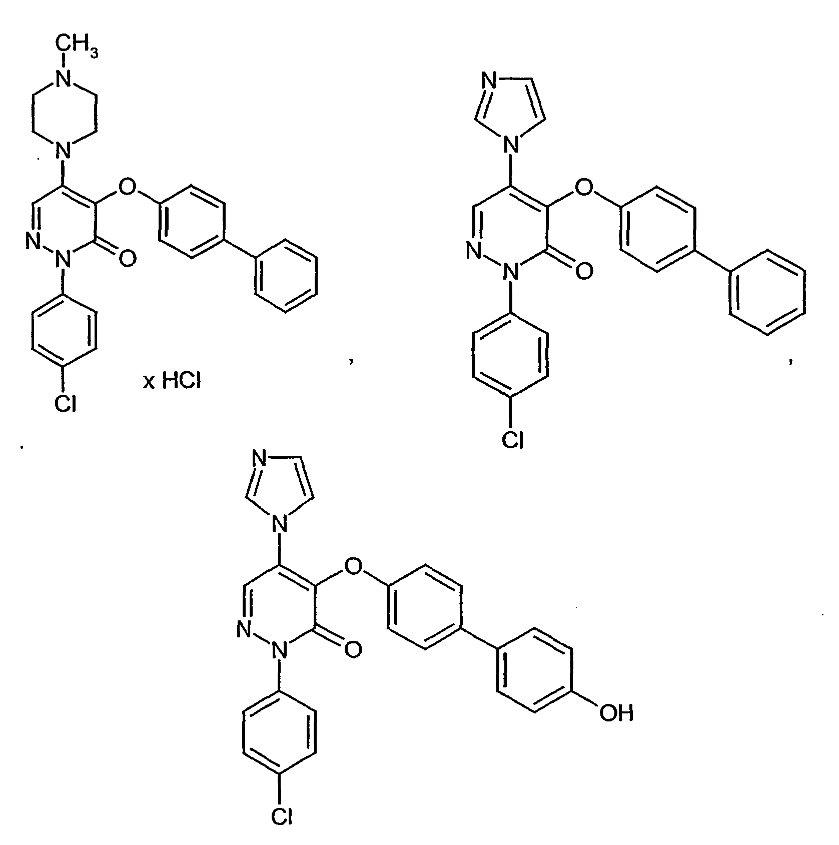

Die Erfindung betrifft weiterhin ein Verfahren zur Herstellung der Verbindungen der Formel (I), dadurch gekennzeichnet, dass man Verbindungen der Formel (II) 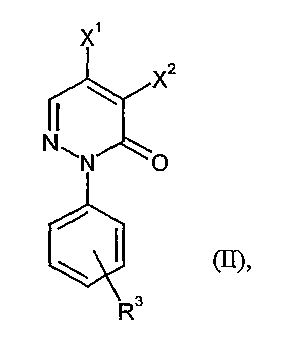 in welcher
- R³: die oben angegebene Bedeutung hat, und
- X¹ und X²: jeweils für Halogen, vorzugsweise für Brom oder Chlor stehen,
zunächst mit einer Verbindung der Formel (III)

R¹-H (III),

in welcher R¹ die oben angegebene Bedeutung hat,
in einem inerten Lösungsmittel gegebenenfalls in Gegenwart einer Hilfsbase und/oder eines Alkalimetalliodids in Verbindungen der Formel (IV) 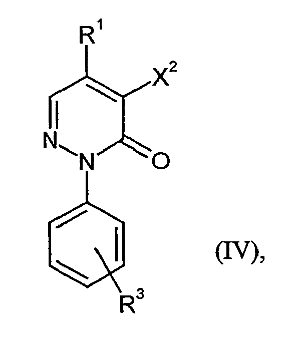 in welcher R¹, R³ und X² jeweils die oben angegebene Bedeutung haben,
überführt und diese dann mit einer Verbindung der Formel (V) 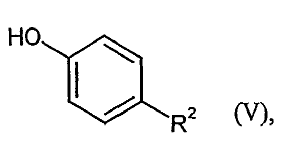 in welcher R² die oben angegebene Bedeutung hat,
in einem inerten Lösungsmittel in Gegenwart einer Base und gegebenenfalls in Gegenwart eines Alkalimetalliodids umsetzt.

Inerte Lösungsmittel für beide Verfahrensschritte sind beispielsweise Alkohole wie Methanol, Ethanol, n-Propanol, iso-Propanol, n-Butanol, tert.-Butanol oder Ethylenglykolmonomethylether, Ether wie Diethylether, Methyl-tert.-butylether, Dioxan, Tetrahydrofuran, Ethylenglykoldimethylether oder Diethylenglykoldimethylether, oder dipolar-aprotische Lösungsmittel wie Dimethylformamid, Dimethylacetamid, Dimethylsulfoxid, Sulfolan oder N-Methylpyrrolidon. Bevorzugt ist Dimethylformamid oder N-Methylpyrrolidon.

Beide Verfahrensschritte können gegebenenfalls auch ohne Lösungsmittel, d.h. in Substanz bzw. in der Schmelze durchgeführt werden.

Als Hilfsbasen für den Verfahrensschritt (II) + (III) → (IV) sind beispielsweise Alkali- oder Erdalkalicarbonate wie Natrium-, Kalium-, Caesium- oder Calciumcarbonat, oder tertiäre organische Amine wie Triethylamin, Diisopropylethylamin, N-Methylmorpholin, N-Methylpiperidin oder 1,4-Diazabicyclo[2.2.2]octan geeignet. Bevorzugt werden tertiäre Amine. Die Hilfsbase wird dabei im Molverhältnis von 0.75:1 bis 2:1, bezogen auf die Verbindung (II), eingesetzt; bevorzugt ist ein Molverhältnis von 0.95:1 bis 1.5:1.

Als Basen für den Verfahrensschritt (IV) + (V) → (I) eignen sich Alkali- oder Erdalkalicarbonate wie Natrium-, Kalium- oder Calciumcarbonat, Alkali-Alkoholate wie Natrium- oder Kaliummethanolat, Natrium- oder Kaliumethanolat oder Kalium-tert.-butylat, Alkalihydride wie Natrium- oder Kaliumhydrid, Amide wie Natriumamid, Lithium-bis-(trimethylsilyl)amid oder Lithiumdiisopropylamid, tertiäre organische Amine wie Triethylamin, Ethyldiisopropylamin, N-Methylmorpholin, N-Methylpiperidin oder 1,4-Diazabicyclo[2.2.2]octan, oder metallorganische Verbindungen wie n-Butyllithium. Bevorzugt ist 1,4-Diazabicyclo[2.2.2]octan, Natriumhydrid, Kaliumhydrid oder Kalium-tert.-butylat. Die Base wird dabei im Molverhältnis von 0.75:1 bis 2:1, bezogen auf die Verbindung (V), eingesetzt; bevorzugt ist ein Molverhältnis von 0.95:1 bis 1.5:1.

Beide Verfahrensschritte können in Gegenwart von Alkalimetalliodiden wie Lithium-, Natrium-, Kalium- oder Caesiumiodid durchgeführt werden. Das Iodid wird dabei in einem molaren Verhältnis von 0.001:1 bis 2:1, bezogen auf die Verbindung (II) bzw. (IV), eingesetzt; bevorzugt ist ein Molverhältnis von 0.01:1 bis 1:1.

Die Verbindungen (II) und (III) werden in einem molaren Verhältnis von 1:1 bis 1:10, bevorzugt von 1:1 bis 1:5, eingesetzt. Die Umsetzung wird in einem Temperaturbereich von +20°C bis +200°C, bevorzugt von +20°C bis +120°C, durchgeführt. Die Umsetzung kann bei Normaldruck, bei vermindertem oder bei erhöhtem Druck durchgeführt werden; bevorzugt ist die Durchführung bei Normaldruck.

Die Verbindungen (IV) und (V) werden in einem molaren Verhältnis von 10:1 bis 1:10, bevorzugt von 1:1 bis 1:3, eingesetzt. Die Umsetzung wird in einem Temperaturbereich von +20°C bis +200°C, bevorzugt von +100°C bis +180°C, durchgeführt. Die Umsetzung kann bei Normaldruck, bei vermindertem oder bei erhöhtem Druck durchgeführt werden; bevorzugt ist die Durchführung bei Normaldruck.

Die Verbindungen der Formel (II) sind bekannt oder können in Analogie zu literaturbekannten Verfahren hergestellt werden [siehe z.B. Drury, Angew. Chemie 77, 282 (1965)].

Die Verbindungen der Formeln (III) und (V) sind kommerziell erhältlich, literaturbekannt oder können in Analogie zu literaturbekannten Verfahren hergestellt werden.

In einer Verfahrensvariante können die Verbindungen der Formel (I) auch dadurch hergestellt werden, dass man entweder
[A] Verbindungen der Formel (VI) 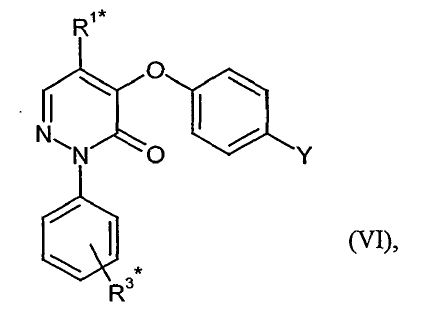 in welcher
   - R^{1*}: die oben angegebene Bedeutung von R¹ hat, jedoch seinerseits nicht durch Brom oder Iod substituiert ist,
   - R^{3*}: die oben angegebene Bedeutung von R³ hat, jedoch nicht für Brom oder Iod steht, und
   - Y: für Brom, Iod oder Trifluormethylsulfonyloxy (Triflat) steht,
   in einer Kupplungsreaktion mit einer Verbindung der Formel (VII) 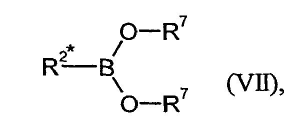 in welcher
   - R^{2*}: die oben angegebene Bedeutung von R² hat, jedoch seinerseits nicht durch Brom oder Iod substituiert ist, und
   - R⁷: für Wasserstoff oder Methyl steht oder beide Reste zusammen eine CH₂CH₂- oder C(CH₃)₂-C(CH₃)₂-Brücke bilden,
   in einem inerten Lösungsmittel in Gegenwart eines geeigneten PalladiumKatalysators und einer Base umsetzt,
   oder
[B] Verbindungen der Formel (VIII) 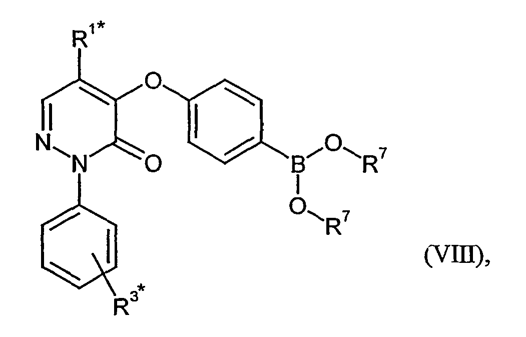 in welcher
   R^{1*}, R^{3*} und R⁷ jeweils die oben angegebene Bedeutung haben,
   in einer Kupplungsreaktion mit einer Verbindung der Formel (IX)

      R²-Z (IX),

      in welcher
      - R²: die oben angegebene Bedeutung hat, und
      - Z: für Brom oder Iod steht,
   in einem inerten Lösungsmittel in Gegenwart eines geeigneten PalladiumKatalysators und einer Base umsetzt.

Als inerte Lösungsmittel für die Verfahrensvariante [A] eignen sich beispielsweise Alkohole wie Methanol, Ethanol, n-Propanol, iso-Propanol oder n-Butanol, aromatische Kohlenwasserstoffe wie Benzol, Xylol oder Toluol, oder dipolar-aprotische Lösungsmittel wie Dimethylformamid, Dimethylacetamid, Dimethylsulfoxid, Sulfolan oder N-Methylpyrrolidon. Ebenso ist es möglich, Gemische der genannten Lösungsmittel einzusetzen. Bevorzugt ist Dimethylformamid oder ein Gemisch aus Ethanol und Toluol.

Als inerte Lösungsmittel für die Verfahrensvariante [B] eignen sich beispielsweise dipolar-aprotische Lösungsmittel wie Dimethylformamid, Dimethylacetamid, Dimethylsulfoxid, Sulfolan oder N-Methylpyrrolidon. Bevorzugt ist Dimethylsulfoxid.

Als Palladium-Katalysatoren eignen sich für beide Verfahrensvarianten die für Suzuki-Kupplungen üblichen Palladium(0)- und Palladium(II)-Komplexverbindungen; bevorzugt ist Palladium-tetrakistriphenylphosphan, [1,1-Bis(diphenylphosphino)-ferrocenyl] dichlor-palladium(II)-Komplex oder Bistriphenylphosphan-dichlorpalladium(II)-Komplex. Die Palladiumverbindung wird dabei in einem Molverhältnis von 0.005:1 bis 0.5:1, bevorzugt in einem Molverhältnis von 0.02:1 bis 0.15:1, bezogen auf die Verbindung (VI) bzw. (VIII), eingesetzt.

Als Basen eignen sich für beide Verfahrensvarianten wäßrige Lösungen von Alkalimetallcarbonaten und -hydrogencarbonaten sowie von Alkalisalzen der Essig- oder Propionsäure; bevorzugt ist Natriumcarbonat. Bezogen auf die Verbindung (VI) bzw. (VIII) wird ein molares Verhältnis von 1:1 bis 10:1, bevorzugt von 1.5:1 bis 8:1, der Base verwendet.

Die Verbindungen (VI) und (VII) werden in einem molaren Verhältnis von 3:1 bis 1:3, bevorzugt von 1.2:1 bis 1:1.2, eingesetzt. Die Umsetzung wird in einem Temperaturbereich von +20°C bis +150°C, bevorzugt von +50°C bis +100°C, durchgeführt. Die Umsetzung kann bei Normaldruck, bei vermindertem oder bei erhöhtem Druck durchgeführt werden; bevorzugt ist die Durchführung bei Normaldruck.

Die Verbindungen (VIII) und (IX) werden in einem molaren Verhältnis von 2:1 bis 1:10, bevorzugt von 1:1 bis 1:3, eingesetzt. Die Umsetzung wird in einem Temperaturbereich von +20°C bis +150°C, bevorzugt von +50°C bis +100°C, durchgeführt. Die Umsetzung kann bei Normaldruck, bei vermindertem oder bei erhöhtem Druck durchgeführt werden; bevorzugt ist die Durchführung bei Normaldruck.

Die Verbindungen der Formel (VI) und (VIII) können entsprechend dem zuvor beschriebenen Verfahren (IV) + (V) → (I) hergestellt werden.

Die Verbindungen der Formel (VIII) können auch analog der Verfahrensvariante [A] durch Palladium-katalysierte Umsetzung von Verbindungen der Formel (VI) mit entsprechenden Borsäure-Derivaten, wie beispielsweise 4,4,4',4',5,5,5',5'-Octamethyl-2,2'-bi-1,3,2-dioxaborolan, erhalten werden [vgl. z.B. A. Suzuki, Acc. Chem. Res., 15, 178 (1982); Miyaura et al., J. Am. Chem. Soc., 111, 314 (1989)].

Die Verbindungen der Formel (VII) sind kommerziell erhältlich, literaturbekannt oder können in Analogie zu literaturbekannten Verfahren hergestellt werden [siehe z.B. A. Suzuki, in: Metal-catalyzed cross-coupling reactions, F. Driedrich und P.J. Stang, Hrsg., Wiley, Weinheim 1998].

Die Verbindungen der Formel (IX) sind kommerziell erhältlich, literaturbekannt oder können in Analogie zu literaturbekannten Verfahren hergestellt werden.

In einer weiteren Verfahrensvariante können die Verbindungen der Formel (I) auch dadurch hergestellt werden, dass man Verbindungen der Formel (II) zunächst mit einer Verbindung der Formel (V) in einem inerten Lösungsmittel in Gegenwart einer Base in Verbindungen der Formel (X) 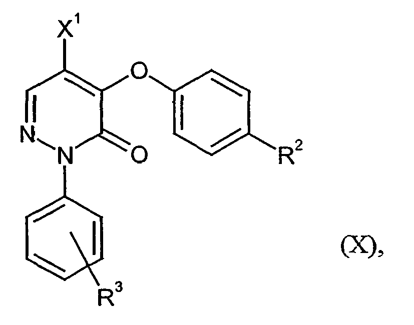 in welcher R², R³ und X¹ jeweils die oben angegebene Bedeutung haben,
überführt und diese dann mit einer Verbindung der Formel (III) in einem inerten Lösungsmittel gegebenenfalls in Gegenwart einer Hilfsbase und/oder eines Alkalimetalliodids umsetzt.

Inerte Lösungsmittel für den Verfahrensschritt (II) + (V) → (X) sind vorteilhafterweise Ether wie Methyl-tert.-butylether, Dioxan, Tetrahydrofuran oder Ethylenglykoldimethylether. Bevorzugt ist Dioxan.

Inerte Lösungsmittel für den Verfahrensschritt (X) + (III) → (I) sind beispielsweise Ether wie Dioxan, Tetrahydrofuran, Ethylenglykoldimethylether oder Diethylenglykoldimethylether, oder dipolar-aprotische Lösungsmittel wie Dimethylformamid, Dimethylacetamid, Dimethylsulfoxid, Sulfolan oder N-Methylpyrrolidon. Bevorzugt ist Dimethylformamid. Dieser Verfahrensschritt kann gegebenenfalls auch ohne Lösungsmittel, d.h. in Substanz bzw. in der Schmelze durchgeführt werden.

Als Basen für den Verfahrensschritt (II) + (V) → (X) eignen sich beispielsweise Metall-Alkoholate wie Natrium- oder Kaliummethanolat, Natrium- oder Kaliumethanolat, Thalliummethanolat oder -ethanolat oder Kalium-tert.-butylat, Metallhydride wie Natrium-, Kalium- oder Lithiumhydrid, Amide wie Natrium- oder Kaliumamid, Lithium- oder Natrium-bis(trimethylsilyl)amid oder Lithiumdiisopropylamid, oder metallorganische Verbindungen wie Methyl-, n-Butyl- oder Phenyllithium. Bevorzugt ist Natriumhydrid. Die Base wird dabei im Molverhältnis von 1.5:1 bis 1:1.5, bezogen auf die Verbindung (V), eingesetzt; bevorzugt ist ein Molverhältnis von 1:1 bis 1.1:1. Die Umsetzung der Base mit der Verbindung (V) wird in einem Temperaturbereich von -78°C bis +30°C durchgeführt; bei Verwendung von Natriumhydrid erfolgt dies bevorzugt in einem Temperaturbereich von 0°C bis +30°C.

Als Hilfsbasen für den Verfahrensschritt (X) + (III) → (I) eignen sich Alkali- oder Erdalkalicarbonate wie Natrium-, Kalium-, Caesium- oder Calciumcarbonat, oder tertiäre organische Amine wie Triethylamin, Ethyldiisopropylamin, N-Methylmorpholin oder N-Methylpiperidin; bevorzugt ist Triethylamin oder Ethyldiisopropylamin. Die Base wird dabei im Molverhältnis von 1:1 bis 2:1, bezogen auf die Verbindung (X), eingesetzt; bevorzugt ist ein Molverhältnis von 1:1 bis 1.5:1.

Der Verfahrensschritt (X) + (III) → (I) kann vorteilhafterweise in Gegenwart von Alkalimetalliodiden wie Lithium-, Natrium-, Kalium- oder Caesiumiodid durchgeführt werden; bevorzugt ist Natrium- oder Kaliumiodid. Das Iodid wird dabei in einem molaren Verhältnis von 0.1:1 bis 2:1, bezogen auf die Verbindung (X), eingesetzt; bevorzugt ist ein Molverhältnis von 1:1.

Die Verbindungen (II) und (V) werden in einem molaren Verhältnis von 0.5:1 bis 2:1, bevorzugt von 1:1, eingesetzt. Die Umsetzung wird in einem Temperaturbereich von 0°C bis +100°C, bevorzugt von +10°C bis +30°C, durchgeführt. Die Umsetzung kann bei Normaldruck, bei vermindertem oder bei erhöhtem Druck durchgeführt werden; bevorzugt ist die Durchführung bei Normaldruck.

Die Verbindungen (III) und (X) werden in einem molaren Verhältnis von 1:3 bis 5:1, bevorzugt von 1:1.5 bis 2:1, eingesetzt. Die Umsetzung wird in einem Temperaturbereich von +50°C bis +200°C, bevorzugt von +120°C bis +170°C, durchgeführt. Die Umsetzung kann bei Normaldruck, bei vermindertem oder bei erhöhtem Druck durchgeführt werden; bevorzugt ist die Durchführung bei Normaldruck.

In einer weiteren Verfahrensvariante können die Verbindungen der Formel (I) auch dadurch hergestellt werden, dass man Verbindungen der Formel (II) zunächst mit einem Überschuß an Verbindung (V) in einem inerten Lösungsmittel in Gegenwart einer Base und gegebenenfalls in Gegenwart eines Alkalimetalliodids in Verbindungen der Formel (XI) 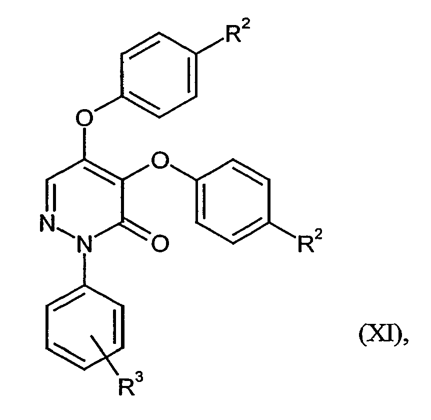 in welcher R² und R³ jeweils die oben angegebene Bedeutung haben,
überführt und diese dann mit einer Verbindung der Formel (III) in einem inerten Lösungsmittel gegebenenfalls in Gegenwart einer Hilfsbase und/oder eines Alkalimetalliodids umsetzt.

Inerte Lösungsmittel für den Verfahrensschritt (II) + (V) → (XI) sind vorteilhafterweise aprotische Lösungsmittel wie beispielsweise Dimethylformamid, Dimethylacetamid, Dimethylsulfoxid oder Sulfolan oder Ether wie Methyl-tert.-butylether, Dioxan, Tetrahydrofuran oder Ethylenglykoldimethylether. Bevorzugt ist Dimethylformamid.

Inerte Lösungsmittel für den Verfahrensschritt (XI) + (III) → (I) sind beispielsweise Ether wie Dioxan, Tetrahydrofuran, Ethylenglykoldimethylether oder Diethylenglykoldimethylether, oder dipolar-aprotische Lösungsmittel wie Dimethylformamid, Dimethylacetamid, Dimethylsulfoxid, Sulfolan oder N-Methylpyrrolidon. Bevorzugt ist Dimethylformamid. Dieser Verfahrensschritt kann gegebenenfalls auch ohne Lösungsmittel, d.h. in Substanz bzw. in der Schmelze durchgeführt werden.

Als Basen für den Verfahrensschritt (II) + (V) → (XI) eignen sich beispielsweise Metall-Alkoholate wie Natrium- oder Kaliummethanolat, Natrium- oder Kaliumethanolat, Thalliummethanolat oder -ethanolat oder Kalium-tert.-butylat, Metallhydride wie Natrium-, Kalium- oder Lithiumhydrid, Amide wie Natrium- oder Kaliumamid, Lithium- oder Natrium-bis(trimethylsilyl)amid oder Lithiumdüsopropylamid, oder metallorganische Verbindungen wie Methyl-, n-Butyl- oder Phenyllithium. Bevorzugt ist Natriumhydrid. Die Base wird dabei im Molverhältnis von 1.5:1 bis 1:1.5, bezogen auf die Verbindung (V), eingesetzt; bevorzugt ist ein Molverhältnis von 1:1 bis 1.1:1. Die Umsetzung der Base mit der Verbindung (V) wird in einem Temperaturbereich von -78°C bis +30°C durchgeführt; bei Verwendung von Natriumhydrid erfolgt dies bevorzugt in einem Temperaturbereich von 0°C bis +30°C.

Als Hilfsbasen für den Verfahrensschritt (XI) + (III) → (I) eignen sich Alkali- oder Erdalkalicarbonate wie Natrium-, Kalium-, Caesium- oder Calciumcarbonat, oder tertiäre organische Amine wie Triethylamin, Ethyldiisopropylamin, N-Methylmorpholin, N-Methylpiperidin oder 1,4-Diazabicyclo[2.2.2]octan; bevorzugt ist 1,4-Diazabicyclo[2.2.2]octan oder Ethyldiisopropylamin. Die Base wird dabei im Molverhältnis von 0.75:1 bis 2:1, bezogen auf die Verbindung (III), eingesetzt; bevorzugt ist ein Molverhältnis von 0.95:1 bis 1.5:1.

Der Verfahrensschritt (II) + (V) → (XI) kann vorteilhafterweise in Gegenwart von Alkalimetalliodiden wie Lithium-, Natrium-, Kalium- oder Caesiumiodid durchgeführt werden; bevorzugt ist Natrium- oder Kaliumiodid. Das Iodid wird dabei in einem molaren Verhältnis von 0.05:1 bis 2:1, bezogen auf die Verbindung (V), eingesetzt; bevorzugt ist ein Molverhältnis von 0.1:1 1 bis 0.5:1.

Der Verfahrensschritt (XI) + (III) → (I) kann gleichfalls vorteilhafterweise in Gegenwart von Alkalimetalliodiden wie Lithium-, Natrium-, Kalium- oder Caesiumiodid durchgeführt werden; bevorzugt ist Natrium- oder Kaliumiodid. Das Iodid wird dabei in einem molaren Verhältnis von 0.1: 1 bis 2:1, bezogen auf die Verbindung (XI), eingesetzt; bevorzugt ist ein Molverhältnis von 1:1.

Die Verbindungen (II) und (V) werden in einem molaren Verhältnis von 0.05:1 bis 0.5:1, bevorzugt von 0.1:1 bis 0.5:1, eingesetzt. Die Umsetzung wird in einem Temperaturbereich von +50°C bis +200°C, bevorzugt von +100°C bis +160°C, durchgeführt. Die Umsetzung kann bei Normaldruck, bei vermindertem oder bei erhöhtem Druck durchgeführt werden; bevorzugt ist die Durchführung bei Normaldruck.

Die Verbindungen (III) und (XI) werden in einem molaren Verhältnis von 1: 1 bis 5:1, bevorzugt von 1:1 bis 2:1, eingesetzt. Die Umsetzung wird in einem Temperaturbereich von +50°C bis +200°C, bevorzugt von +100°C bis +170°C, durchgeführt. Die Umsetzung kann bei Normaldruck, bei vermindertem oder bei erhöhtem Druck durchgeführt werden; bevorzugt ist die Durchführung bei Normaldruck.

Verbindungen der Formel (I), in der R¹ für gegebenenfalls substituiertes 1,2,3-Triazol-1-yl steht, können auch dadurch hergestellt werden, dass man Verbindungen der Formel (X) zunächst mit einem Metallazid in einem inerten Lösungsmittel in Verbindungen der Formel (XII) 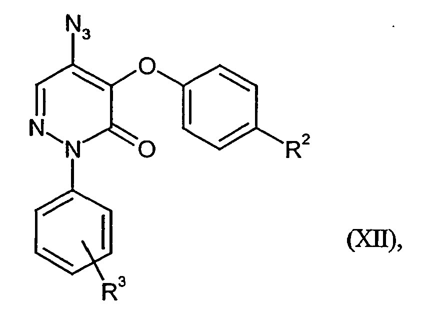 in welcher R² und R³ jeweils die oben angegebene Bedeutung haben,
überführt und diese dann mit einer Verbindung der Formel (XIII)

R⁸-C≡C-R⁹ (XIII),

in welcher
- R⁸ und R⁹: unabhängig voneinander für Wasserstoff, (C₁-C₆)-Alkoxycarbonyl oder für (C₁-C₆)-Alkyl, welches seinerseits durch Hydroxy oder Halogen substituiert sein kann, stehen,
in Anwesenheit oder Abwesenheit eines inerten Lösungsmittels zu Verbindungen der Formel (XIVa) bzw. (XIVb) 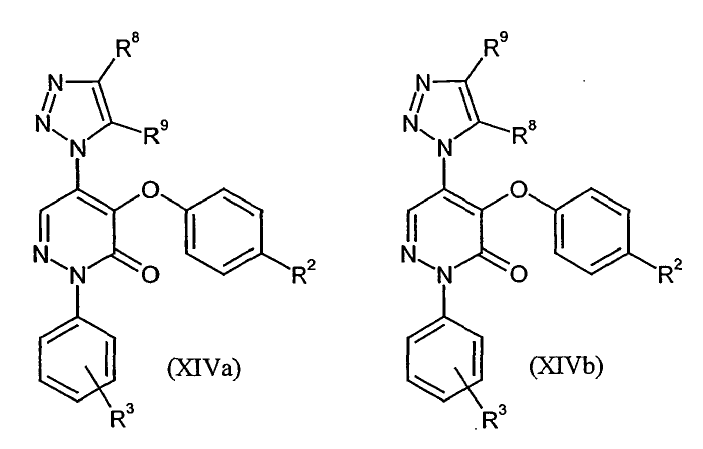 in welcher R², R³, R⁸ und R⁹ jeweils die oben angegebene Bedeutung haben, umsetzt.

Inerte Lösungsmittel für den Verfahrensschritt (X) → (XII) sind vorteilhafterweise aprotische Lösungsmittel wie beispielsweise Dimethylformamid, Dimethylacetamid, Dimethylsulfoxid, Sulfolan, Aceton, Acetonitril oder Ether wie Dioxan, Tetrahydrofuran oder Ethylenglykoldimethylether. Bevorzugt ist Dimethylformamid.

Inerte Lösungsmittel für den Verfahrensschritt (XII) + (XIII) → (XIVa) bzw. (XIVb) sind beispielsweise Ether wie Dioxan, Tetrahydrofuran, Ethylenglykoldimethylether oder Diethylenglykoldimethylether, dipolar-aprotische Lösungsmittel wie Dimethylformamid, Dimethylacetamid, Dimethylsulfoxid, Sulfolan oder N-Methylpyrrolidon, oder aromatische Kohlenwasserstoffe wie Benzol, Toluol oder isomere Xylole, oder Chlorbenzol oder Nitrobenzol. Dieser Verfahrensschritt kann gegebenenfalls auch ohne Lösungsmittel durchgeführt werden. Bevorzugt wird die Umsetzung in Toluol oder ohne Lösungsmittel durchgeführt.

Die Verbindungen (X) werden mit dem Azid in einem molaren Verhältnis von 1:1 bis 1:15, bevorzugt von 1:2 bis 1:6, umgesetzt. Als Azide werden Alkalimetall-Salze der Stickstoffwasserstoffsäure verwendet; bevorzugt ist Natriumazid. Die Umsetzung wird in einem Temperaturbereich von +20°C bis +80°C, bevorzugt von +40°C bis +60°C, durchgeführt. Die Umsetzung kann bei Normaldruck, bei vermindertem oder bei erhöhtem Druck durchgeführt werden; bevorzugt ist die Durchführung bei Normaldruck.

Die Verbindungen (XII) und (XIII) werden in einem molaren Verhältnis von 1:1 bis 1:15, bevorzugt von 1:2 bis 1:8, eingesetzt. Die Umsetzung wird in einem Temperaturbereich von +20°C bis +130°C, bevorzugt von +80°C bis +120°C, durchgeführt. Die Umsetzung kann bei Normaldruck, bei vermindertem oder bei erhöhtem Druck durchgeführt werden; bevorzugt ist die Durchführung bei Normaldruck.

Die Verbindungen der Formel (XIII) sind kommerziell erhältlich, literaturbekannt oder können in Analogie zu literaturbekannten Verfahren hergestellt werden.

Überraschenderweise zeigen die Verbindungen der Formel (I) ein nicht vorhersehbares, wertvolles pharmakologisches Wirkspektrum und sind daher insbesondere zur Prophylaxe und/oder Behandlung von Erkrankungen bei Menschen und Tieren geeignet.

Die pharmazeutische Wirksamkeit der Verbindungen der Formel (I), insbesondere ihre antifibrotische Wirkung, lässt sich durch ihre Wirkung als Lysyloxidase-Inhibitoren erklären.

Die erfindungsgemäßen Verbindungen sind aufgrund ihrer pharmakologischen Eigenschaften allein oder in Kombination mit einem oder mehreren anderen Wirkstoffen zur Prophylaxe und/oder Behandlung von Erkrankungen bei Menschen und Warmblütern geeignet, insbesondere von Erkrankungen, die durch den schädlichen Aufbau von Kollagen und/oder durch die übermäßige enzymatische Aktivität der Lysyloxidase gekennzeichnet sind; unabhängig von der diesen Schädigungen zugrundeliegenden Erkrankung.

Schädigende Grunderkrankungen in diesem Sinne können Strahlenschäden z.B. in Folge einer Radiotherapie, Schädigungen aufgrund einer Chemotherapie z.B. mit Bleomycin oder Adriamycin, virale Erkrankungen z.B. Hepatitis B-Infektion, chronische Vergiftungen wie z.B. Alkoholabusus, parasitäre Erkrankungen wie z.B. Schistosomiasis, angeborene Erkrankungen wie z.B. Wilson's Syndrom, Stoffwechselerkrankungen wie z.B. Diabetes, Autoimmunerkrankungen oder auch Erkrankungen unbekannter Herkunft sein. Allen ist gemeinsam, dass sie in einem oder mehreren Organen fibrotische Schäden auslösen. Durch die Therapie mit den erfindungsgemäßen Verbindungen werden die fibrotische Schädigung und damit zusammenhängende Folgeschäden, wie z.B. Organversagen, Varizenbildung, portaler Hochdruck, Bildung bösartiger Geschwülste, bekämpft.

Die therapeutische Verwendung der erfindungsgemäßen Verbindungen umfaßt ebenfalls die Prophylaxe und/oder Behandlung fibrotischer Erkrankungen der inneren Organe. Beispielhaft seien hier Lunge, Herz, Niere, Knochenmark und insbesondere Leber genannt. Behandelt werden können mit den erfindungsgemäßen Verbindungen somit zum Beispiel Leberfibrose, Lebercirrhose, Lungenfibrose, Endomyocardfibrose, Kardiomyopathie, Nephropathie, Glomerulonephritis, interstitielle Nierenfibrose, fibrotische Schäden in Folge von Diabetes, Knochenmarksfibrose und ähnliche fibrotische Erkrankungen.

In einem weiteren Aspekt der Erfindung werden durch Verbindungen der Formel (I) dermatologische Fibrosen therapiert. Als Beispiele für derartige Erkrankungen seien Skleroderma, Morphaea, Keloide, Hypertrophe Narbenbildung (auch nach chirurgischen Eingriffen) und Naevi genannt. Auch können die erfindungsgemäßen Verbindungen bei alternder und verhornender Haut eingesetzt werden.

Auch fibrotische Erkrankungen des Auges, wie diabetische Retinopathie und proliferative Vitroretinopathie, können mit den erfindungsgemäßen Verbindungen therapiert werden; sie lassen sich auch vorteilhaft zur Bekämpfung postoperativer Narbenbildung, z.B. in Folge von Glaukoma-Operationen einsetzen.

Des weiteren können die erfindungsgemäßen Verbindungen bei der Bekämpfung neoplastischer Erkrankungen, insbesondere solcher Erkrankungen, die durch mit Neoangiogenese verbundene Metastasierung gekennzeichnet sind, therapeutisch eingesetzt werden.

Die vorliegende Erfindung betrifft auch die Verwendung der Verbindungen der Formel (I) zur Herstellung von Arzneimitteln zur Prophylaxe und/oder Behandlung der zuvor genannten Krankheitsbilder.

Die vorliegende Erfindung betrifft weiterhin ein Verfahren zur Prophylaxe und/oder Behandlung der zuvor genannten Krankheitsbilder mit den Verbindungen der Formel (I).

Weiterer Gegenstand der vorliegenden Erfindung sind Arzneimittel, die mindestens eine Verbindung der Formel (I), vorzugsweise zusammen mit einem oder mehreren pharmakologisch unbedenklichen Hilfs- oder Trägerstoffen enthalten, sowie deren Verwendung zu den zuvor genannten Zwecken.

Die Wirkstoffe können in bekannter Weise in die üblichen Formulierungen überführt werden, wie Tabletten, Dragees, Pillen, Granulate, Aerosole, Sirupe, Cremes, Emulsionen, Suspensionen und Lösungen, unter Verwendung inerter, nicht-toxischer, pharmazeutisch geeigneter Trägerstoffe und Lösemittel. Hierbei soll die therapeutisch wirksame Verbindung jeweils in einer Konzentration von etwa 0.01 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral, parenteral oder topisch.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0.001 bis 20 mg/kg, vorzugsweise etwa 0.01 bis 10 mg/kg Körpergewicht zur Erzielung wirksamer Ergebnisse zu verabreichen, und bei oraler Applikation beträgt die Dosierung etwa 0.01 bis 60 mg/kg, vorzugsweise 0.1 bis 30 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Die vorliegende Erfindung wird an den folgenden, nicht einschränkenden bevorzugten Beispielen veranschaulicht, die die Erfindung jedoch keinesfalls beschränken.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Gemischen beziehen sich jeweils auf das Volumen.

### A. Bewertung der physiologischen Wirksamkeit

Zur Untersuchung der *in vitro-* und in vivo-Wirkung der erfindungsgemäßen Verbindungen können folgende biologische Assays verwendet werden:

### 1. Test auf Lysyloxidase-inhibitorische Wirkung:

In einer Modfizierung einer Vorschrift von Kagan et al. [Biochem. J. 177, 203-214 (1979)] wurde das Enzym aus Rinderaorten durch Extraktion mit Phosphatpuffer, der Harnstoff enthielt, gefolgt von Ausfällung und Chromatographie auf DEAE-Sephadex gewonnen.

Die Testlösung (Gesamtvolumen 200 µl) setzt sich zusammen aus 1.5 mM Homovanillinsäure, 5 mM 1,5-Diaminopentan, 1 Einheit Meerrettich-Peroxidase und 1.5 M Harnstoff in 50 mM Natriumboratpuffer pH 8.2. Das Enzym wurde 15 Minuten lang mit der zu testenden Verbindung vorinkubiert; die Reaktion wurde durch Zugabe von Diaminopentan und Homovanillin / Meerrettich-Peroxidase gestartet. Es wurde die Fluoreszenz des sich bildenden Homovanillin-Dimeren bei 315 nm Excitation und 425 nm Emission gemessen. Diese Testanordnung ist eine Modifizierung der Vorschrift von Trackman et al. [Anal. Biochem. 113, 336-341 (1981)].

Der IC₅₀-Wert als Maß für die Inhibition der Lysyloxidase wird durch Testung verschiedener Substanzkonzentrationen anhand der Konzentrations-Wirkungskurven ermittelt. Beispielhafte IC₅₀-Werte zu den erfindungsgemäßen Verbindungen sind in der nachstehenden Tabelle 1 aufgeführt:

| Tabelle 1: | IC₅₀ (µM) |
|---|---|
| *Vergleichsverbindungen:* | |
| β-Aminopropionitril (BAPN) | 10 |
| 4-Ethoxy-2-methyl-5-morpholino-3(2H)-pyridazinon (Emorfazone) | > 4 |
| | |

| *Ausführungsbeispiele:* | |
|---|---|
| Bsp. 5 | 0.017 |
| Bsp. 8 | 0.007 |
| Bsp. 82 | 0.011 |
| Bsp. 12 | 0.003 |
| Bsp. 25 | 0.005 |
| Bsp. 34 | 0.009 |
| Bsp. 81 | 0.007 |
| Bsp. 176 | 0.010 |

Die erfindungsgemäßen Verbindungen zeigen eine deutlich stärkere Hemmwirkung des Enzyms Lysyloxidase als die Referenzverbindung BAPN oder das dem strukturell nächsten Stand der Technik entsprechende 4-Ethoxy-2-methyl-5-morpholino-3(2H)-pyridazinon (Emorfazone).

### 2. Test auf antifibrotische Wirkung in der Leber

### 2.1 Tiermodelle:

### a) Modell der chronischen Tetrachlorkohlenstoff- Vergiftung:

Chronische Tetrachlorkohlenstoff (CCl₄)-Behandlung ist eine Standardmethode zur Erzeugung einer Leberfibrose nichtviralen Ursprungs und nachfolgender Zirrhose. Sie wird allgemein als Modell für die Leberfibrose und -zirrhose im Menschen akzeptiert [E. McLean, A. McLean, P. Sutton, Instant Fibrosis: An improved method for producing cirrhosis of the liver in rats by simultaneous administration of carbon tetrachloride and phenobarbitone, Br. J. Exp. Pathol. 50, 502-506 (1969)].

Es wurden weibliche Sprague-Dawley-Ratten (200-220 g) eingesetzt. Um eine maximale mikrosomale Metabolisierung des CCl₄ zu erreichen, erhielten die Tiere eine Woche vor Beginn der Gabe von Tetrachlorkohlenstoff Isoniazid im Trinkwasser (lg/l). CCl₄ wurde oral jeden fünften Tag in einer Dosis von 0.2 ml/100 g Körpergewicht in einer 1:1-Mischung mit Mineralöl verabreicht. Die erfindungsgemäßen Verbindungen wurden, wie in Tabelle 3 angegeben, im gleichen Zeitraum oral gegeben, bevorzugt in Solutol^{®} / Ethanol / PBS-Gemischen. Die Nekropsie wurde 6 Wochen nach Beginn der Behandlung durchgeführt.

### b) Modell der Gallengangsligatur:

Die Versuche wurden mit weiblichen Sprague-Dawley-Ratten (200-220 g) durchgeführt. Um eine chronische Fibrose zu erzeugen, wurde in einem chirurgischen Verfahren der Gallengang (*ductus hepaticus communis*) doppelt ligiert. Ethibloc^{®}-Okklusionsemulsion (Ethicon, Norderstedt, Deutschland), eine Mischung aus Prolamin und Ethanol, wurde retrograd in das Gallenwegssystem instilliert [J. Kountouras, B. Billing, P. Scheuer, Prolonged bile obstruction: A new experimental model for cirrhosis in the rat, Br. J. Exp. Pathol. 65, 305-311 (1984); G. Boigk, L. Stroedter, H. Herbst, J. Waldschmidt, E.O. Riecken, D. Schuppan, Silymarin retards collagen accumulation in early and advanced biliary fibrosis secondary to complete bile duct obliteration in rats, Hepatology 26, 643-649 (1997)]. Die erfindungsgemäßen Verbindungen wurden, wie in Tabelle 2 angegeben, im gleichen Zeitraum oral gegeben, bevorzugt in Solutol^{®} / Ethanol / PBS-Gemischen. Die Tiere wurden drei Wochen nach dem chirurgischen Eingriff getötet und untersucht.

### c) Modell der Serum-induzierten Leberfibrose:

Eine septale Fibrose kann bei Ratten auch durch wiederholte Injektion von heterologem Serum ausgelöst werden. Hierzu werden pro Tier zweimal wöchentlich 0.5 ml steril filtriertes Schweineserum i.p. verabreicht [Bhunchet und Wake, Hepatology 16, 1452-73 (1992)]. Die Versuche wurden mit weiblichen Sprague-Dawley-Ratten (200-220 g) durchgeführt. Nach 6-8 Wochen wird die septale Fibrose histologisch manifest und läßt sich auch durch Erhöhung des Leber-Hydroxyprolin-Gehalts nachweisen.

### 2.2 Auswertung der Tiermodelle:

### a) Histologische Bewertung / Morphometrie:

Aus dem rechten Vorderleberlappen jeder Ratte wurden standardisierte, transversale Zylinder aus Lebergewebe (ca. 10 x 2 mm) geschnitten. Gefrierschnitte wurden mit 0.1 % Picrosiriusrot-Lösung zum Nachweis von Leberfibrose-assoziiertem Narbenkollagen gefärbt. Zur Kontrasterhöhung wurde mit Fast Green gegengefärbt. Das Ausmaß an Leberfibrose wurde als Prozentsatz des mit Picrosiriusrot anfärbbaren Teils in jedem Schnitt ermittelt. Zur automatisierten Morphometrie wurde ein Leica-Quantimed 500 MC-System (Leica, Deutschland) verwendet. Dabei wurden die Parameter der Farbdetektion standardisiert und während eines Experiments konstant gelassen. Zur Auswertung wurden 64 Beobachtungsfelder, die mittels eines Standardmeßgitters, das 31 mm² überdeckt, bei 100-facher Vergrößerung herangezogen.

### b) Kollagen-Gehalt:

Zur Abschätzung des Gesamtkollagen-Gehalts wurde die Gewebekonzentration an 4-Hydroxyprolin herangezogen. Es wurde die Methode von Prockop und Udenfried [D.J. Prockop and S. Udenfried, A specific method for the analysis of hydroxyproline in tissues and urine, Anal. Biochem. 1, 228-239 (1960)] in modifizierter Form angewendet: Leberproben mit einem Naßgewicht von 60-90 mg wurden getrocknet und anschließend in 6 N Salzsäure bei 100°C über 17 Stunden hydrolysiert. Das Hydrolysat wurde durch Eindampfen getrocknet und in 5 ml entionisiertem Wasser rekonstituiert. 200 µl dieses Hydrolysats wurden mit 200 µl Ethanol versetzt und für 20 Minuten bei Raumtemperatur mit 200 µl einer 0.7%-igen Lösung von Chloramin-T in Citratpuffer (5.7 g Natriumacetat, 3.75 g Citronensäure-Trinatriumsalz, 0.55 g Citronensäure, 38.5 ml Ethanol, auf 100 ml mit Wasser aufgefüllt) oxidiert. Hierzu wurden danach 400 µl Ehrlichs-Reagenz (12 g p-Dimethylaminobenzaldehyd und 2.7 g Schwefelsäure in 40 ml Ethanol) gegeben. Nach 3 Stunden Inkubation bei 35°C wurde die Absorption bei 573 nm gemessen. Der Gehalt an Hydroxyprolin, bezogen auf das Trockengewicht der eingesetzten Leberproben, wurde anhand einer Eichkurve aus diesen Absorptionswerten ermittelt.

### 2.3 Ergebnisse:

Wie Tabelle 2 zeigt, reduzieren die erfindungsgemäße Verbindungen den Anteil an Narbenkollagen in Ratten mit durch Gallengangsligatur erzeugter Leberfibrose. Aus Tabelle 3 ist ersichtlich, dass auch durch Tetrachlorkohlenstoff erzeugte Fibrose im Tiermodell durch erfindungsgemäße Verbindungen gehemmt wird, da sich unter Substanzgabe der Gehalt an Gesamtleberkollagen verringert. In Tabelle 4 ist die Beeinflussung des Gesamtkollagens bei seruminduzierter Leberfibrose dargelegt.

**Tabelle 2: Beeinflussung des Anteils an fibrotischem Gewebe bei Leberfibrose infolge einer Gallengangsligatur in der Ratte**

| Tiere | intakt (Shamoperiert) | Gallengangsligiert | Gallengangsligiert |
|---|---|---|---|
| Substanzgabe | Solutol/Ethanol/ PBS | Solutol/Ethanol/ PBS | 15 mg/kg Bsp. 82 p.o. b.i.d. in Tolutol/ Ethanol/ PBS |
| Anteil Picrosiriusrot-färbbare | | | |
| Fläche: Mittelwert in % | 0.21 | 7.4 | 4.98 |
| S.E.M. | 0.03 | 0.72 | 0.58 |
| N (Anzahl Tiere) | 5 | 11 | 11 |
| P | | | < 0.05 vs. Vehikel |

Dieser Effekt entspricht einer Hemmung der Fibrose um ca. 34 %.

**Tabelle 3: Beeinflussung des Gesamtkollagen bei Leberfibrose infolge einer chronischen Tetrachlorkohlenstoff-Gabe in der Ratte**

| Tiere | intakt | CCl₄ | CCl₄ |
|---|---|---|---|
| Substanzgabe | Solutol/Ethanol/ PBS | Solutol/Ethanol/ PBS | 30 mg/kg Bsp. 82 p.o. o.d. in Solutol/Ethanol/PBS |
| Gehalt an 4-Hydroxyprolin: Mittelwert in mg/g Trockengewicht | 0.504 | 1.921 | 1.260 |
| S.E.M. | 0.019 | 0.308 | 0.121 |
| N (Anzahl Tiere) | 5 | 11 | 11 |
| P | | | = 0.059 vs. Vehikel |

**Tabelle 4: Beeinflussung des Gesamtkollagens bei seruminduzierter Leberfibrose in der Ratte**

| Tiere | intakt | Serumbehandelt | Serumbehandelt |
|---|---|---|---|
| Substanzgabe | Solutol/Ethanol/ PBS | Solutol/Ethanol/ PBS | 3 mg/kg Bsp. 34 p.o. o.d. in Solutol/Ethanol/PBS |
| Gehalt an 4-Hydroxyprolin: Mittelwert in mg/g Trockengewicht | 0.577 | 2.070 | 1.293 |
| S.E.M. | 0.038 | 0.188 | 0.180 |
| N (Anzahl Tiere) | 5 | 15 | 15 |
| P | < 0.001 | | < 0.01 |

### B. Beispiele

### Abkürzungen:

- APCI: Atmosphärendruck - Chemische Ionisation (bei MS)
- Boc: tert.-Butoxycarbonyl
- DC: Dünnschichtchromatographie
- DCI: direkte chemische Ionisation (bei MS)
- DMAP: 4-*N,N* Dimethylaminopyridin
- DMF: *N,N* Dimethylformamid
- DMSO: Dimethylsulfoxid
- EDC: N'-(3-Dimethylaminopropyl)-N-ethylcarbodiimid x HCl
- EI: Elektronenstoß-Ionisation (bei MS)
- eq: Äquivalent(e)
- ESI: Elektrospray-Ionisation (bei MS)
- HOBt: 1-Hydroxy-1H-benzotriazol x H₂O
- HPLC: Hochdruck-, Hochleistungsflüssigchromatographie
- LC-MS: Flüssigchromatographie-gekoppelte Massenspektroskopie
- MOM: Methoxymethyl
- NMR: Kernresonanzspektroskopie
- R_{f}: Retentionsindex (bei DC)
- Rₜ: Retentionszeit (bei HPLC)
- Schmp.: Schmelzpunkt
- Zers.: Zersetzung

### Analytische Methoden:

Massenspektroskopische Methoden:
A: DCI, NH₃;
B: ESI;
C: ESIpos;
D: LC-MS/ESIpos;
E: APCI;
F: LC-MS

### HPLC-Standardmethode:

Säule: Kromasil C-18 125 x 2 mm; Fluß: 0.5 ml/min; Wellenlänge: 210 nm; Temp.: 30°C; Eluent: Gradienten von CH₃CN / 0.01 M H₃PO₄

### Allgemeine Herstellungsverfahren:

### Allgemeine Vorschrift (1)

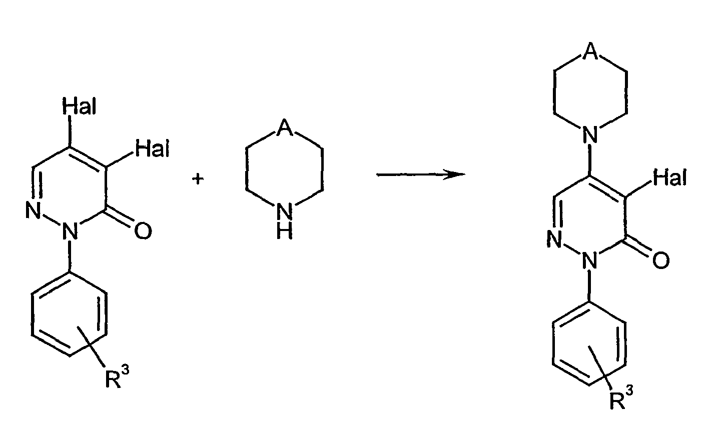

### Variante (1a)

Zu einer Suspension aus 4,5-Dihalopyridazin-3-on-Derivat (1 eq.) in 1,2-Dichlorethan (0.4-0.5 mol/l) werden Amin (1.8-2.0 eq.) und als Hilfsbase Triethylamin (1.5 eq.) gegeben. Die Mischung wird für 15-20 h auf Rückfluß erhitzt und nach Abkühlen mit Dichlormethan verdünnt. Es wird mit 0.5 N Salzsäurelösung und gesättigter Natriumchlorid-Lösung gewaschen, über Magnesiumsulfat getrocknet und eingeengt. Das Rohprodukt wird durch Kristallisation aus Dichlormethan / Diethylether-Gemischen oder durch Chromatographie an Silicagel (Dichlormethan / Methanol-Gemische) gereinigt.

### Variante (1b):

Zu einer Lösung von 4,5-Dihalopyrazin-3-on-Derivat (1 eq.) in N-Methyl-2-pyrrolidin (NMP) werden Amin (1-3 eq.) und Natriumiodid (8-10 Mol-%; katalytische Menge) gegeben und 4-8 h bei 65°C gerührt. Nach Abkühlen gibt man Wasser hinzu und saugt das ausgefallene Produkt ab. Der Filterrückstand wird in Diethylether verrührt, abgesaugt und im Hochvakuum getrocknet.

### Variante (1c): Dichlormethan- bzw. Dioxan-Verfahren

Zu einer Suspension von 4,5-Dichlorpyrazin-3-on-Derivat (1 eq.) in Dichlormethan oder Dioxan (im Falle von Dioxan erfolgt Zugabe katalytischer Mengen von Natriumiodid) wird das Amin (1-7 eq.) langsam zugeschüttet und 20 h zum Rückfluß erhitzt. Nach Abkühlen wird mit 1 molarer Natronlauge gewaschen, die Dichlormethan-Phase mit Natriumsulfat getrocknet und eingeengt. Im Dioxan-Fall wird das Lösemittel abdestilliert und der Rückstand in Dichlormethan/Wasser verteilt, die organische Phase abgetrennt und eingeengt. Durch Verrühren des Rückstandes mit Ether erfolgt Kristallisation.

### Allgemeine Vorschrift (2)

### (Vorschrift zur Acylierung von Amin-/Anilin-Fragmenten in R¹, R² und R³)

### Beispiel:

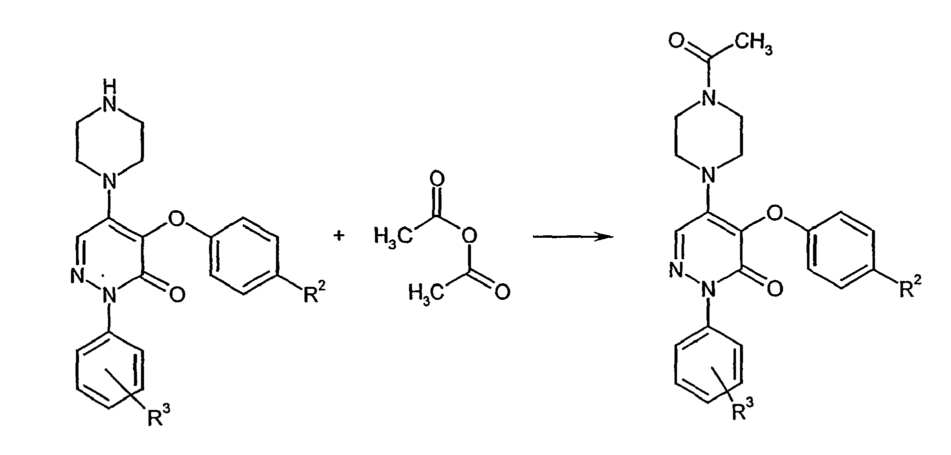

Zu einer eisgekühlten Lösung eines Amin- oder Anilin-Derivats (1 eq.) in absolutem 1,2-Dichlorethan (0.25-0.5 mol/l) wird tropfenweise Acylierungsreagenz (Anhydrid oder Säurechlorid, 1.2-2.0 eq.) gegeben. Nach vollständigem Umsatz (gegebenenfalls erfolgt Zusatz einer katalytischen Menge 4-DMAP) wird die auf Raumtemperatur erwärmte Reaktionsmischung mit Dichlormethan verdünnt. Es wird mit 1 N Salzsäurelösung, gesättigter Natriumhydrogencarbonat-Lösung und gesättigter Natriumchlorid-Lösung gewaschen, über Magnesiumsulfat getrocknet und eingeengt. Das Rohprodukt wird durch Kristallisation aus Dichlormethan / Diethylether-Gemischen oder durch Chromatographie an Silicagel (Dichlormethan / Methanol-Gemische) gereinigt.

### Allgemeine Vorschrift (3)

### (Vorschrift zur Formylierung von Amin-/Anilin-Fragmenten in R¹, R² und R³)

### Beispiel:

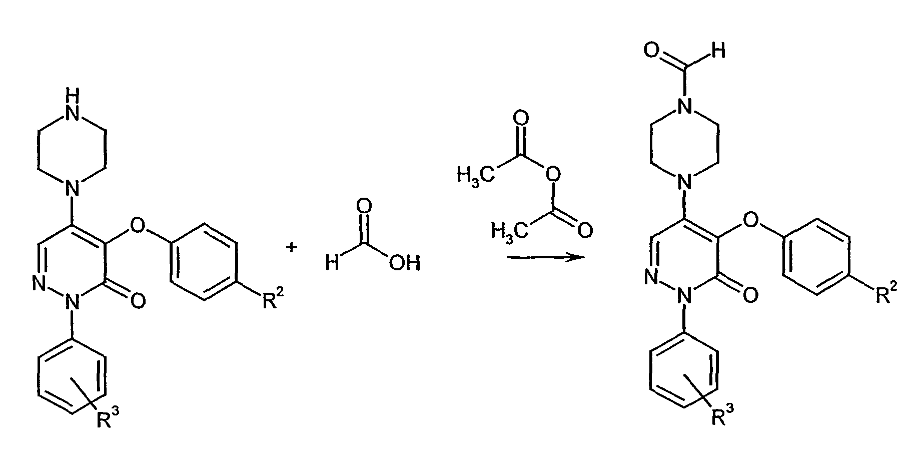

Eine Mischung aus Ameisensäure (2.5 eq.) und Acetanhydrid (2.0 eq.) wird 1 h auf 50°C erhitzt, nach Abkühlen mit absolutem Dichlormethan (0.5 mol/l) verdünnt und zu einer Mischung aus Amin- oder Anilin-Derivat (1.0 eq.) und Pyridin (3.0 eq.) in Dichlormethan (0.5 mol/l) gegeben. Die Reaktionsmischung wird mehrere Stunden bei Raumtemperatur gerührt, bevor mit Dichlormethan verdünnt, mit Wasser und gesättigter Natriumhydrogencarbonat-Lösung gewaschen, über Magnesiumsulfat getrocknet und eingeengt wird. Das Rohprodukt wird gegebenenfalls durch Kristallisation aus Dichlormethan / Diethylether-Gemischen gereinigt.

### Allgemeine Vorschrift (4)

### (Vorschrift zur Mesylierung von Amin-/Anilin-Fragmenten in R¹, R² und R³)

### Beispiel:

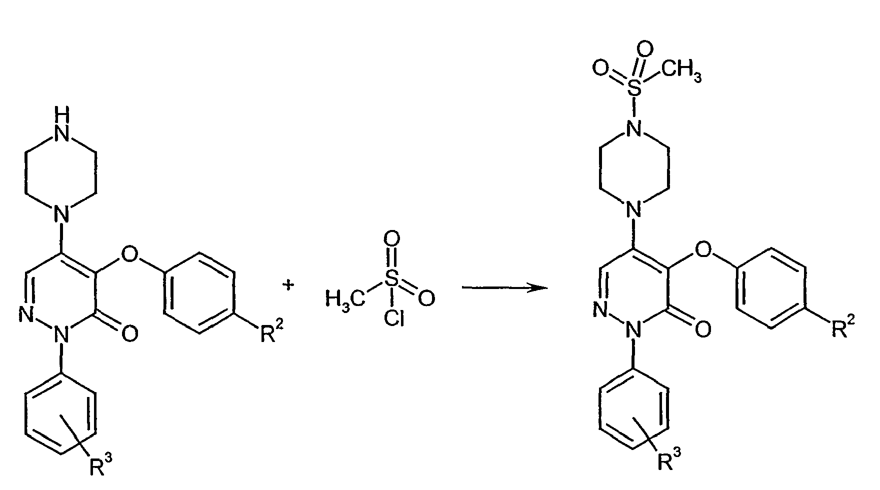

Zu einer eisgekühlten Lösung eines Amin- oder Anilin-Derivats (1 eq.) und einer Hilfsbase (Pyridin oder Triethylamin, 2.0-3.0 eq.) in absolutem Dichlormethan (0.25-0.5 mol/l) wird tropfenweise Methansulfonsäurechlorid (1.2-2.0 eq.) gegeben. Nach vollständigem Umsatz wird die auf Raumtemperatur erwärmte Reaktionsmischung mit Dichlormethan verdünnt. Es wird mit 1 N Salzsäurelösung, gesättigter Natriumhydrogencarbonat-Lösung und gesättigter Natriumchlorid-Lösung gewaschen, über Magnesiumsulfat getrocknet und eingeengt. Das Rohprodukt wird durch Kristallisation aus Dichlormethan / Diethylether-Gemischen oder durch Chromatographie an Silicagel (Dichlormethan / Methanol-Gemische) gereinigt.

### Allgemeine Vorschrift (5)

### (Vorschrift zur Carbamoylierung von Amin-/Anilin-Fragmenten in R¹, R² und R³)

### Beispiel:

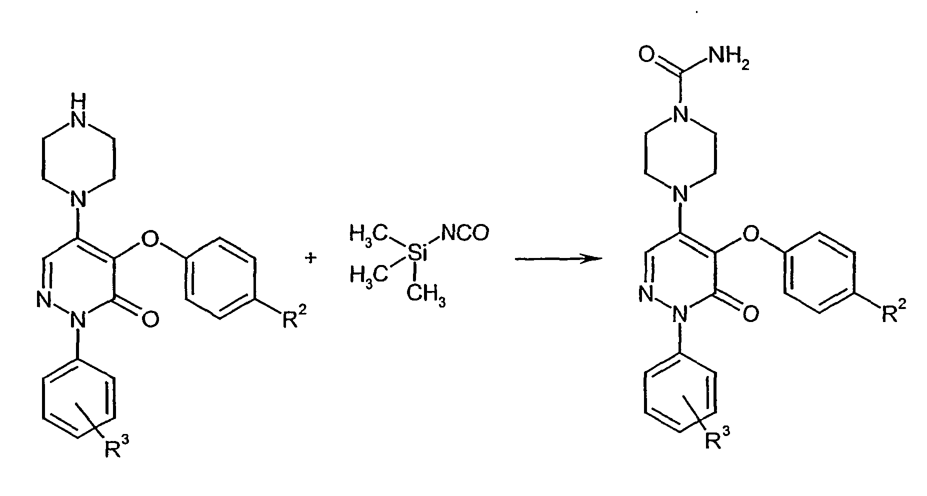

Trimethylsilylisocyanat (8.0 eq.) wird zu einer Mischung eines Amin- oder Anilin-Derivats (1 eq.) in absolutem Dichlormethan getropft. Die Reaktionsmischung wird über Nacht bei Raumtemperatur gerührt, anschließend eingeengt und im Hochvakuum getrocknet und der Rückstand mit Dichlormethan aufgenommen. Die Lösung wird mit Wasser, 2 N Kaliumcarbonat- und gesättigter Natriumchlorid-Lösung gewaschen, über Magnesiumsulfat getrocknet und eingeengt. Der Rückstand wird durch Chromatographie an Silicagel oder präparative Dünnschichtchromatographie (Dichlormethan / Methanol-Gemische) gereinigt.

### Allgemeine Vorschrift (6)

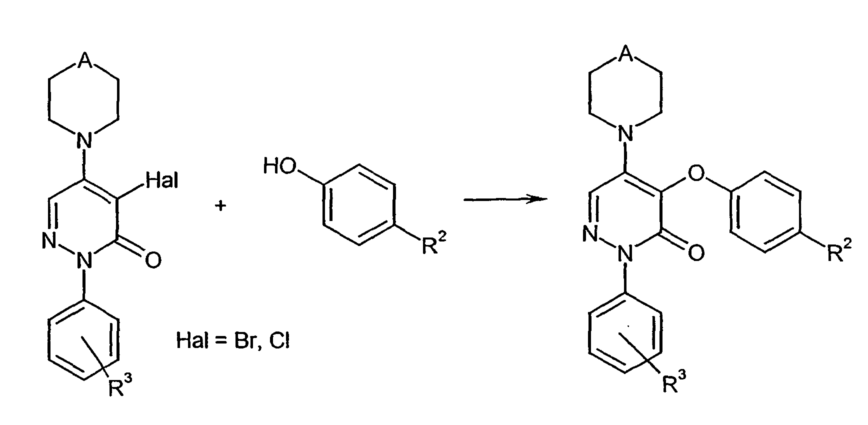

### Variante (6-a), Kaliumsalz-Methode:

In eine Lösung von Kalium-tert.-butylat (3.0 eq.) in absolutem Tetrahydrofuran (0.5 mol/l) wird portionsweise ein Phenol-Derivat (3.0 eq.) eingetragen. Nach 30 min bei Raumtemperatur wird die Lösung konzentriert und der Rückstand im Hochvakuum getrocknet. Zum getrockneten Kaliumphenolat werden 4-Halopyridazin-3-on-Derivat (1.0 eq.), Kaliumiodid (0.1 eq.) und absolutes Dimethylformamid (0.5 mol/l) gegeben. Die Mischung wird für 20-30 min in einem 170°C heißem Ölbad kräftig gerührt, bevor nach Abkühlen das Lösungsmittel im Hochvakuum entfernt wird. Der Rückstand wird in Dichlormethan aufgenommen und mit 1 N Natriumhydroxid-Lösung (zweimal) und gesättigter Natriumchlorid-Lösung gewaschen, über Magnesiumsulfat getrocknet und eingeengt. Das Rohprodukt wird durch Kristallisation aus Dichlormethan / Diethylether-Gemischen oder durch Chromatographie an Silicagel (Dichlormethan / Methanol-Gemische) gereinigt.

### Variante (6-b), Caesiumsalz-Methode:

In eine Suspension von Caesiumcarbonat (1.5 eq.) in absolutem Methanol (0.5 mol/l) wird portionsweise ein Phenol-Derivat (3.0 eq.) eingetragen. Nach 1 h bei Raumtemperatur wird die klare Lösung konzentriert und der Rückstand im Hochvakuum getrocknet. Zum getrockneten Caesiumphenolat werden 4-Halopyridazin-3-on-Derivat (1.0 eq) und absolutes Dimethylformamid (ca. 0.3 mol/l) gegeben. Die Mischung wird über Nacht bei 120°C kräftig gerührt, bevor nach Abkühlen das Lösungsmittel im Hochvakuum entfernt wird. Der Rückstand wird in Dichlormethan aufgenommen und mit Wasser, 1 N Natriumhydroxid-Lösung und gesättigter Natriumchlorid-Lösung gewaschen, über Magnesiumsulfat getrocknet und eingeengt. Das Rohprodukt wird durch Kristallisation aus Dichlormethan / Diethylether-Gemischen oder durch Chromatographie an Silicagel (Dichlormethan / Methanol-Gemische) gereinigt.

### Variante (6-c), über 4-Aryloxy-5-halo-pyridazin-3-on-Derivate:

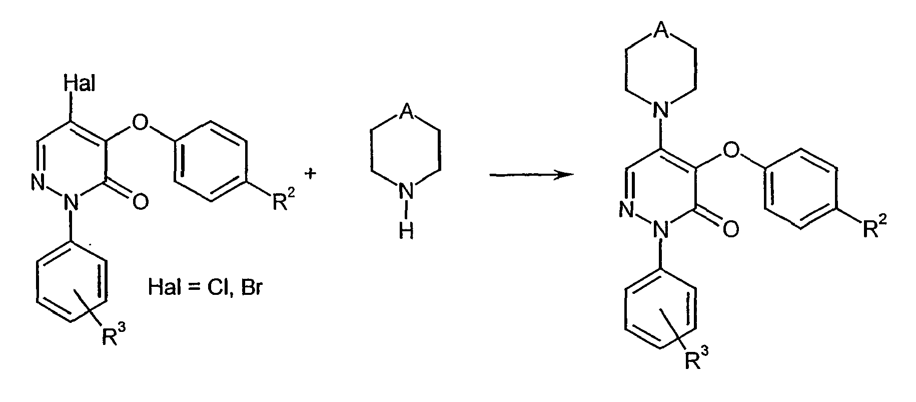

Eine Mischung aus einem 4-Aryloxy-5-halo-pyridazin-3-on-Derivat (1.0 eq., Herstellung siehe Vorschrift **12**) und einem cyclischen Aminderivat (1.2 bis 1.5 eq.), Kaliumiodid (1.0 eq.) und Ethyldiisopropylamin (2.0 eq.) in absolutem Dimethylformamid wird für 8 bis 18 h auf 120°C bis 140°C erhitzt. Nach dem Abkühlen wird im Hochvakuum eingeengt und der Rückstand in Dichlormethan aufgenommen. Man wäscht mit verdünnter Salzsäurelösung und gesättigter Natriumchlorid-Lösung, trocknet über Magnesiumsulfat und konzentriert im Vakuum. Aus der Rohmischung wird das Produkt durch Chromatographie an Silicagel (Dichlormethan / Methanol-Gemische) oder durch Kristallisation aus Diethylether bzw. Diethylether / Dichlormethan-Gemischen erhalten.

### Variante (6-d), Umsetzung in der Schmelze [Variante der Methode (6a)]:

Das Kaliumsalz eines Phenols wird mit 4-Halopyrazin-3-on-Derivaten (1 eq.) in einem auf 175°C vorgeheizten Ölbad 30 min in DMF gerührt. Nach Abkühlen auf Raumtemperatur wird mit Dichlormethan verdünnt und mit 1 molarer Natronlauge gewaschen. Die organische Phase wird mit Natriumsulfat getrocknet und eingeengt. Das Rohprodukt wird durch Kristallisation oder durch Chromatographie an Silicagel (Toluol / Acetonitril-Gemische) gereinigt.

### Allgemeine Vorschrift (7)

### (Vorschrift zur Freisetzung von Amin-/Anilin-Fragmenten in R¹, R² und R³ aus Bocgeschützten Vorläufern)

### Beispiel:

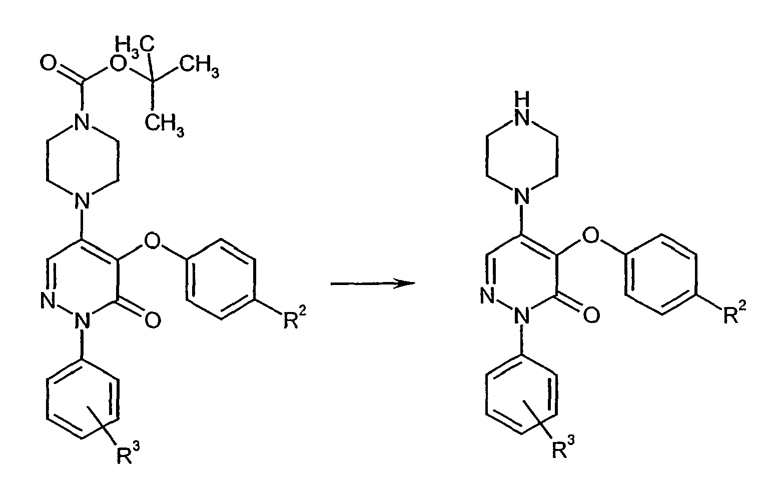

Eine eisgekühlte Mischung aus tert.-Butyloxycarbonat (Boc)-geschütztem Amin (1.0 eq.) in Dichlormethan (ca. 0.15 mol/l, Zusatz von 2% Wasser) wird mit Trifluoressigsäure (60% der Menge an Dichlormethan) versetzt. Die Mischung wird ca. 3 h kräftig bei Raumtemperatur gerührt, bevor eingeengt und im Hochvakuum getrocknet wird. Der Rückstand wird in Dichlormethan aufgenommen, mit gesättigter Natriumhydrogencarbonat-Lösung und gesättigter Natriumchlorid-Lösung gewaschen, über Magnesiumsulfat getrocknet und eingeengt. Das Rohprodukt wird durch Kristallisation aus Dichlormethan / Diethylether-Gemischen oder durch Chromatographie an Silicagel (Dichlormethan / Methanol-Gemische) gereinigt.

### Allgemeine Vorschrift (8)

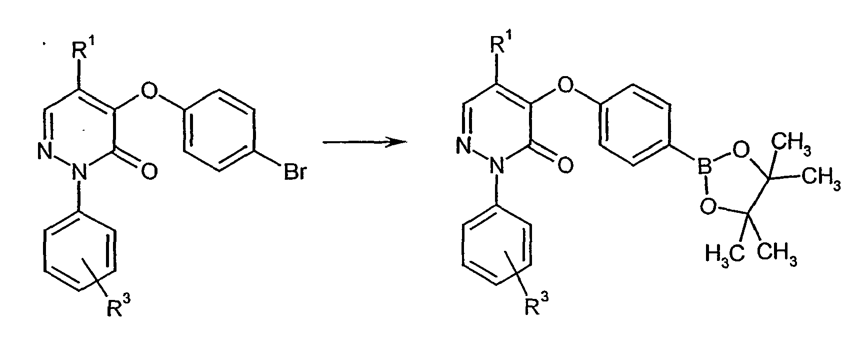

In einem mit Argon gespülten Kolben werden nacheinander Arlylbromid (1.0 eq., Herstellung siehe Vorschrift **6**), Bis(pinacolato)diboron (1.1 eq.), Kaliumacetat (3.0 eq.), DMSO und PdCl₂(dppf) (0.04 eq.) gegeben. Die Mischung wird unter Argon bei 80°C kräftig gerührt. Nach Ende des Umsatzes (Kontrolle per HPLC) wird abgekühlt, mit Dichlormethan verdünnt und zweimal mit Wasser gewaschen. Man trocknet über Magnesiumsulfat und engt ein. Das Rohprodukt wird durch Chromatographie an Silicagel (Dichlormethan / Methanol-Gemische) gereinigt, gegebenenfalls erfolgt Kristallisation aus Dichlormethan / Diethylether-Gemischen.

### Allgemeine Vorschrift (9)

### Beispiel:

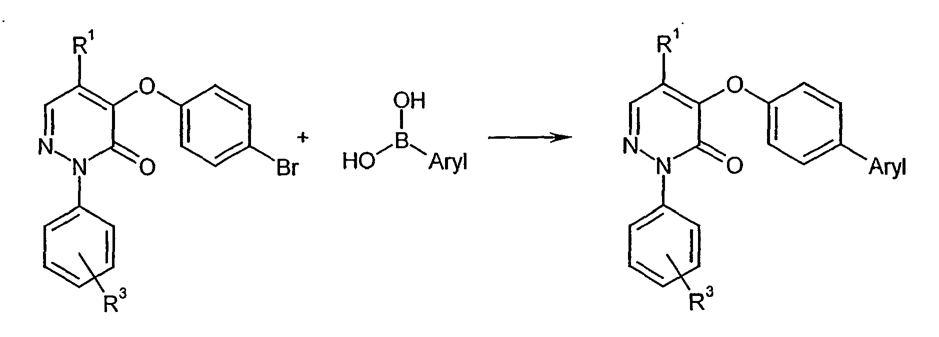

### Variante (9-a), über Boronsäure-Derivate:

In einem mit Argon gespülten Kolben werden nacheinander Arylbromid (1.0 eq., Herstellung siehe Vorschrift **6**), Arylboronsäure-Derivat (1.2-2.0 eq.), PdCl₂(dppf) (0.05 eq.), Dimethylformamid (ca. 0.15 mol/l) und Natriumcarbonat (2.5 bis 5.0 eq., als 2 N wäßrige Lösung) gegeben. Die Mischung wird unter Argon bei 80°C kräftig gerührt (in der Regel über Nacht), bevor nach Abkühlen im Hochvakuum eingeengt wird. Der Rückstand wird entweder wäßrig aufgearbeitet oder an Silicagel (Dichlormethan / Methanol-Gemische) säulenfiltriert. Das Rohprodukt wird durch Kristallisation aus Dichlormethan / Diethylether-Gemischen, durch Chromatographie an Silicagel (Dichlormethan / Methanol-Gemische) oder durch präparative Dünnschichtchromatographie (Dichlormethan / Methanol-Gemische) gereinigt.

### Variante (9-b), über Borinatester-Derivate:

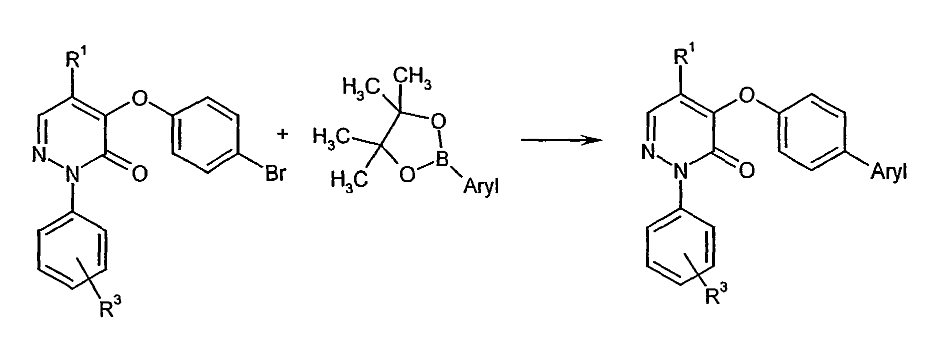

In einem mit Argon gespülten Kolben werden nacheinander Aryl-Bromid oder -Iodid (2.0 eq.), Arylborinatester-Derivat (1.0 eq., Herstellung siehe Vorschrift **8**), PdCl₂(dppf) (0.05 eq.), Dimethylformamid (ca. 0.15 mol/l) und Natriumcarbonat (5.0 eq., als 2 N wäßrige Lösung) gegeben. Die Mischung wird unter Argon bei 80°C kräftig gerührt (in der Regel über Nacht), bevor nach Abkühlen im Hochvakuum eingeengt wird. Der Rückstand wird entweder wäßrig aufgearbeitet oder an Silicagel (Dichlormethan / Methanol-Gemische) säulenfiltriert. Das Rohprodukt wird durch Kristallisation aus Dichlormethan / Diethylether-Gemischen, durch Chromatographie an Silicagel (Dichlormethan / Methanol-Gemische) oder durch präparative Dünnschichtchromatographie (Dichlormethan / Methanol-Gemische) gereinigt.

### Variante (9-c), über in situ generierte Borinatester-Derivate:

In einem mit Argon gespülten Kolben werden nacheinander Arylbromid (1.0 eq., Herstellung siehe Vorschrift **6**), Bis(pinacolato)diboron (1.1 eq.), Kaliumacetat (3.0 eq.), DMSO und PdCl₂(dppf) (0.04 eq.) gegeben. Die Mischung wird unter Argon bei 80°C kräftig gerührt. Nach Ende des Umsatzes (Kontrolle per HPLC) wird abgekühlt und filtriert (an dieser Stelle kann eine Aliquotierung bei parallelsynthetischen Ansätzen erfolgen). Zur erhaltenen Lösung werden unter Argon nacheinander Aryl-Bromid oder -Iodid (2.0 eq.), PdCl₂(dppf) (0.05 eq.) und Natriumcarbonat (5.0 eq., als 2 N wäßrige Lösung) gegeben. Die Reaktionsmischung wird unter Argon bei 80°C kräftig gerührt (in der Regel über Nacht), bevor nach Abkühlen Rohprodukte entweder durch wäßrige Aufarbeitung (Zusatz von Wasser und Dichlormethan mit anschließender Phasentrennung durch Chromaphil-Filter) oder durch Fällung nach Zusatz von Wasser erhalten werden. Die weitere Reinigung geschieht mittels Chromatographie an Silicagel (Dichlormethan / Methanol-Gemische), präparativer Dünnschichtchromatographie (Dichlormethan / Methanol-Gemische) oder präparativer RP-HPLC; gegebenenfalls erfolgt Kristallisation aus Dichlormethan / Diethylether-Gemischen.

### Allgemeine Vorschrift (10)

### (Vorschrift zur Freisetzung von Phenol-Fragmenten in R² und R³ aus MOM-geschützten Vorläufern)

### Beispiel:

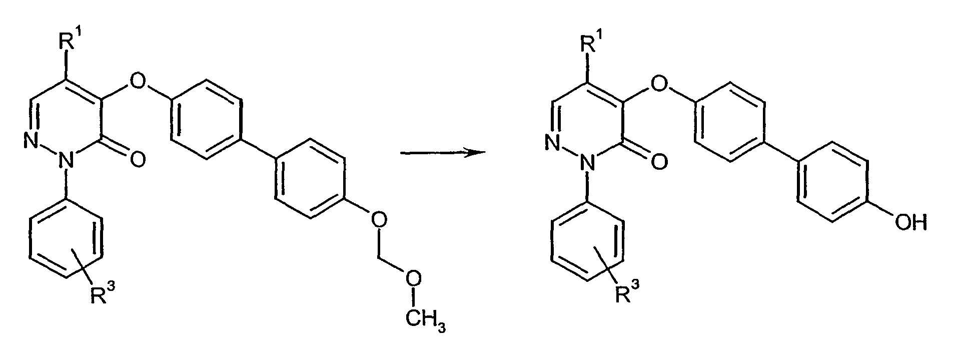

Eine Mischung eines Methoxymethyl (MOM)-geschützten Phenol-Derivats in einem Gemisch aus Essigsäure, Wasser und Trifluoressigsäure (3:1:1, ca. 0.1 bis 0.2 mol/l) wird bei Raumtemperatur bis zum vollständigen Umsatz kräftig gerührt. Man engt die Reaktionsmischung ein und trocknet im Hochvakuum. Der Rückstand wird mit gesättigter Natriumhydrogencarbonat- und Natriumchlorid-Lösung gewaschen, über Magnesiumsulfat getrocknet und eingeengt. Das Produkt wird, falls erforderlich, durch Kristallisation aus Dichlormethan / Diethylether-Gemischen, durch Chromatographie an Silicagel (Dichlormethan / Methanol-Gemische) oder durch präparative Dünnschichtchromatographie (Dichlormethan / Methanol-Gemische) gereinigt.

### Allgemeine Vorschrift (11)

### (Vorschrift zur Veretherung von Phenol-Fragmenten in R² und R³)

### Beispiel:

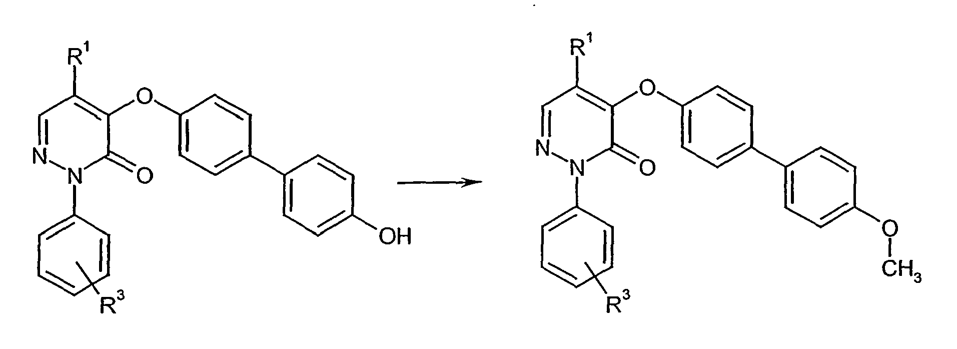

Zu einer Mischung eines Phenol-Derivats (1.0 eq.) und Kaliumcarbonat (2.0 eq.) in absolutem Tetrahydrofuran (ca. 0.1 mol/l) wird ein Überschuß Methyliodid (ca. 5.0 bis 10 eq.) getropft. Die Reaktionsmischung wird (in der Regel über Nacht) auf Rückfluß erhitzt, nach Abkäflüen bis zur Trockne eingeengt und mit Wasser versetzt. Das nach Filtration und Trocknen im Hochvakuum erhaltene Produkt wird gegebenenfalls durch Kristallisation aus Dichlormethan / Diethylether-Gemischen oder durch präparative Dünnschichtchromatographie (Dichlormethan / Methanol-Gemische) weiter aufgereinigt.

### Allgemeine Vorschrift (12)

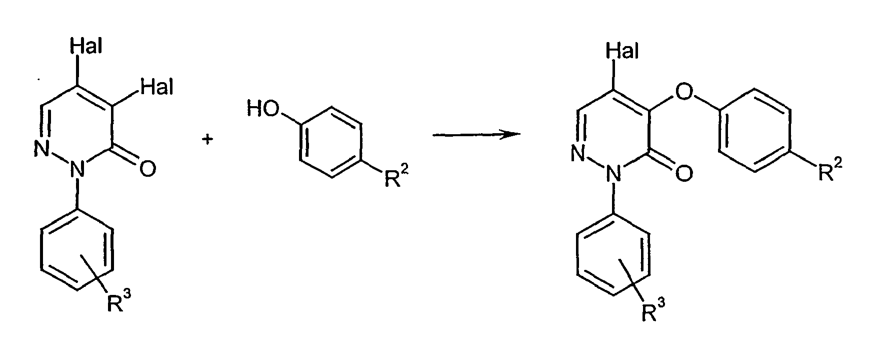

In eine wassergekühlte Lösung eines Phenol-Derivats (1.0 eq.) in absolutem 1,4-Dioxan (0.4 mol/l) wird unter kräftigem Rühren portionsweise Natriumhydrid (1.0 eq., 60%-ig in Mineralöl) eingetragen (bei größeren Ansätzen empfiehlt sich eine inverse Vorgehensweise). Nach 1 h wird die Lösung bei Raumtemperatur zu einer Suspension eines 4,5-Dihalopyridazin-3-on-Derivats (1.0 eq.) in absolutem 1,4-Dioxan (0.4 mol/l) gegeben. Die Reaktionsmischung wird 15-20 h bei Raumtemperatur kräftig gerührt, bevor das Dioxan im Vakuum nahezu vollständig entfernt wird. Der Rückstand wird in Dichlormethan aufgenommen, mit Wasser und gesättigter Natriumchlorid-Lösung gewaschen, über Magnesiumsulfat getrocknet und konzentriert. Aus dem Gemisch wird das Produkt durch Kristallisation aus Dichlormethan oder durch Chromatographie an Silicagel (Dichlormethan / Cyclohexan-Gemische) erhalten.

### Allgemeine Vorschrift (13)

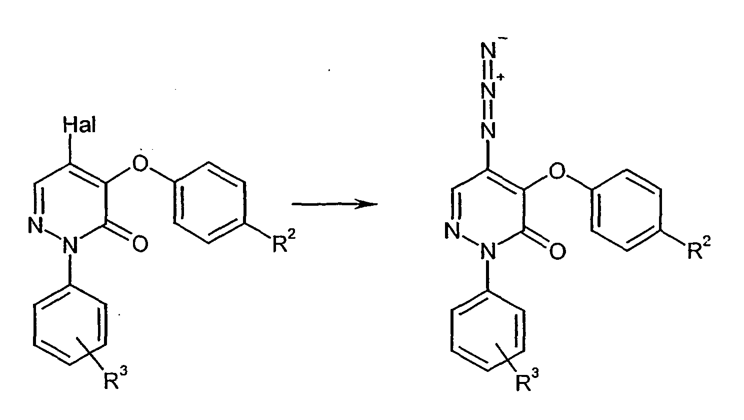

Eine Suspension aus einem 5-Halopyridazin-3-on-Derivat (1.0 eq., Herstellung siehe Vorschrift **12**) und Natriumazid (5.0 eq.) in Dimethylformamid (0.5 mol/l) wird 30 bis 45 min auf 50-55°C erhitzt. Nach dem Abkühlen wird mit Dichlormethan verdünnt, mit Wasser und gesättigter Natriumchlorid-Lösung gewaschen, über Magnesiumsulfat getrocknet und vorsichtig eingeengt. Der am Hochvakuum getrocknete Rückstand wird mit Dichlormethan / Diethylether kristallisiert.

### Allgemeine Vorschrift (14)

### (Vorschrift zur 1,2,3-Triazolsynthese)

### Beispiel:

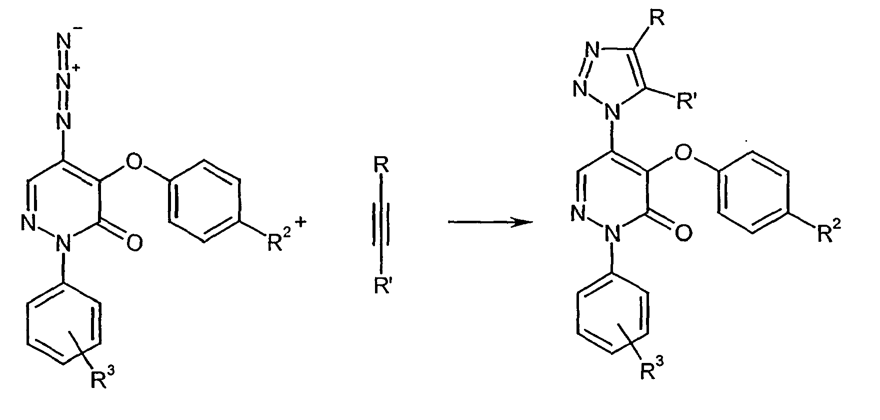

Eine Suspension eines 5-Azidopyridazin-3-on-Derivat (1.0 eq., Herstellung siehe Vorschrift **13**) und eines mono- oder disubstituierten Alkinderivats (2.0 bis 8.0 eq.) in Toluol (0.1 bis 0.4 mol/l) wird auf Rückfluß erhitzt (2 bis 24 h). Nach dem Abkühlen wird die Reaktionsmischung eingeengt und das Produktgemisch durch Kristallisation aus Diethylether bzw. Diethylether / Dichlormethan-Gemischen und Chromatographie an Silicagel (Dichlormethan / Methanol-Gemische) in gegebenenfalls anfallende Regioisomere getrennt und gereinigt.

### Allgemeine Vorschrift (15)

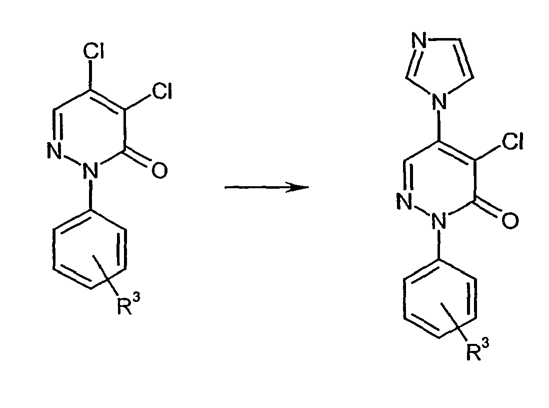

### Variante (15a), 4-Chlor-5-(1H-imidazol-1 -yl)-2-aryl-3(2H)-pyridazinone:

25 mmol 4,5-Dichlor-2-aryl-2(3H)-pyridazinon, 75 mmol Imidazol und 0.7 mmol Natriumiodid in 100 - 150 ml Dimethylformamid werden 8 h bei 90°C gerührt (DC-Kontrolle). Nach Abkühlen auf Raumtemperatur wird mit Wasser verdünnt und mehrmals mit Dichlormethan extrahiert. Die organische Phase wird mit Wasser gewaschen und über Natriumsulfat getrocknet. Das nach Einengen am Rotationsverdampfer verbleibende Rohprodukt wird an Kieselgel (0.063-0.2 mm) chromatographiert. Überschüssiges Edukt wird zunächst mit Dichlormethan eluiert; das gewünschte Produkt erhält man durch Elution mit Petrolether / Ethylacetat 1:1. Nach Eindampfen erhält man die entsprechenden 4-Chlor-5-(1H-imidazol-1-yl)-2-aryl-3(2H)-pyridazinone.

### Variante (15b), 4-Brom-5-(1H-imidazol-1-yl)-2-aryl-3(2H)-pyridazinone:

Beim Einsatz von 4,5-Dibrom-2-aryl-2(3H)-pyridazinonen erhält man analog zu Methode (19a) die entsprechenden 4-Brom-Derivate.

Die entsprechenden 4,5-Dibrom- bzw. 4,5-Dichlor-2-aryl-2(3H)-pyridazinone sind bekannt oder können aus käuflichen Arylhydrazinen und Mucobrom- bzw. Mucochlorsäure unter sauren Bedingungen nach bekannten Verfahren hergestellt werden [H.R. Hensel und G. Lützel, Angewandte Chemie 77, 303 (1965].

### Allgemeine Vorschrift (16)

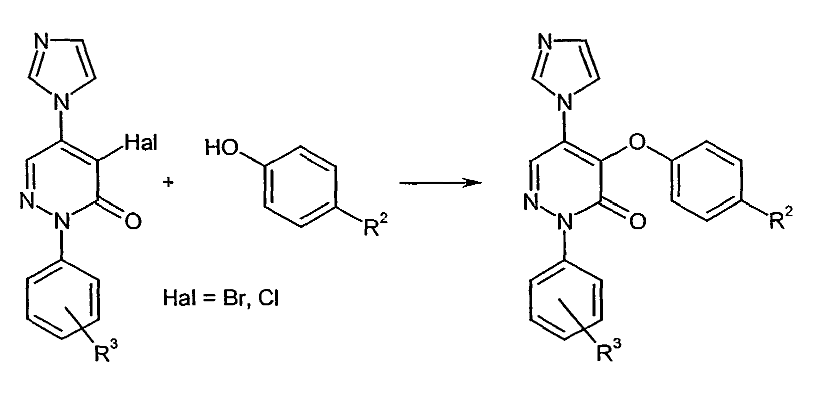

### Variante (16a):

10 mmol 4-Chlor- oder 4-Brom-5-(1H-imidazol-1-yl)-2-aryl-3(2H)-pyridazinon, 15.0 mmol Phenol, 0.1 g Natriumiodid und 12 mmol 1,4-Diazabicyclo[2.2.2]octan werden in 4 - 10 ml Dimethylformamid aufgenommen und über Nacht auf 100°C erhitzt. Nach Abkühlen wird mit Dichlormethan verdünnt. Die organische Phase wird mit verdünnter Salzsäure (pH 3), Wasser, 1 N Natronlauge und Wasser gewaschen. Der nach Trocknen über Natriumsulfat und Einengen verbleibende Rückstand wird mit Diethylether / Methanol 95:5 verrührt. Durch chromatographische Reinigung (Kieselgel 0.063-0.2 mm, Dichlormethan / Methanol 40:1) der Mutterlauge wird weitere Zielverbindung erhalten.

### Variante (16b):

1,4-Diazabicyclo[2.2.2]octan kann durch Natriumhydrid ersetzt werden.

### Variante (16c):

1,4-Diazabicyclo[2.2.2]octan kann durch Natriummethylat ersetzt werden. Das Natriumsalz wird analog zu Methode (6a) vorgebildet und das Lösemittel eingeengt.

### Allgemeine Vorschrift (17)

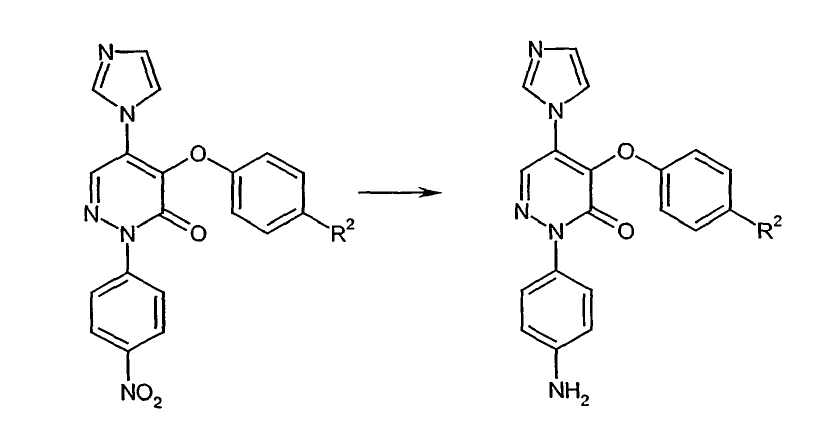

1.1 mmol 2-(4-Nitrophenyl)-5-(1H-imidazol-1-yl)-4-aryloxy-3(2H)-pyridazinon in 50 ml Eisessig werden in Gegenwart von 0.05 g Palladium auf Aktivkohle (10%-ig) bei Raumtemperatur und 3 bar hydriert. Nach 4 Stunden wird vom Katalysator abfiltriert, eingeengt und mit Dichlormethan aufgenommen. Die organische Phase wird mit 1 N Natriumhydrogencarbonat-Lösung und Wasser gewaschen. Nach Trocknen und Einengen liefert Chromatographie an Silicagel (0.063-0.2mm, Ethylacetat) das gewünschte 2-(4-Aminophenyl)- 3(2H)-pyridazinon.

Aus den nach den Allgemeinen Herstellungsverfahren erhaltenen Produkten können die entsprechenden Hydrochloride durch Behandeln mit Lösungen von Chlorwasserstoff in inerten Lösemitteln erhalten werden.

### Ausgangs- und Zwischenverbindungen:

### Beispiel 1A

### tert.-Butyl 4-[5-chlor-1-(4-chlorphenyl)-6-oxo-1,6-dihydro-4-pyridazinyl]-1-piperazin-carboxylat

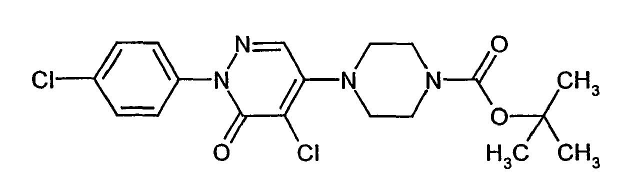

Gemäß allgemeiner Vorschrift **1** aus 20.66 g (75 mmol) 2-(4-Chlorphenyl)-4,5-dichlorpyridazin-3-on mit 25.14 g (135 mmol) Boc-Piperazin.
Ausbeute: 25.22 g (79.1% d.Th.)
MS (Methode A): m/z = 442 (M+NH₄)⁺, 425 (M+H)⁺
HPLC: Rₜ = 9.51 min.

### Beispiel 2A

### 4-Chlor-2-(4-chlorphenyl)-5-(1-piperazinyl)-3(2H)-pyridazinon

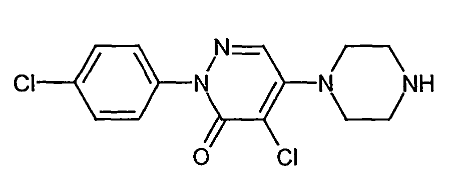

55.1 g (200 mmol) 2-(4-Chlorphenyl)-4,5-dichlorpyridazin-3-on werden mit 41.6 ml (300 mmol) Triethylamin in 400 ml Dimethylformamid vorgelegt. Man gibt 137.82 g (1600 mmol) Piperazin zu und rührt über Nacht bei 80°C nach. Die Reaktionsmischung wird nach Abkühlen im Hochvakuum eingeengt. Der Rückstand wird in Dichlormethan aufgenommen und zweimal mit Wasser gewaschen, über Magnesiumsulfat getrocknet und eingeengt. Das Produkt wird durch Chromatographie (Gradient: Dichlormethan/Methanol von 95:5 bis 9:1) an Silicagel und anschließender Kristallisation mit Ether isoliert.
Ausbeute: 40.9 g (62.9 % d.Th.)
MS (Methode A): m/z = 325 (M+H)⁺
HPLC: Rₜ = 5.38 min.

### Beispiel 3A

### 5-(4-Acetyl-1-piperazinyl)-4-chlor-2-(4-chlorphenyl)-3 (2H)-pyridazinon

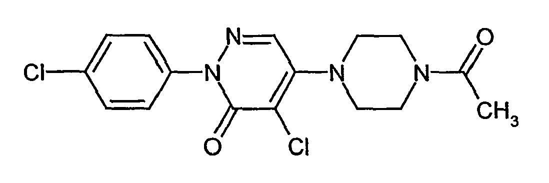

Gemäß allgemeiner Vorschrift 2 aus 9.76 g (30 mmol) der Verbindung aus Beispiel 2A mit 5.7 ml (60 mmol) Acetanhydrid erhältlich.
Ausbeute: 10.6 g (96.2 % d.Th.)
MS (Methode B): m/z = 367 (M+H)⁺
HPLC: Rₜ = 7.01 min.

### Beispiel 4A

### 5-(4-Acetyl-1-piperazinyl)-4-chlor-2-(4-methylphenyl)-3(2H)-pyridazinon

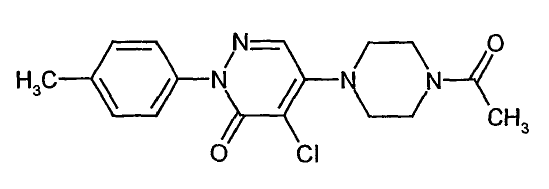

Gemäß allgemeiner Vorschrift **1** aus 10.0 g (39.2 mmol) 2-(4-Chlorphenyl)-4,5-dichlorpyridazin-3-on mit 8.2 g (64 mmol) N-Acetylpiperazin erhältlich.
Ausbeute: 7.86 g (57.8 % d.Th.)
MS (Methode F): m/z = 347 (M+H)⁺
HPLC: Rₜ = 6.67 min.

### Beispiel 5A

### 4-Chlor-2-(4-chlorphenyl)-5-[4-(cyaopropylcarbonyl)- 1 -piperazinyl]-3(2H)-pyridazinon

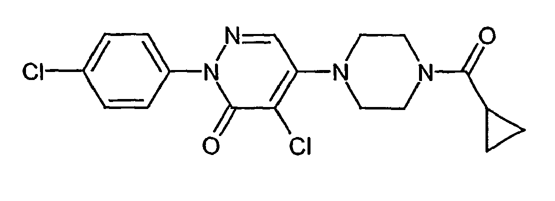

Gemäß allgemeiner Vorschrift **2** aus 9.76 g (30.0 mmol) der Verbindung aus Beispiel 2A mit 3.3 ml (36 mmol) Cyclopropylcarbonsärechlorid erhältlich.
Ausbeute: 11.22 g (95.1 % d.Th.)
MS (Methode F): m/z = 393 (M⁺)
HPLC:Rₜ = 7.72 min.

### Beispiel 6A

### 4-Chlor-2-(4-chlorphenyl)-5-[4-(methylsulfonyl)-1-piperazinyl]-3(2H)-pyridazinon

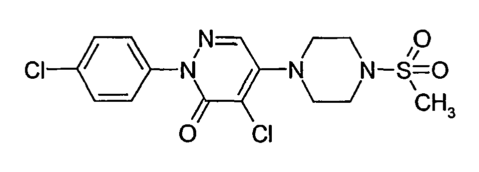

Gemäß allgemeiner Vorschrift **4** aus 10.0 g (30.75 mmol) der Verbindung aus Beispiel 2A mit 3.6 ml (46.1 mmol) Methansulfonsäurechlorid erhältlich.
Ausbeute: 11.1 g (89.5 % d.Th.)
MS (APCI): m/z = 403 (M+H)⁺
HPLC: Rₜ = 7.81 min.

### Beispiel 7A

### 4-Chlor-2-(4-chlorphenyl)-5-(3-oxo-1-piperazinyl)-3(2H)-pyridazinon

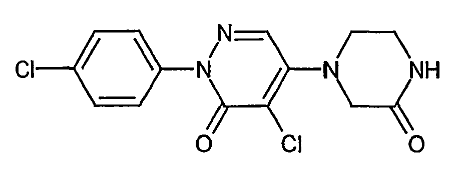

Gemäß allgemeiner Vorschrift **1** aus 4.68 g (17 mmol) 2-(4-Chlorphenyl)-4,5-dichlorpyridazin-3-on mit 3.06 g (30.6 mmol) erhältlich.
Ausbeute: 3.51 g (60.9 % d.Th.)
MS (EI-POS): m/z = 338 (M⁺)
HPLC: Rₜ = 6.42 min.

### Beispiel 8A

### 4,5-Bis(1,1'-biphenyl-4-yloxy)-2-(4-chlorphenyl)-3 (2H)-pyridazinon

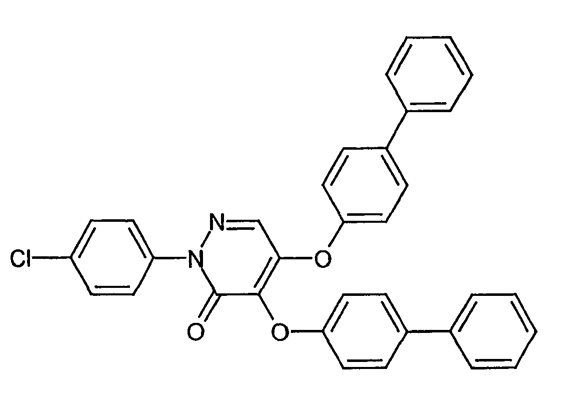

Zu einer Lösung von 44.9 g (400 mmol) Kalium-tert.-butylat in 680 ml absolutem Tetrahydrofuran werden portionsweise 68.09 g (400 mmol) Biphen-4-ol gegeben. Nach 15 min wird die Lösung eingeengt und gründlich am Hochvakuum getrocknet. Es werden 27.3 g (100 mmol) 2-(4-Chlorphenyl)-4,5-dichlorpyridazin-3-on, 8.30 g (50 mmol) Kaliumjodid und 300 ml absolutem Dimethylformamid hinzugefügt und die Mischung wird unter kräftigem Rühren für 4 h auf 140°C erhitzt. Nach Erkalten wird im Hochvakuum konzentriert und der Rückstand mit Dichlormethan aufgenommen. Man filtriert die Suspension über Celite und wäscht das Filtrat zweimal mit IN Natriumhydroxidlösung. Die organische Phase wird über Magnesiumsulfat getrocknet und eingeengt. Das Produkt wird durch Säulenfiltration an Silicagel (Dichlormethan/Cyclohexan 3:1) isoliert.
Ausbeute: 10.20 g (18.8 % d.Th.)
MS (Methode B): m/z = 543 (M+H)⁺
HPLC: Rₜ = 11.85 min.

### Beispiel 9A

### 4-(1,1'-Biphenyl-4-yloxy)-5-chlor-2-(4-chlorphenyl)-3 (2H)-pyridazinon

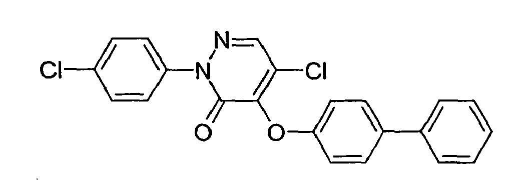

Gemäß allgemeiner Vorschrift **12** aus 22.04 g (80.0 mmol) 2-(4-Chlorphenyl)-4,5-dichlorpyridazin-3-on erhältlich.
Ausbeute: 19.54 g (59.7 % d.Th.)
MS (Methode B): m/z = 409 (M+H)⁺
HPLC: Rₜ = 10.95 min.

### Beispiel 10A

### 4-(4-Bromphenoxy)-5-chlor-2-(4-chlorphenyl)-3(2H)-pyridazinon

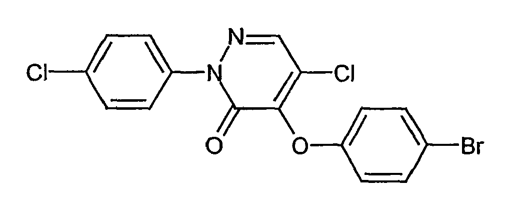

Gemäß allgemeiner Vorschrift **12** aus 27.55 g (100.0 mmol) 2-(4-Chlorphenyl)-4,5-dichlorpyridazin-3-on erhältlich.
MS (Methode B): m/z = 428/430 (M+NH₄)⁺
HPLC: Rₜ = 10.48 min.

### Beispiel 11A

### 5-Brom-2-(4-chlorphenyl)-4-[(4'-fluor-1,1'-biphenyl-4-yl)oxy]-3(2H)-pyridazinon

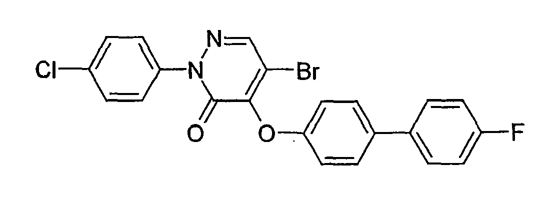

Gemäß allgemeiner Vorschrift **12** aus 2-(4-Chlorphenyl)-4,5-dibrompyridazin-3-on erhältlich.
HPLC: Rₜ = 10.96 min.

### Beispiel 12A

### 5-Chlor-2-(4-chlorphenyl)-4-{[4'-fluor-2'-(methoxymethoxy)-1,1'-biphenyl-4-yl]-oxy}-3(2H)-pyridazinon

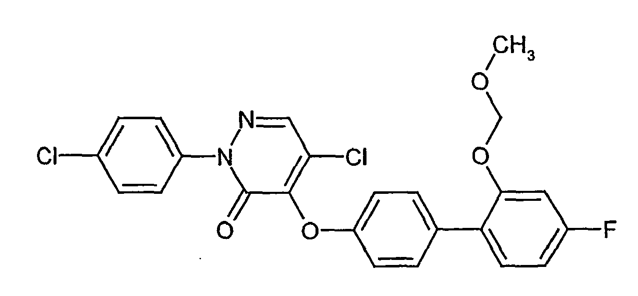

Gemäß allgemeiner Vorschrift **12** aus 1.82 g (6.6 mmol) 2-(4-Chlorphenyl)-4,5-dichlorpyridazin-3-on erhältlich.
Ausbeute: 1.84 g (57.3 % d.Th.)
MS (Methode A): m/z = 487/489 (M+H)⁺
HPLC: Rₜ = 10.91 min.

### Beispiel 13A

### 5-Chlor-2-(4-chlorphenyl)-4-[(2',4'-difluor-1,1'-biphenyl-4-yl)oxy]-3 (2H)-pyridazinon

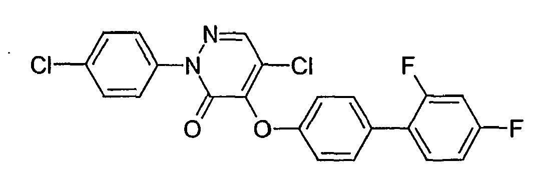

Gemäß allgemeiner Vorschrift **12** aus 5.51 g (20.0 mmol) 2-(4-Chlorphenyl)-4,5-dichlorpyridazin-3-on erhältlich.
Ausbeute: 5.07 g (56.9 % d.Th.)
MS (Methode A): m/z = 462 (M+NH₄)⁺
HPLC: Rₜ = 10.97 min.

### Beispiel 14A

### 5-Azido-4-( 1,1'-biphenyl-4-yloxy)-2-(4-chlorphenyl)-3 (2H)-pyridazinon

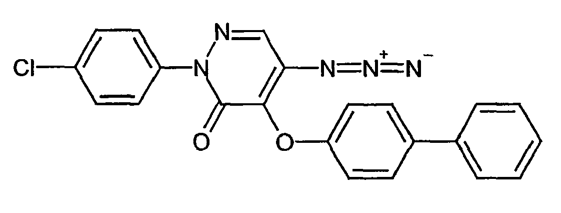

Gemäß allgemeiner Vorschrift **13** aus 2.05 g (5.0 mmol) der Verbindung aus Beispiel 9A mit 1.63 g (25.0 mmol) Natriumazid erhältlich.
Ausbeute: 0.998 g (48.0 % d.Th.)
MS (Methode B): m/z = 416 (M+H)⁺
HPLC: Rₜ = 10.74 min.

### Beispiel 15A

### 5-azido-4-(4-bromphenoxy)-2-(4-chlorphenyl)-3(2H)-pyridazinon

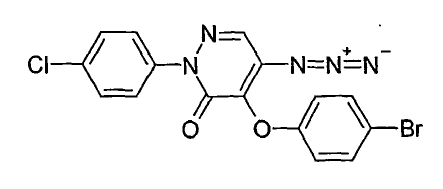

Gemäß allgemeiner Vorschrift **13** aus 7.55 g (18.3 mmol) der Verbindung aus Beispiel 10A mit 5.96 g (91.6 mmol) Natriumazid erhältlich.
Ausbeute: 3.73 g (48.6 % d.Th.)
MS (Methode A): m/z = 435/437 (M+H)⁺
HPLC: Rₜ = 10.25 min.

### Beispiel 16A

### 5-Azido-2-(4-chlorphenyl)-4-[(4'-fluor-1,1'-biphenyl-4-yl)oxy]-3 (2H)-pyridazinon

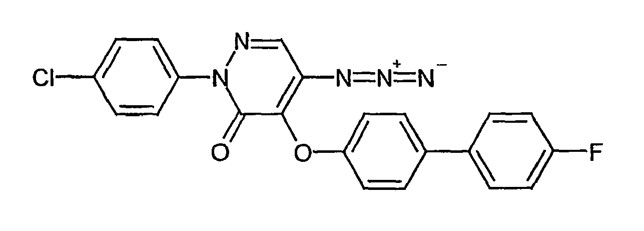

Gemäß allgemeiner Vorschrift **13** aus 800 mg (1.70 mmol) der Verbindung aus Beispiel 11A erhältlich.
Ausbeute: 455 mg (55.7 % d.Th.)
MS (Methode A): m/z = 451 (M+NH₄)⁺, 434 (M+H)⁺
HPLC: Rₜ = 10.70 min.

### Beispiel 17A

### 5-Azido-2-(4-chlorphenyl)-4-{[4'-fluor-2'-(methoxymethoxy)-1,1'-biphenyl-4-yl]-oxy}-3(2H)-pyridazinon

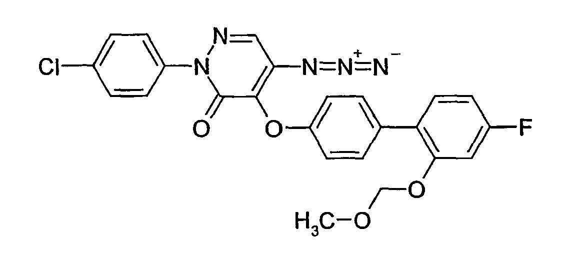

Gemäß allgemeiner Vorschrift **13** aus 305 mg (0.626 mmol) der Verbindung aus Beispiel 12A erhältlich.
Ausbeute: 118 mg (38.2 % d.Th.)
MS (Methode D): m/z = 494 (M+H)⁺
HPLC: Rₜ =10.74 min.

### Beispiel 18A

### 5-Azido-2-(4-chlorphenyl)-4-[(2',4'-difluor-1,1'-biphenyl-4-yl)oxy]-3 (2H)-pyridazinon

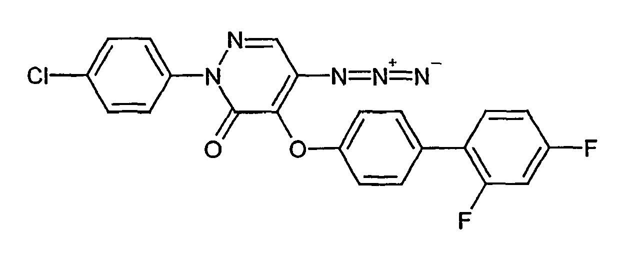

Gemäß allgemeiner Vorschrift **13** aus 890.5 mg (2.0 mmol) der Verbindung aus Beispiel 13A erhältlich.
Ausbeute: 379 mg (37.7 % d.Th.)
MS (Methode D): m/z = 452 (M+H)⁺
HPLC: Rₜ = 10.77 min.

### Beispiel 19A

### 4-(tert.-Butoxycarbonyl)-R-(-)-2-methyl-piperazin

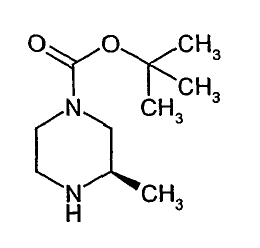

Zu einer Lösung von 3.6 g (35.94 mmol) R-(-)-2-Methylpiperazin in 50 ml Tetrahydrofuran werden 8.49 g (37.74 mmol) Di-tert.-butylpyrocarbonat und katalytische Mengen 4-Dimethylaminopyridin zugegeben und 24 h bei Raumtemperatur nachgerührt. Man engt bis zur Trockene ein, nimmt in Dichlormethan auf, wäscht dreimal mit Wasser und einmal mit Natriumchlorid-Lösung und engt die organische Phase ein.
Ausbeute: 4.62 g (64.2 % d.Th.)
MS (Methode A): m/z = 318 (M₂+H)⁺
DC: R_{f} = 0.29 (Dichormethan/Methanol 9:1)

### Beispiel 20A

### 4-(tert.-Butoxycarbonylamino)-1-carbethoxy-piperidin

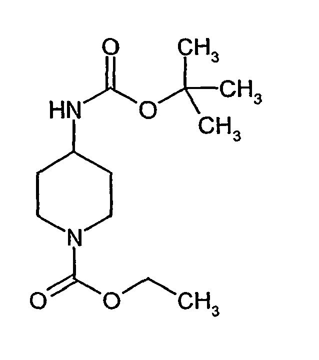

Zu einer Lösung von 10.2 g (58.06 mmol) 4-Amino-1-carbethoxy-piperidin in 250 ml Tetrahydrofuran werden bei 0°C 26.13 g (119.72 mmol) Pyrokohlensäure-di-tert.-butylester und katalytische Mengen von 4-Dimethylaminopyridin hinzugegeben. Man läßt auf Raumtemperatur kommen und rührt 2 h nach. Die Reaktionslösung wird eingeengt, der Rückstand in Dichlormethan gelöst und mit Wasser und Natriumchloridlösung gewaschen. Die organische Phase wird eingeengt und im Hochvakuum getrocknet.
Ausbeute: 20.2 g (127.7 % d.Th., Produkt enthält Lösemittel)
MS (Methode A): m/z = 290 (M+NH₄)⁺_{'} 273 (M+H)⁺

### Beispiel 21A

### 4-(tert.-Butoxycarbonylammo)-piperidin

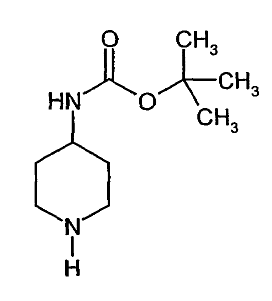

Zu einer Mischung von 32.33 g (0.576 mol) KOH in 175 ml Wasser und 175 ml Ethanol werden 19.91 g (0.073 mol) der Verbindung aus Beispiel 20A gegeben und 4 h zum Rückfluß erhitzt. Danach wird bei Raumtemperatur über Nacht nachgerührt, die Reaktionslösung mit 350 ml konz. Natriumchlorid-Lösung verdünnt und viermal mit insgesamt 1500 ml Ethylacetat extrahiert. Das organische Extrakt wird viermal mit je 100 ml Natriumchloridlösung gewaschen, über Natriumacetat getrocknet, eingeengt und im Hochvakuum getrocknet.
Ausbeute: 11.49 g (89.2 % d.Th.)
MS (Methode A): m/z = 201 (M+H)⁺

### Beispiel 22A

### 2-Chlor-2-(4-chlorphenyl)-5-(1,4-dioxa-5-aza-spiro[4.5]dec-8-yl)-2H-pyridazin-3-on

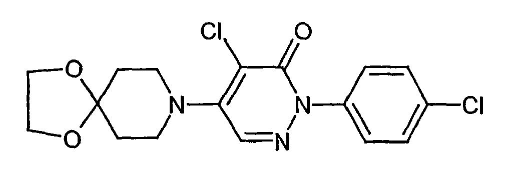

Zu einer Lösung von 20.0 g (72.59 mmol) 4,5-Dichlor-2-(4-chlorphenyl)-2-(3H)-pyridazinon in 200 ml N-Methyl-2-pyrrolidin werden nacheinander 25.98 g (0.181 mol) Piperidinon-4-ethylenketal 1.023 g (6.82 mmol) Natriumiodid gegeben, 4 h bei 65°C und über Nacht bei Raumtemperatur gerührt. Die Reaktionsmischung wird in viel Wasser eingerührt und das ausgefallene Produkt abgesaugt. Der Nutschenrückstand wird in Ether verrührt, abgesaugt und im Vakuum getrocknet.
Ausbeute: 25.36 g (91.4 % d.Th.)
MS (Methode B): m/z = 382 (M+H)⁺
HPLC: Rₜ = 8.49 min.

### Beispiel 23A

### 4-[5-Chlor-1-(4-chlor-phenyl)-6-oxo-1,6-dihydro-pyridazin-4-yl]-3-methylpiperazin-1-carbonsäure-tert.-butylester

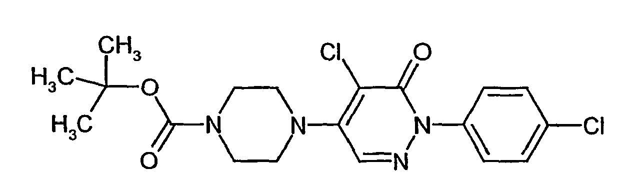

Gemäß allgemeiner Vorschrift **1c** werden zu einer Suspension von 10.09 g (30.3 mmol) 2-(4-Chlorphenyl)-4,5-dichlor-2(3H)-pyridazinon in 1000 ml Dioxan, 15.47 g (80.6 mmol) Piperazin-1-carbonsäure-tert.-butylester und 0.22 g (1.49 mmol) Natriumjodid hinzugefügt und über Nacht bei 100°C gerührt. Dioxan wird abdestilliert und der Rückstand in 200 ml Dichlormethan und 150 ml Wasser gelöst.

Die organische Phase wird getrocknet, eingeengt und 1 h mit 200 ml Ether verrührt. Das Kristallisat wird abgesaugt, mit Ether gewaschen und im Vakuum getrocknet.
Ausbeute: 9.6 g (62.2 % d.Th.)
MS (EI): m/z = 424 (M⁺)
HPLC: Rₜ = 9.54 min.

### Beispiel 24A

### 4-(4-Bromphenoxy)-2-(4-chlorphenyl)-5-[4-(2-hydroxyethyl)-1-piperazinyl]-3 (2H)-pyridazinon

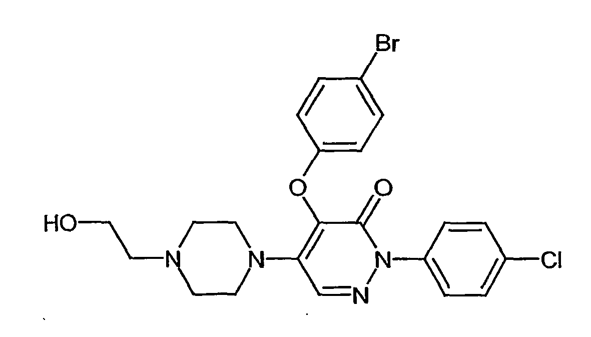

Zu einer Mischung von 4.18 (9.05 mmol) 4-(4-Bromphenoxy)-2-(4-chlorphenyl)-5-(1-piperazinyl)-3(2H)-pyridazinon (Beispiel 57A), 0.15 g Kaliumiodid und 3.75 g (27.2 mmol) Kaliumcarbonat in 25 ml 1,4-Dioxan werden 1.35 ml (18.11 mmol) 2-Bromethanol getropft. Man rührt über Nacht unter Rückfluß, bevor mit Dichlormethan verdünnt und zweimal mit Wasser gewaschen, getrocknet und eingeengt wird. Man reinigt durch Flash-Chromatographie an Silicagel (Gradient: Dichlormethan/Methanol von 99:1 bis 97:3).
Ausbeute: 3.5 g (76.4 % d.Th.)
MS (Methode A): m/z = 505/507 (M+H)⁺
HPLC: Rₜ = 6.87 min.

Gemäß allgemeinem Verfahren **8** werden als Zwischenverbindungen erhalten:

### Beispiel 25A

### 2-(4-Chlorphenyl)-5-(1H-imidazol-1-yl)-4-[4-(4,4,5,5-tetramethyl-1,3,2-dioxaborolan-2-yl)phenoxy]-3(2H)-pyridazinon

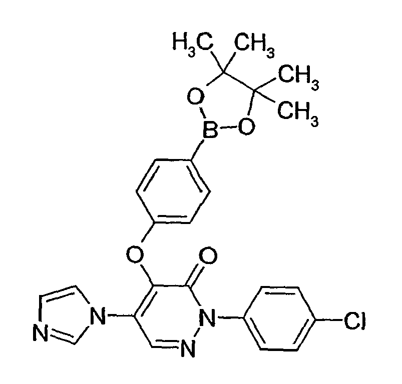

MS (Methode C): m/z = 490 (M⁺)
HPLC: Rₜ = 6.31 min.

### Beispiel 26A

### 2-(4-Chlorphenyl)-5-[4-(2-hydroxyethyl)-1-piperazinyl]-4-[4-(4,4,5,5-tetramethyl-1,3,2-dioxaborolan-2-yl)phenoxy]-3 (2H)-pyridazinon

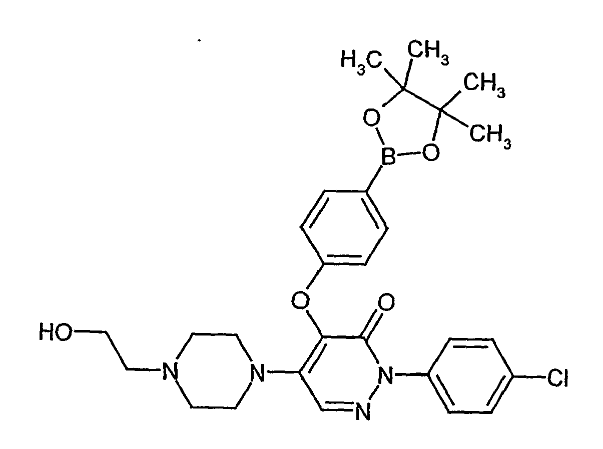

MS (Methode B): m/z = 553 (M+1-1)⁺
HPLC: Rₜ = 7.42 min.

Nach dem allgemeinen Verfahren **15a** wurden die in Tabelle 5 aufgeführten Zwischenverbindungen Beispiel 27A - Beispiel 37A hergestellt:

| **Beispiel-Nr.** | | **Physikalische Daten** | **Edukt** |
|---|---|---|---|
| 27A | 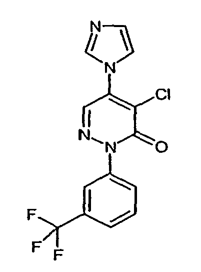 | Schmp.: 101°C | 4,5-Dichlor-2-(3-trifluormethylphenyl)-(3H)-pyridazinon |
| 28A | 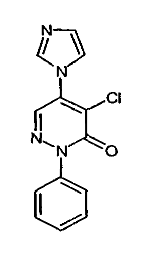 | Schmp.:162°C | 4,5-Dichlor-2-phenyl-(3H)-pyridazinon |
| 29A | 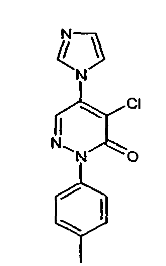 | Schmp.:157°C | 4,5-Dichlor-2-(4-methylphenyl)-(3H)-pyridazinon |
| 30A | 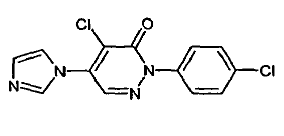 | Schmp.:175°C | 4,5-Dichlor-2-(4-chlorphenyl)-(3H)-chlorphenyl)-(3H)-pyridazinon |
| 31A | 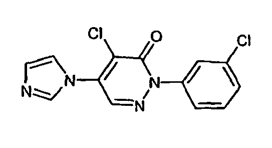 | Schmp.: 176°C | 4,5-Dichlor-2-(3-chlorphenyl)-(3H)-pyridazinon |
| 32A | 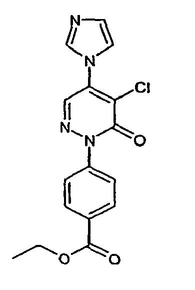 | Schmp.: 149°C | 4,5-Dichlor-2-(4-ethoxycarbonylphenyl)-(3H)-pyridazinon |
| 33A | 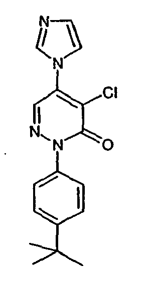 | Schmp.:170-3°C | 2-[4-(tert-Butyl)phenyl]-4,5-dichlor-3(2H)-pyridazinon |
| 34A | 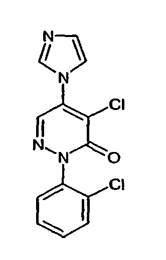 | Schmp.:139-41°C | 4,5-Dichlor-2-(2-chlorphenyl)-(3H)-pyridazinon |
| 35A | 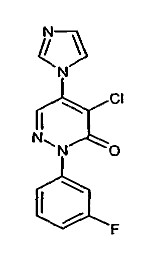 | Schmp.: 122°C | 4,5-Dichlor-2-(3-fluorphenyl) (3H)-pyridazinon |
| 36A | 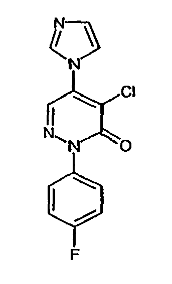 | Schmp.: 181 °C | 4,5-Dichlor-2-(4-fluorphenyl) (3H)-pyridazinon |
| 37A | 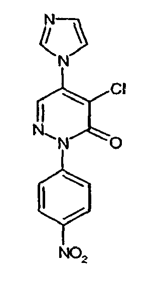 | | 4,5-Dichlor-2-(4-nitrophenyl) (3H)-pyridazinon |

Gemäß allgemeinem Verfahren **6a** wurden die in Tabelle 6 aufgeführten Zwischenverbindungen Beispiel 38A - Beispiel 63A erhalten:

| **Beispiel-Nr**. | **Struktur** | **Physikalische Daten (Angaben in °C bezielien sich auf den Schmelzpunkt)** | **Allgemeines Verfahren** | **Ausgangsmaterialien** |
|---|---|---|---|---|
| 38A | 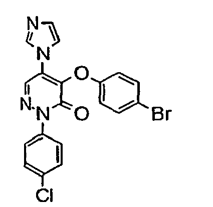 | 182°C | 16a | Beispiel-Nr.30A; 4-Bromphenol |
| 39A | 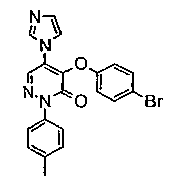 | MS (A): 423 (M+H) (100) | 16a | Beispiel-Nr. 29A; 4-Bromphenol |
| 40A | 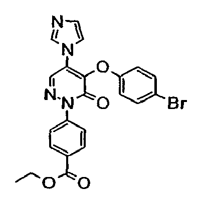 | 155-7°C | 16a | Beispiel-Nr.32A; 4-Bromphenol |
| 41A | 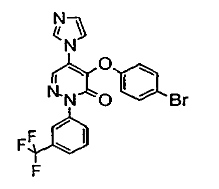 | 168-70°C | 16a | Beispiel-Nr.27A; 4-Bromphenol |
| 42A | 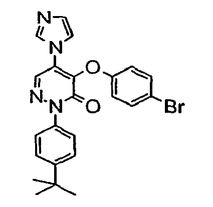 | 225°C | 16a | Beispiel-Nr.33A; 4-Bromphenol |
| 43A | 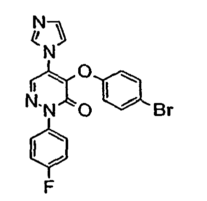 | 185-190°C | 16a | Beispiel-Nr.36A; 4-Bromphenol |
| 44A | 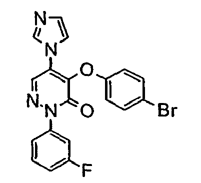 | 180-185°C | 16a | Beispiel-Nr.35A; 4-Bromphenol |
| 45A | 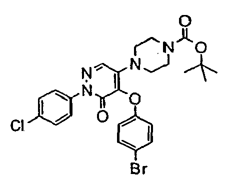 | MS (A): 561/563 (M+H) (100); HPLC: 10.64 (94) | 6-a | Beispiel-Nr.23A; 4-Bromphenol |
| 46A | 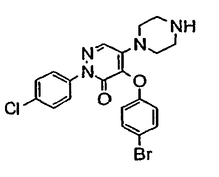 | MS (B): 462/463 (M+H) (100); HPLC: 6.72 (98) | 7 | Beispiel-Nr.45A |
| 47A | 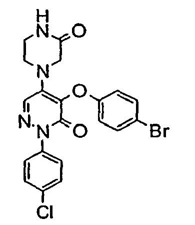 | MS (B): 475/477 (M+H) (100); HPLC: 7.98 (100) | 6-a | Beispiel Nr. 7A; 4-Bromphenol |
| 48A | 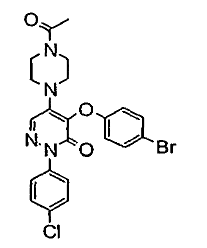 | MS (B): 503/505 (M+H); HPLC: 8.48 (91) | 6-b | Beispiel-Nr. 3A; 4-Bromphenol |
| 49A | 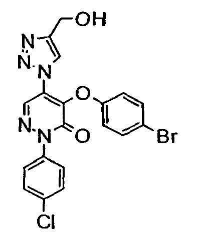 | MS (B): 474/476 (M+H) (100); HPLC: 8.78 (100) | 14 | Beispiel-Nr. 15A; Propargylalkohol |
| 50A | 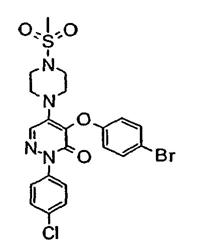 | MS (B): 539/541 (M+H) (100); HPLC: 9.16 (98) | 6-b | Beispiel-Nr. 6A; 4-Bromphenol |
| 51A | 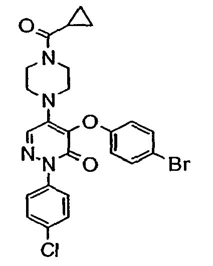 | MS (D): 529/531 (M+H) (100); HPLC: 9.12 (93) | 6-b | Beispiel-Nr. 5A; 4-Bromphenol |
| 52A | 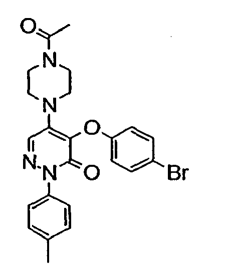 | MS (A): 483/485 (M+H) (100); HPLC: 8.16 (94) | 6-b | Beispiel-Nr.4A; 4-Bromphenol |
| 53A | 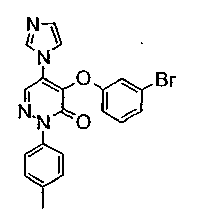 | MS (A): 423 (M+H) (100) | 16a | Beispiel-Nr. 29A; 3-Bromphenol |
| 54A | 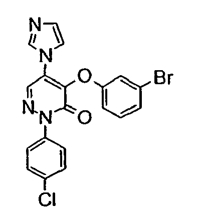 | 83-86°C | 16a | Beispiel-Nr.30A; 3-Bromphenol |
| 55A | 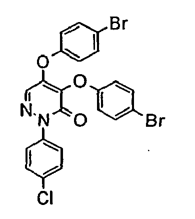 | MS (A): 566 (M+NH₄) (100); HPLC: 8.98 (100) | 12 | 2-(4-Chlorphenyl)-4,5-dichlor-pyridazinon; 4-Bromphenol |
| 56A | 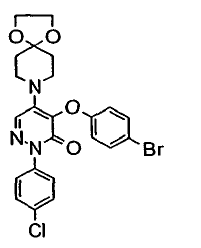 | MS (D): 382 (M+H) (87); HPLC: 9.16 (92) | 16a | Beispiel-Nr. 22A; 4-Bromphenol |
| 57A | 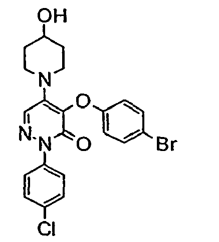 | MS (A): 478 (M+H) (100); HPLC: 8.65 (93) | 1; 16a | 2-(4-Chlorphenyl)-4,5-dichlor-pyridazinon; 4-Hydroxypiperidin; 4-Bromphenol |
| 58A | 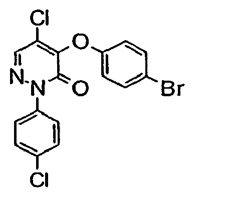 | MS (A): 428 (M+NH₄) (100); HPLC: 10.48 (99) | 12 | 2-(4-Chlorphenyl)-4,5-dichlor-pyridazinon; 4-Bromphenol |
| 59A | 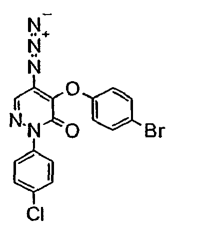 | MS (A): 435 (M+NH₄) (100); HPLC: 10.25 (95) | 13 | Beispiel-Nr.10A |
| 60A | 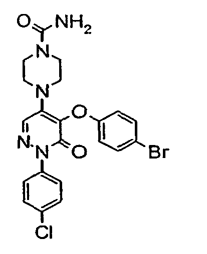 | MS (C): 506 (M+H) (100); HPLC: 7.93 (92) | 5 | Beispiel-Nr. 46A |
| 61A | 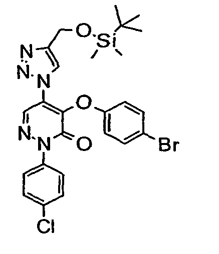 | MS (C): 590 (M+H) (100); HPLC: 12.25 (95) | 14 | Beispiel-Nr. 59A; 1-(tert.-Butyl-dimethylsilyloxy)-prop-2-in |
| 62A | 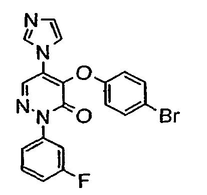 | roh weiter umgesetzt | 16a | Beispiel-Nr. 35A; 4-Bromphenol |
| 63A | 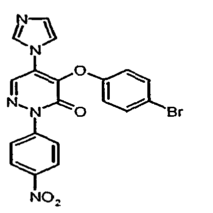 | roh weiter umgesetzt | 16a | Beispiel-Nr. 37A; 4-Bromphenol |

### Ausführunssbeispiele:

### Beispiel 1.

### N-(4'-{[2-(4-Chlorphenyl)-5-(1H-imidazol-1-yl)-3-oxo-2,3-dihydro-4-pyridazinyl]-oxy}-1,1'-biphenyl-2-yl)-N-(methylsulfonyl)methmsulfonamid

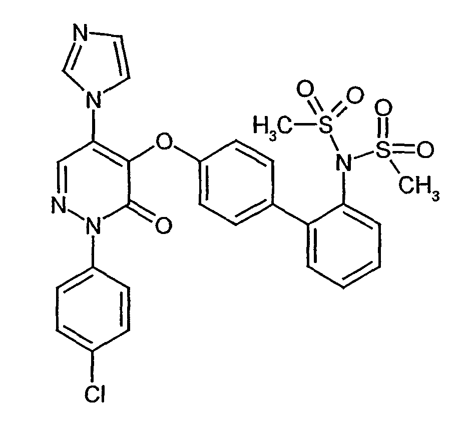

Zu einer Mischung von 16.5 mg (0.036 mmol) 4-[(2'-Amino-1,1'-biphenyl-4-yl)oxy]-2-(4-chlorphenyl)-5-(1H-imidazol-1-yl)-3(2H)-pyridazinon (Beispiel 156), 0.02 ml Triethylamin und einer katalytischen Menge 4-DMAP in 0.2 ml absolutem Dichlormethan werden bei Raumtemperatur 0.06 ml (0.072 mmol) Methansulfonsäurechlorid getropft. Nach 30 min wird die Reaktion durch Zusatz von wenig Wasser abgebrochen und die Reaktionsmischung direkt an Silicagel säulenfiltriert (Laufmittel: Gradient Dichlormethan / Methanol von 99:1 bis 98:2).
Ausbeute: 15.3 mg (69.1 % d.Th.)
MS (ESI): m/z = 612 (M+H)⁺
HPLC: Rₜ = 8.17 min.

### Beispiel 2

### tert.-Butyl 4-[5-({4'-[(2,2-dimethylpropanoyl)oxy]-1,1'-biphenyl-4-yl}oxy)-1-(4-methylphenyl)-6-oxo-1,6-dihydro-4-pyridazinyl]-1-piperazincarboxylat

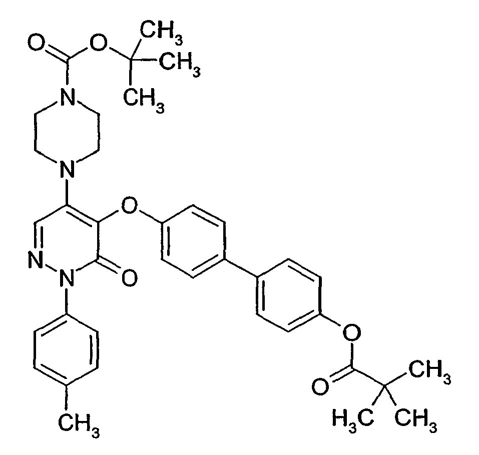

Zu einer Suspension aus 728 mg (1.18 mmol) tert.-Butyl 4-[5-[(4'-hydroxy-1,1'-biphenyl-4-yl)oxy]-1-(4-methylphenyl)-6-oxo-1,6-dihydro-4-pyridazinyl]-1-piperazin-carboxylat (Beispiel 46) und einer katalytischen Menge 4-DMAP in einer Mischung aus Dichlormethan (4.0 ml) und Pyridin (0.4 ml) werden bei Raumtemperatur 0.66 ml Pivaloylchlorid getropft. Die heterogene Reaktionsmischung wird über Nacht bei ca. 40°C gerührt. Die leicht trübe Suspension wird mit Dichlormethan verdünnt und mit 0.2 N Salzsäurelösung und gesättigter Natriumchlorid-Lösung gewaschen, über Magnesiumsulfat getrocknet und konzentriert. Das Rohprodukt wird durch Chromatographie an Silicagel gereinigt.
Ausbeute: 568 mg (75.3 % d.Th.)
MS (ESI): m/z = 639 (M+H)⁺
HPLC: Rₜ = 10.21 min.

### Beispiel 3

### 4'-{[2-(4-Methylphenyl)-3-oxo-5-(1-piperazinyl)-2,3-dihydro-4-pyridazinyl]oxy}-1,1'-biphenyl-4-yl-pivalat-Trifluoressigsäuresalz

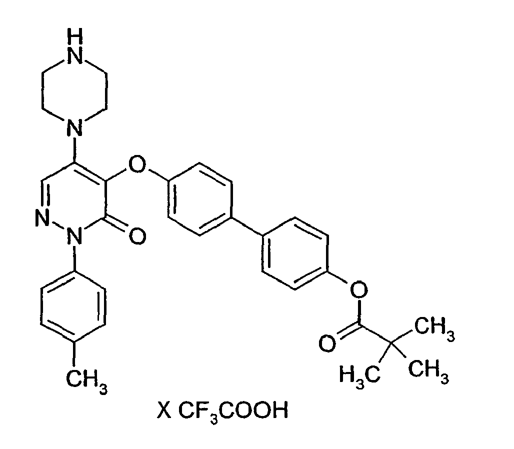

Zu einer Lösung von 568 mg (0.889 mmol) der Verbindung aus Beispiel 2 in 4.5 ml Dichlormethan werden bei Raumtemperatur 0.2 ml Wasser und 2.2 ml Trifluoressigsäure getropft. Nach 45 min. wird die Reaktionsmischung eingeengt und gründlich am Hochvakuum getrocknet.
Ausbeute: 702 mg (96.5 % d.Th.)
MS (ESI): m/z = 539 (M+H)⁺ (freie Base).

### Beispiel 4

### 4'-{[5-(4-Ethyl-1-piperazinyl)-2-(4-methylphenyl)-3-oxo-2,3-dihydro-4-pyridazinyl]-oxy}-1,1'-biphenyl-4-yl-pivalat

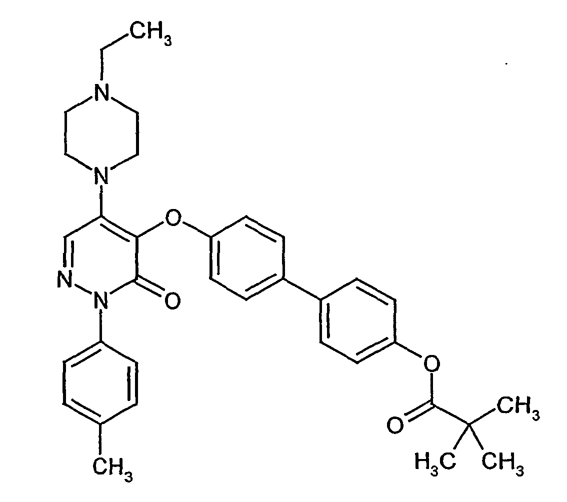

360 mg (0.44 mmol) der Verbindung aus Beispiel 3 werden in 2.0 ml Aceton vorgelegt und nacheinander werden 0.046 ml (0.57 mmol) Ethyliodid und 0.18 ml (1.32 mmol) Triethylamin zugegeben. Die Mischung wird über Nacht bei Raumtemperatur gerührt, bevor mit Dichlormethan verdünnt, mit gesättigter Natriumhydrogencarbonat-Lösung gewaschen, über Magnesiumsulfat getrocknet und eingeengt wird. Das Rohprodukt wird durch Säulenfiltration an Silicagel (Laufmittel: Dichlormethan/Methanol 95:5) gereinigt.
Ausbeute: 211 mg (84.4 % d.Th.)
MS (ESI): m/z = 567 (M+H)⁺
HPLC: Rₜ = 7.00 min.

### Beispiel 5

### 5-(4-Ethyl-1 -piperazmyl)-4-[(4'-hydroxy-1, 1'-biphenyl-4-yl)oxy]-2-(4-methylphenyl)-3 (2H)-pyridazinon

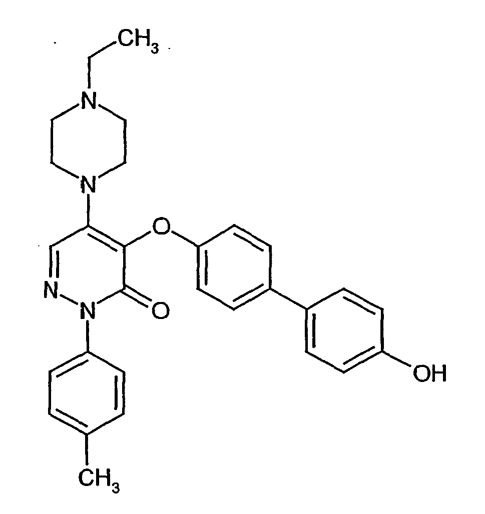

210 mg (0.37 mmol) der Verbindung aus Beispiel 4 werden in 4.5 ml trockenem Methanol vorgelegt. Nach Zugabe von 0.9 ml Triethylamin wird 24 h auf Rückfluß erhitzt. Die Suspension wird konzentriert und der erhaltene, farblose Feststoff im Hochvakuum getrocknet.
Ausbeute: 134 mg (74.9% d.Th.)
MS (DCI/NH₃): m/z = 483 (M+H)⁺
HPLC: Rₜ = 6.59 min.

### Beispiel 6

### 4-[(4'-Hydroxy-1,1'-biphenyl-4-yl)oxy]-2-(4-methylphenyl)-5-(4-methyl-1-piperazinyl)-3 (2H)-pyridazinon

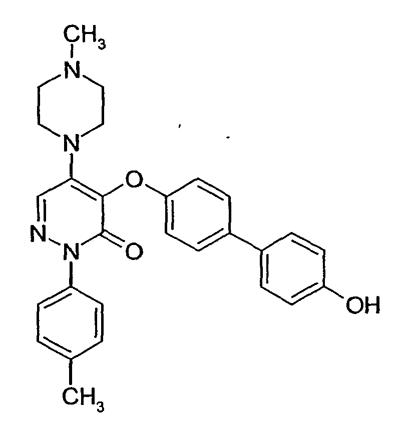

360 mg (0.44 mmol) der Verbindung aus Beispiel 3 werden in 2.0 ml Aceton vorgelegt und nacheinander werden ein Überschuß Methyliodid und 0.18 ml Triethylamin zugegeben. Nach Abklingen der Reaktion wird 1 h bei Raumtemperatur gerührt, bevor mit Dichlormethan verdünnt wird. Der ausgefallene Feststoff wird abfiltriert und das Filtrat mit Wasser gewaschen, über Magnesiumsulfat getrocknet und eingeengt. Das Rohprodukt wird durch Chromatographie an Silicagel (Laufmittel: Dichlormethan/Methanol 95:5) gereinigt. Man löst das Produkt (23 mg) in absolutem Methanol, gibt 0.3 ml Triethylamin hinzu und rührt 36 h unter Rückfluß. Nach Abkühlen wird eingeengt und das erhaltene Produkt gründlich im Hochvakuum getrocknet.
Ausbeute: 19 mg (9.1 % d.Th.)
MS (DGI/NH₃): m/z = 486 (M+NH₄)⁺, 469 (M+H)⁺
HPLC: Rₜ = 6.51 min.

### Beispiel 7

### 5-(4-Ethyl-1-piperazinyl)-4-[(4'-hydroxy-1,1'-biphenyl-4-yl)oxy]-2-(4-methylphenyl)-3(2H)-pyridazinon-Hydrochlorid

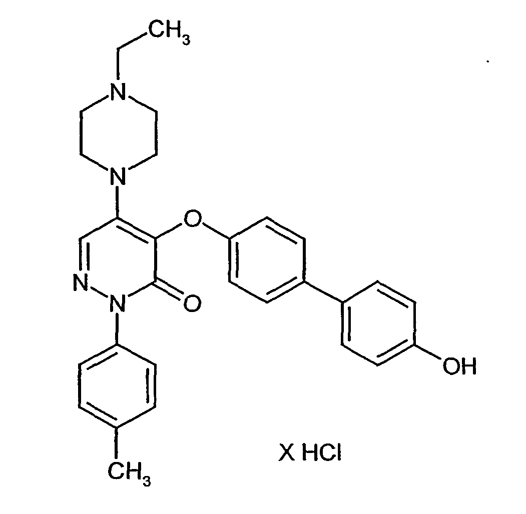

Zu einer Mischung von 48.3 mg (0.1 mmol) der Verbindung aus Beispiel 5 in 4.0 ml 1,4-Dioxan wird Salzsäure (0.1 ml einer 1 molaren Lösung in 1,4-Dioxan) gegeben. Nach 30 min. wird die weiße Suspension konzentriert und im Hochvakuum gründlich getrocknet.
Ausbeute: 51 mg (98.3 % d.Th.)
MS (DCI/NH₃): m/z = 500 (M+NH₄)⁺, 483 (M+H)⁺
HPLC: Rₜ = 6.44 min.

### Beispiel 8

### 4-{[3'-(Aminomethyl)-4'-fluor-1,1'-biphenyl-4-yl]oxy}-2-(4-chlorphenyl)-5-[4-(methylsulfonyl)-1-piperazinyl]-3 (2H)-pyridazinon

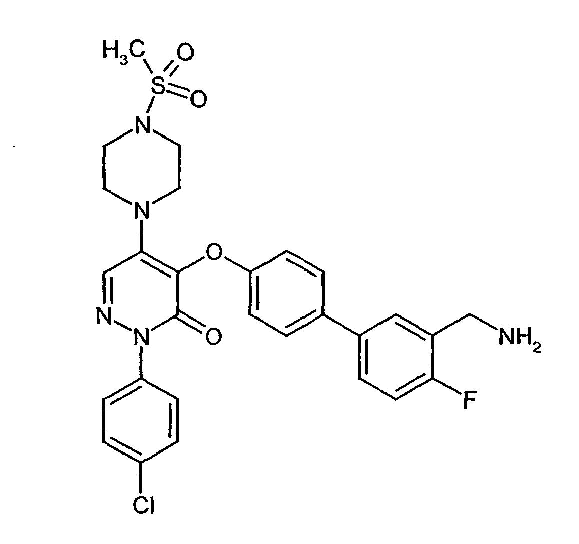

Gemäß allgemeiner Vorschrift 9-c wird die Verbindung aus Beispiel 50A mit 2-Fluor-S-brom-(N tent-butylo:cycarbonyl)-benzylamin umgesetzt. Das Kupplungsprodukt wird gemäß allgemeiner Vorschrift 4 zum entsprechenden Sulfonamid derivatisiert. Nach Abspalten der Boc-Schutzgruppe (allgemeine Vorschrift 7) wird das Zielprodukt erhalten.
MS (ESIpos): m/z = 584 (M+H)⁺
HPLC: Rₜ = 6.88 min.

### Beispiel 9

### 2-(4-Chlorphenyl)-4-{ [4'-fluor-3'-(hydroxymethyl)-1,1'-biphenyl-4-yl]oxy}-5-[4-(methylsulfonyl)-1-piperazinyl]-3 (2H)-pyridazinon

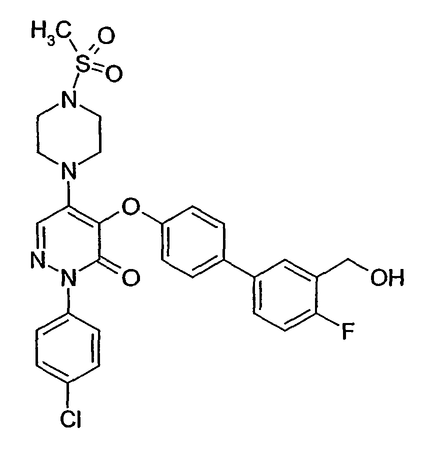

Gemäß allgemeiner Vorschrift **9-c** wird die Verbindung aus Beispiel 50A mit 2-Fluor-5-brom-(*O*-*tert*-butyldimethylsilyl)-benzylalkohol umgesetzt. Das Kupplungsprodukt wird gemäß allgemeiner Vorschrift **4** zum entsprechenden Sulfonamid derivatisiert. Das erhaltene Zwischenprodukt wird in Acetonitril bei 0°C mit HF-Lösung (5 % einer 48 %-igen Lösung) behandelt und 2 h bei Raumtemperatur kräftig gerührt. Die Reaktionsmischung wird mit gesättigter Natriumhydrogencarbonat-Lösung neutralisiert und mit Dichlormethan extrahiert. Die organische Phase wird über Magnesiumsulfat getrocknet und eingeengt. Das Produkt wird im Hochvakuum gründlich getrocknet.
LC-MS (ESIpos): m/z = 585/587 (M+H)⁺
HPLC: Rₜ = 8.63 min.

### Beispiel 10

### 2-(4-Chlorphenyl)-5-(1H-imidazol-1-yl)-4-[4-(4-pyridmyl)phenoxy]-3(2H)-pyridazinon

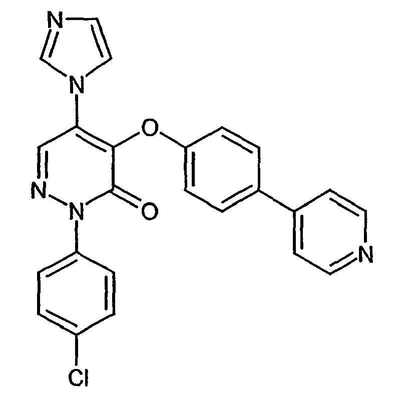

1.5 mmol 4-(4-Bromphenyl)oxy-2-(4-chlorphenyl)-5-(1H-imidazol-1-yl)-3(2H)-pyridazinon, 2.1 mmol 4-Trimethylstannylpyridin, 0.1 mmol Bis(triphenylphosphin)-palladiumdichlorid und 1.3 mmol Ethyldiisopropylamin in 5 ml Dimethylformamid werden 5 Stunden auf 100°C erhitzt. Das Lösemittel wird am Rotationsverdampfer entfernt, der Rückstand durch Chromatographie an Kieselgel (Elution mit Essigsäureethylester/Methanol-Gemischen) gereinigt. Man erhält 0.3 g (45 % d.Th.) vom Schmp. 188°C.

### Beispiel 11

### tert-Butyl 4-[5-(1,1'-biphenyl-4-yloxy)-1-(4-chlorphenyl)-6-oxo-1,6-dihydro-4-pyridazinyl]-1-piperazincarboxylat

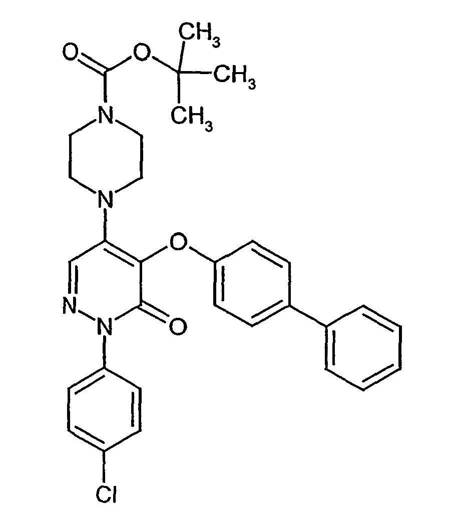

Gemäß allgemeiner Vorschrift 6-d aus 9.60 g (22.6 mmol) der Verbindung aus Beispiel 23A und 14.1 g (67.65 mmol) 4-Phenylphenolat-Kalium erhältlich.
Ausbeute: 7.37 g (37.6 % d.Th.)
MS (DCI/NH₃): m/z = 559 (M+H)⁺
HPLC: Rₜ = 11.11 min.

### Beispiel 12

### 4-(1,1'-Biphenyl-4-ylo_{Y}y)-2-(4-chlorphenyl)-5-( 1-piperazinyl)-3 (2H)-pyridazinon

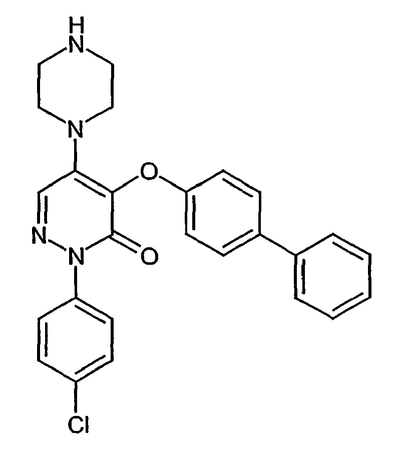

Gemäß allgemeiner Vorschrift 7 aus 4.23 g (7.56 mmol) der Verbindung aus Beispiel 11 erhältlich.
Ausbeute: 3.44 g (99.2 % d.Th.)
MS (DCI/NH₃): m/z = 459 (M+H)⁺
HPLC: Rₜ = 7.19 min.

### Beispiel 13

### 4-(Biphenyl-4-yloxy)-2-(4-chlor-phenyl)-5-(1,4-dioxa-8-aza-spiro[4.5]dec-8-yl)-2H-pyridazin-3-on

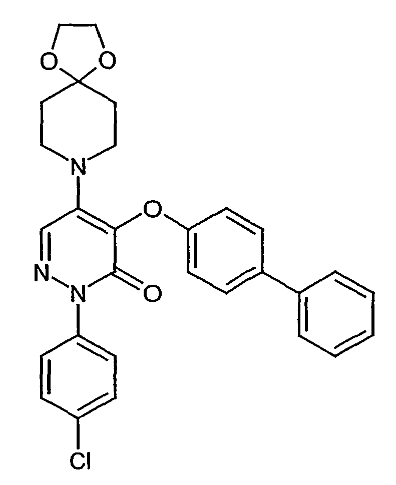

Erhältlich gemäß Methode **6-d** aus 6.0 g (15.7 mmol) der Verbindung aus Beispiel 22A.
Ausbeute: 1.42 g (17.5 % d.Th.)
MS (ESI): m/z = 516 (M+H)⁺
HPLC: Rₜ = 10.45 min.

### Beispiel 14

### 4-(Biphenyl-4-yloxy)-2-(4-chlor-phenyl)-5-(4-oxo-piperidin-1-yl)-2H-pyridazin-3-on

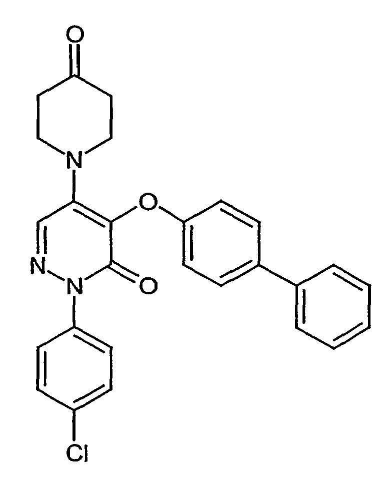

1.4 g (2.71 mmol) der Verbindung aus Beispiel 13 werden 2 h in 170 ml Aceton und 85 ml 6 N Salzsäure unter Rückfluß gerührt. Nach Abkühlen destilliert man das Lösemittel ab, versetzt mit Essigsäureethylester und wäscht mit gesättigter Natriumcarbonat-Lösung und Wasser. Die organische Phase wird getrocknet und eingeengt. Der Rückstand wird mit Ether verrührt und abgesaugt.
Ausbeute: 705 mg (55.1 % d.Th.)
MS (EI): m/z = 472 (M+H)⁺
HPLC: Rₜ = 9.69 min.

### Beispiel 15

### 4-(Biphenyl-4-yloxy)-2-(4-chlor-phenyl)-5-(4-hydroxy-piperidin-1-yl)-2H-pyridazin-3-on

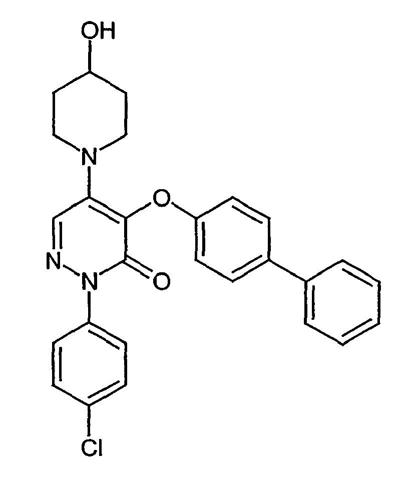

0.2 g (0.424 mmol) der Verbindung aus Beispiel 14 werden in 7 ml Methanol gelöst und portionsweise mit 50 mg (1.3 mmol) Natriumborhydrid bei Raumtemperatur versetzt. Nach 2 h Rühren wird mit Dichlormethan verdünnt, mit gesätttigter Ammoniumchlorid-Lösung gewaschen, über Natriumsulfat getrocknet und eingeengt. Das Rohprodukt wird mit 25 ml Methanol in der Siedehitze verrührt, danach abgekühlt und abgesaugt.
Ausbeute: 78 mg (39 % d.Th.)
MS (ESI): m/z = 474 (M+H)⁺
HPLC:Rₜ = 9.27 min.
Durch Einengen der Mutterlauge und Verrühren mit Dichlormethan erhält man eine 2. Fraktion:
Ausbeute: 56 mg (27.9 % d.Th.)

### Beispiel 16

### 4-(Biphenyl-4-yloxy)-2-(4-chlor-phenyl)-5-(4-fluor-piperidin-1-yl)-2H-pyridazin-3-on

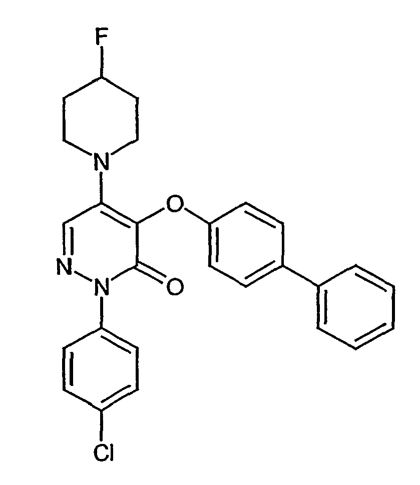

Zu einer Lösung von 15 mg (0.03 mmol) der Verbindung aus Beispiel 15 in 3 ml Dichlormethan werden 0.122 g (0.757 mmol) Diethylamino-schwefeltrifluorid bei Eiskühlung und unter Argon zugegeben. Nach 4 h wird die Reaktionslösung auf Ammoniumchlorid-Lösung gegossen und dreimal mit Dichlormethan extrahiert. Die organische Phase wird getrocknet, eingeengt und durch Chromatographie an Silicagel (Laufmittel-Gradient von Toluol bis Toluol/Acetonitril 18:1) gereinigt.
Ausbeute: 5.6 mg (37.2 % d.Th.)
MS (ESI): m/z = 476 (M+H)⁺
DC: R_{f} = 0.37 (Toluol / Acetonitril 9:1)

### Beispiel 17

### 4-(Biphenyl-4-yloxy)-2-(4-chlor-phenyl)-5-(4,4-difluor-piperidin-1-yl)-2H-pyridazin-3-on

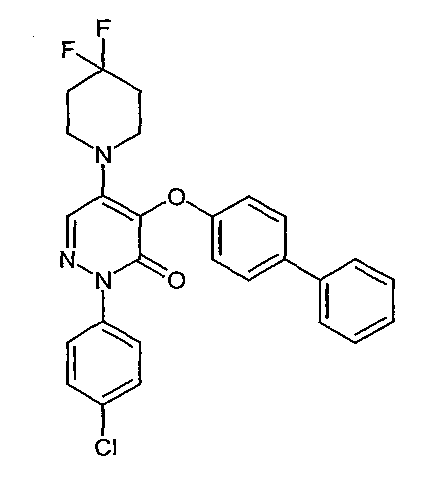

Zu einer Lösung von 60 mg (0.127 mmol) der Verbindung aus Beispiel 14 in 5 ml Dichlormethan werden bei 0°C und unter Argon 170 mg (1.06 mmol) Diethylaminschwefeltrifluorid zugetropft und 30 min bei 0°C nachgerührt. Anschließend läßt man ohne Kältebad 1.5 h weiterrühren, verdünnt mit Dichlormethan und fügt Ammoniumchlorid-Lösung hinzu. Die organische Phase wird abgetrennt, über Natriumsulfat getrocknet und eingeengt. Das Produkt wird nach Chromatographie an Silicagel (Laufmittel: 180 ml Toluol / 5 ml Acetonitril bis 180 ml Toluol / 15 ml Acetonitril) erhalten.
Ausbeute: 31 mg (49.4 % d.Th.)
MS (DCI/NH₃): m/z = 494 (M+H)⁺
DC: R_{f} = 0.49 (Toluol / Acetonitril 8:2)

### Beispiel 18

### 2-(4-Chlor-phenyl)-4-(4'-fluor-biphenyl-4-yloxy)-5-(4-chlor-piperidin-1-yl)-2H-pyridazin-3-on

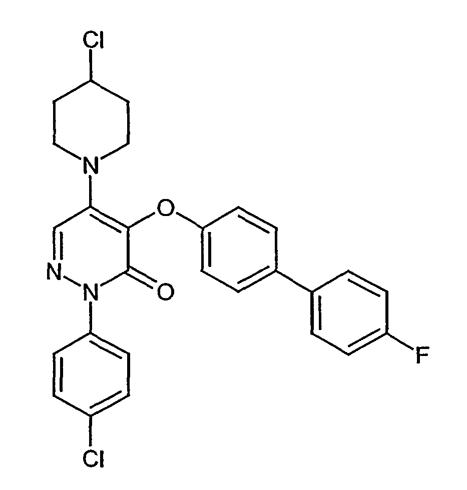

Eine Suspension von 150 mg (0.305 mmol) 2-(4-Chlorphenyl)-4-(4'-fluor-biphenyl-4-yloxy)-5-(4-hydroxy-piperidin-1-yl)-2H-pyridazin-3-on (erhältlich analog zu Beispiel 15) wird 60 min. mit 4 ml Thionylchlorid unter Rückfluß erhitzt. Die Reaktionslösung wird nach Abkühlen im Hochvakuum eingeengt, der Rückstand in 50 ml Essigsäureethylester und 20 ml gesättigter Natriumhydrogencarbonat-Lösung verteilt, die organische Schicht abgetrennt und die wäßrige Phase dreimal mit Essigsäureethylester extrahiert. Die vereinigten organischen Phasen werden über Natriumsulfat getrocknet und das Lösemittel im Vakuum entfernt. Die Reinigung des Produktes erfolgt durch Chromatographie über Silicagel mit Toluol / Essigsäureethylester von 9:1 bis 8:2 als Laufmittel.
Ausbeute: 30.9 mg (18.2 % d.Th.)
MS (ESI): m/z = 512 (M+H)⁺
HPLC: Rₜ = 11.02 min.

### Beispiel 19

### (2-{4-[5-(Biphenyl-4-yloxy)-1-(4-cMor-phenyl)-6-oxo-1,6-dihydro-pyridazin-4-yl]-piperazin-1-yl}-1-methyl-2-oxo-ethyl)-carbonsäure-tert.butylester

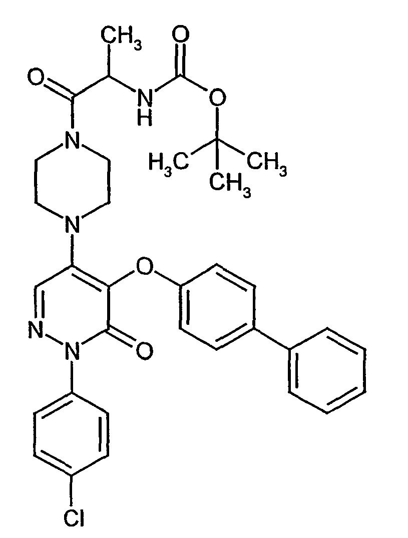

Zu einer Lösung von 92 mg (0.487 mmol) Boc-L-Alanin, 93.4 mg (0.487 mmol) EDC, 65.8 mg (0.487 mmol) HOBt und 0.1 ml (0.717 mmol) Triethylamin in 2 ml Tetrahydrofuran und 10 ml Dichlormethan gibt man unter Rühren und Argon bei 0°C 160 mg (0.348 mmol) der Verbindung aus Beispiel 12, gelöst in 1 ml Dichlormethan, tropfenweise hinzu. Die Mischung wird über Nacht bei Raumtemperatur gerührt. Die Reaktionslösung wird mit 100 ml Dichlormethan verdünnt, mit gesättigter Natriumhydrogencarbonat- und Kochsalz-Lösung gewaschen, über Natriumsulfat getrocknet und eingeengt. Der Rückstand wird mit Ether verrührt und abgesaugt.
Ausbeute: 195.5 mg (89.0 % d.Th.)
MS (ESI): m/z = 630 (M+H)⁺
HPLC: Rₜ = 10. 15 min.

### Beispiel 20

### 4-(Biphenyl-4-yloxy)-2-(chlorphenyl)-5-(4-L-alanyl-piperazin-1-yl)-2H-pyridazin-3-on

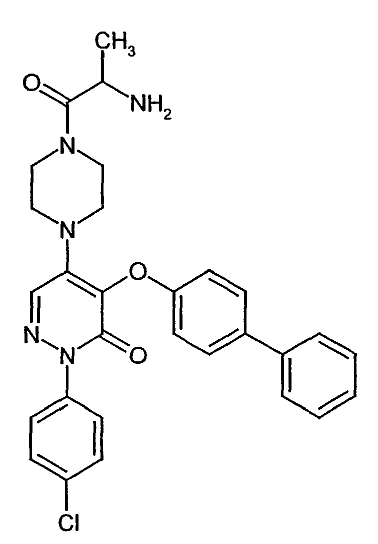

Erhältlich aus 180 mg (0.286 mmol) der Verbindung aus Beispiel 19 gemäß allgemeiner Methode 7.
Ausbeute: 80 mg (52.9 % d.Th.)
MS (ESI): m/z = 530 (1M+H)⁺
HPLC:Rₜ = 7.33 min.

### Beispiel 21

### 4-[5-(4'-Fluor-biphenyl-4-yloxy)-6-oxo-1-(4-trifluormethyl-phenyl)-1,6-dihydropyridazin-4-yl]-piperazin-1-carbaldehyd

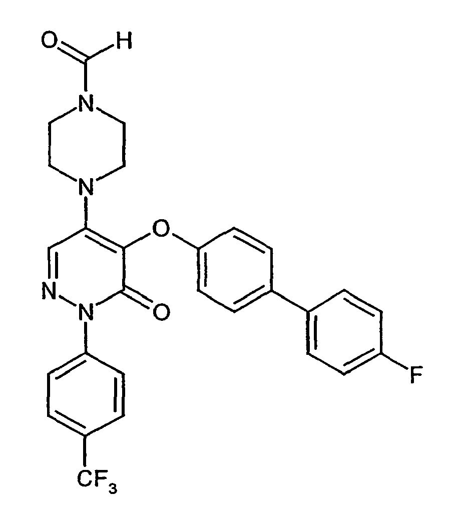

Zu einer Lösung von 288.5 mg (6.27 mmol) Ameisensäure in 4 ml Dichlormethan gibt man zunächst 613.1 mg (3.13 mmol) EDC hinzu und rührt 20 min bei 0°C nach. Diese Reaktionslösung wird anschließend mit 80 mg (0.157 mmol) 4-(4'-Fluorbiphenyl-4-oxy)-2-(4-trifluormethylphenyl)-5-(piperazin-1-y1)-3(2H)-pyridazinon (erhältlich in Analogie zu Beispiel 12) und 0.1 ml (0.717 mmol) Triethylamin bei 0°C versetzt und bei 0°C über Nacht nachgerührt. Durch wässrige Aufarbeitung mit verdünnter Salzsäure und anschließend gesättigter Natriumhydrogencarbonat-Lösung erhält man ein Rohprodukt, das an Silicagel mit Dichlormethan / Methanol 10:1 als Laufmittel chromatographiert wird.
Ausbeute: 24.6 mg (7.3 % d.Th.)
MS (ESI): m/z = 539 (M+H)⁺
HPLC: Rₜ = 9.40 min.

### Beispiel 22

### 4-(Biphenyl-4-yloxy)-2-(4-chlor-phenyl)-5(4-cyclopropyl-piperazin-1-yl)-2H-pyridazin-3-on

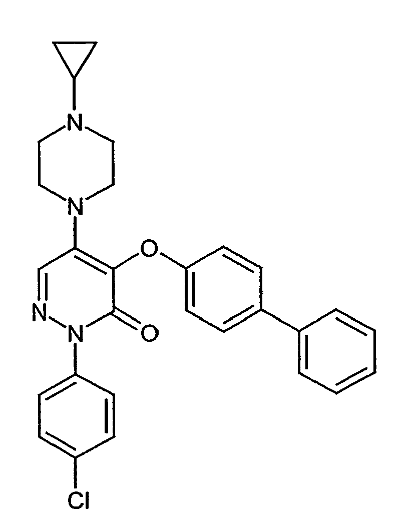

Zu einer Lösung von 150 mg (0.327 mmol) der Verbindung aus Beispiel 40 in 5 ml DMF und 0.34 ml (2.45 mmol) Triethylamin tropft man 1.77 ml (22.1 mmol) Cyclopropylbromid zu und rührt anschließend bei 120°C über Nacht. Die Reaktionsmischung wird auf 150 ml Wasser gegossen und zweimal mit Essigsäureethylester extrahiert. Die organische Phase wird über Natriumsulfat getrocknet und eingeengt. Das Produkt wird durch Chromatographie an Silicagel (Laufmittel: Gradient Toluol / Acetonitril von 40:1 bis 4:1) gereinigt.
Ausbeute: 41 mg (21.1 % d.Th.)
MS (DCI/NH₃): m/z = 499 (M+H)⁺
HPLC: Rₜ = 7.71 min.

### Beispiel 23

### 2-(4-Chlor-phenyl)-5-(3,5-dihydro-2H-pyridin-1-yl)-4-(4'-fluor-biphenyl-4-yloxy)-2H-pyridazin-3-on

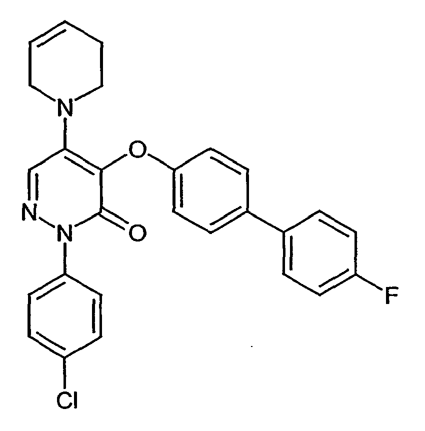

Gemäß allgemeiner Vorschrift 6-c aus 90 mg (1.1 mmol) 1,2,5,6-Tetrahydropyridin und 300 mg (0.702 mmol) 5-Chlor-4-(4-fluorphenylphenyloxy)-2-(4-chlorphenyl)-2(3H)-pyridazinon erhältlich.
Ausbeute: 196 mg (58.8 % d.Th.)
MS (DCI/NH₃): m/z = 474 (M+H)⁺
HPLC: Rₜ = 10.87 min.

### Beispiel 24

### 2-(4-Chlorphenyl)-5-(3,4-dihydroxy-1-piperidinyl)-4-[(4'-fluor-1,1'-biphenyl-4-yl)oxy]-3 (2H)-pyridazinon

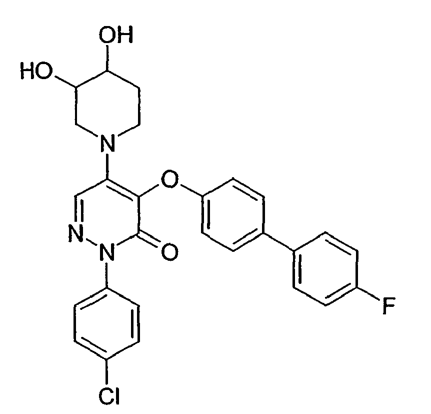

Zu einer Lösung von 100 mg (0.211 mmol) der Verbindung aus Beispiel 23 in 8 ml THF werden 90 mg (0.753 mmol) N-Methylmorpholin-N-oxid und 0.64 ml (0.949 mmol) Osmiumtetroxid als 2.5 Gew.-%-ige Lösung in tert.-Butanol zugegeben und im Dunkeln bei Raumtemperatur über Nacht gerührt. Danach wird das Reaktionsgemisch mit 80 mg Natriumsulfit in 0.5 ml Wasser versetzt, mit 80 ml Dichlormethan und 20 ml Natriumchlorid-Lösung verdünnt und 1 h gerührt. Die organische Phase wird über Natriumsulfat getrocknet, eingeengt und der Rückstand durch Chromatographie an Silicagel mittels Toluol / Acetonitril 9:1 als Laufmittel gereinigt. Anschließend wird der Rückstand in Ether verrührt, abgesaugt und im Hochvakuum getrocknet.
Ausbeute: 23 mg (21.5 % d.Th.)
MS (ESI): m/z = 508 (M+H)⁺
HPLC: Rₜ = 8.71 min.

### Beispiel 25

### 2-(4-Chlorphenyl)-4- [(4'-fluor-1,1'-biphenyl-4-yl)oxy]-5-[4-(hydroxymethyl)-1 - piperidinyl]- 3 (2H)-pyridazinon

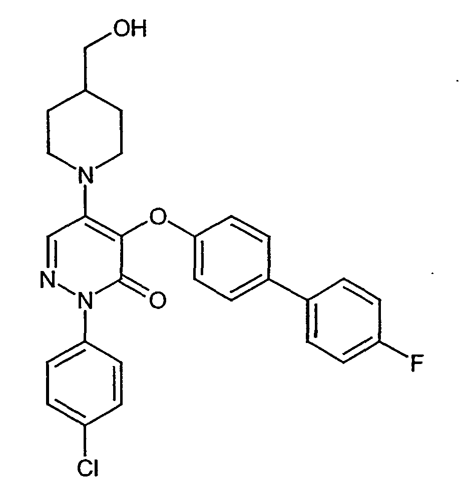

20.0 g (46.8 mmol) 5-Chlor-4-(4-fluorphenylphenyl-oxy)-2-(4-chlorphenyl)-2(3H)-pyridazinon, 6.47 g (56.17 mmol) 4-Hydroxymethylpiperidin, 7.77 g (46.81 mol) Kaliumjodid und 16.31 ml (93.62 mmol) Ethyldiisopropylamin werden in 200 ml DMF unter Argon bei 120°C über Nacht gerührt. Die auf Raumtemperatur abgekühlte Reaktionsmischung wird mit Dichlormethan verdünnt und dreimal mit Wasser gewaschen. Die organische Phase wird getrocknet, eingeengt, der resultierende Rückstand mit Ether verrührt und abgesaugt. Das Rohprodukt wird durch Chromatographie an Silicagel im Gradientenverfahren mit Toluol und Toluol / Acetonitril (von 20:1 über 10:1 bis 5:1) als Laufmittel gereinigt.
Ausbeute: 13.2 g (55.7 % d.Th.)
MS (ESI): m/z = 506 (M+H)⁺
HPLC: Rₜ = 9.50 min.

### Beispiel 26

### 4-(1,1'-Biphenyl-4-yloxy)-2-(4-chlorphenyl)-5-(1-oxido-4-thiomorpholinyl)-3(2H)-pyridazinon

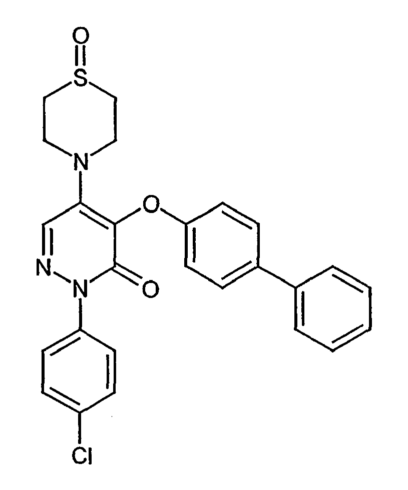

Zu einer Lösung von 150 mg (0.308 mmol) 2-(4-Chlorphenyl)-4-(4-phenylphenyloxy)-5-(thiomorpholinyl-1-)-2(3H)-pyridazinon in 15 ml Dichlormethan werden portionsweise 110 mg (0.308 mmol) 3-Chlorperoxybenzoesäure (57-86%-ig) innerhalb von 5 min. zugegeben und 3 h bei Raumtemperatur nachgerührt. Anschließend wird die Reaktionslösung auf ca. 100 ml trockenes Silicagel aufgetragen und mit Essigsäureethylester und Essigsäureethylester-Aceton-Gemischen im Verhältnis 4:1, 1:1 und 1:4 als Laufmittel chromatographiert.
Ausbeute: 56 mg (36.6 % d.Th.)
MS (DCI/NH₃): m/z = 492 (M+H)⁺
HPLC: Rₜ = 8.68 min.

### Beispiel 27

### 4-(1,1'-Biphenyl-4-yloxy)-2-(4-chlorphenyl)-5-(1,1-dioxido-4-thiomorpholinyl)-3(211)-pyridazinon

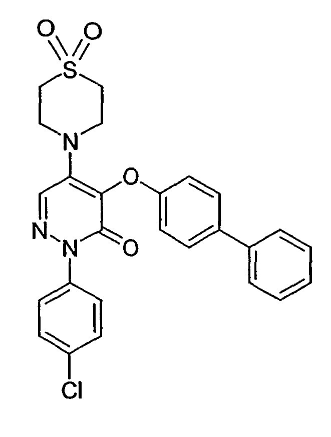

Diese Verbindung wurde als weiteres Produkt bei der Herstellung des Beispiels 26 erhalten und durch Verreiben mit Diisopropylether kristallisiert.
Ausbeute: 95 mg (52.7 % d.Th.)
MS (ESI): m/z = 508 (M+H)⁺
HPLC: Rₜ = 9.38 min.

Nach den allgemeinen Verfahren 1-17 wurden die in Tabelle 7 aufgeführten Ausführungsbeispiele 28 - 223 hergestellt:

| **Beispiel-Nr.** | **Struktur** | **Analytik (Angaben in °C beziehen sich auf den Schmelzpunkt)** | **Allgemeines Verfahren** |
|---|---|---|---|
| **28** | 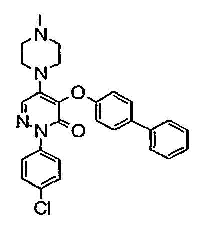 | DCI/NH₃: 490 (M+NH₄, 100%) HPLC:rt(%)= 7,38 (99,4) | 6a |
| **29** | 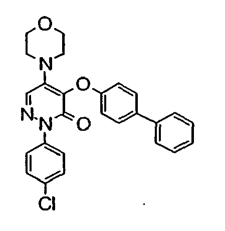 | DCI/NH₃: 460 (M+H, 100%) HPLC: rt (%) = 7,88 (98,3) | 6a |
| **30** | 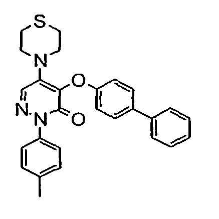 | DCI/NH₃: 456 (M+H, 100%) HPLC: rt (%) = 8,34 (78,7) | 6a |
| **31** | 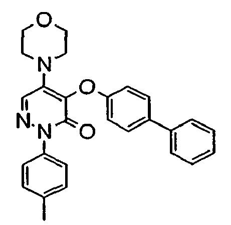 | DCI/NH₃: 440 (M+H, 100%) HPLC: rt (%) = 7,64 (98,1) | 6a |
| **32** | 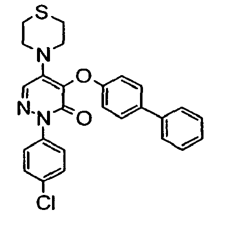 | DCI/NH₃: 476 (M+H, 100%) HPLC: rt (%) = 8,50 (97,9) | 6a |
| **33** | 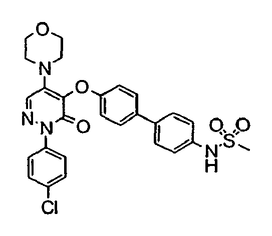 | DCI/NH₃: 553 (M+H, 100%) HPLC: rt (%) = 6,72 (94,0) | 6a |
| **34** | 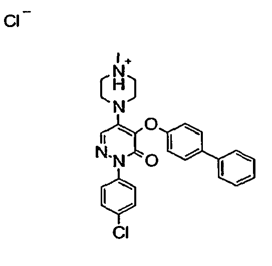 | Schmp.: 162°C ESI: 473 (M+H, 100) HPLC: rt (%) = 7,42 (99,6) ¹H-NMR (300 MHz, DMSO-d₆): δ = 2.7 (s, 3H), 3.0-4.2 (mehrere breite m, 8H), 7.1 (m, 2H), 7.3-7.7 (m, 11H), 8.3 (s, 1H) | 6a |
| **35** | 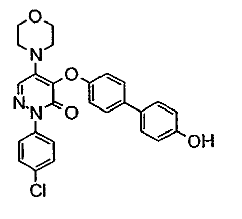 | DCI/NH₃: 476 (M+H, 100%) HPLC: rt (%) = 6,72 (96,7) | 6a |
| **36** | 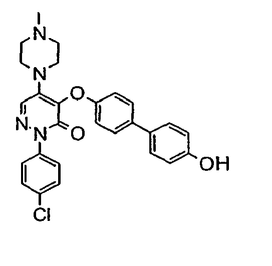 | DCI/NH₃: 489 (M+H, 100%) HPLC: rt (%) = 5,40 (93,3) | 6a |
| **37** | 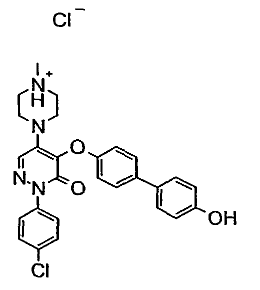 | DCI/NH3:489 (M+H, 100%) HPLC: rt (%) = 6,74 (100) | 6a |
| **38** | 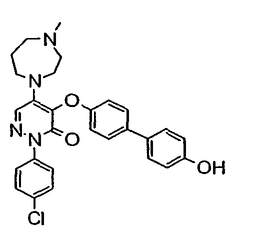 | DCI/NH3: 503 (M+H, 100%) HPLC: rt (%) = 6,80 (94,1) | 6a |
| **39** | 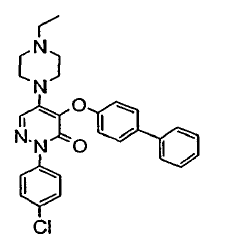 | DCI/NH3: 487 (M+H, 100%) HPLC: rt (%) = 7,44 (94,7) | 6a |
| **40** | 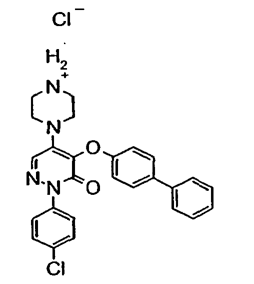 | MS (ESI): 459 (M+H) (freie Base) HPLC: rt (%) = 7,22 (99,9) | 7 |
| **41** | 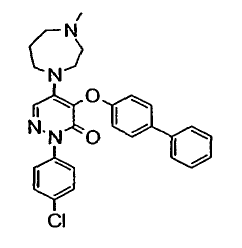 | ESI: 487 (M+H, 100%) HPLC: rt (%) = 7,47 (100) | 6a |
| **42** | 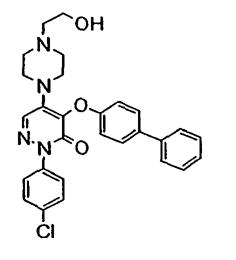 | ESI: 503 (M+H, 100%) HPLC: rt (%) = 7,20 (91,2) | 6a |
| **43** | 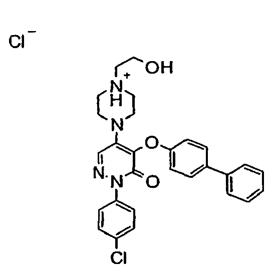 | ESI (CH₃CN/H₂O; 0, 1 % CH₃COOH): 503 (100%) HPLC: rt (%) = 6,07 (99,6) | 6a |
| **44** | 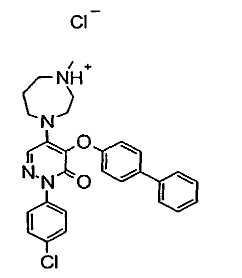 | ESI (CH₃CN/H₂O; 0,1% CH₃COOH): 487 (100%) HPLC: rt (%) = 7,42 (95,3) | 6a |
| **45** | 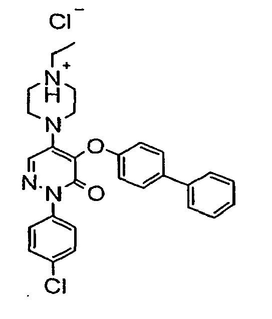 | ESI (CH₃CN/H₂O; 0,1% CH₃COOH): 487 (100%) HPLC: rt (%) = 6,27 (99,0) | 6a |
| **46** | 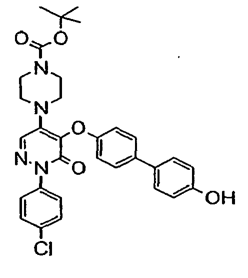 | DCI/NH₃: 592 (M+NH₄, 100%) HPLC: rt (%) = 9,86 (50,8) | 6a |
| **47** | 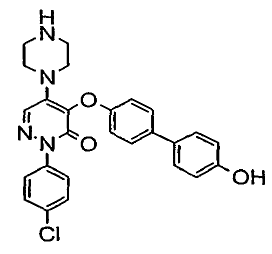 | DCI/NH₃: 492 (M+NH₄, 100%), 475 (M+H, 51 %) HPLC: rt (%) = 6,58 (88,8) | 7 |
| **48** | 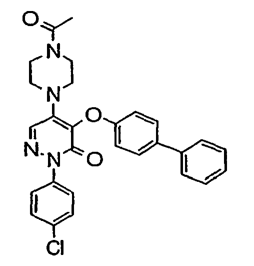 | ESI (CH₃CN/H₂O; 0,1% CH₃COOH): 501 (M+H, 100%) HPLC: rt (%) = 9,15 (91,6) | 2 |
| **49** | 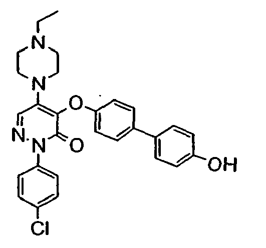 | DCI/NH3: 503 (M+H, 100%) HPLC: rt (%) = 6,74 (99,6) | 6a |
| **50** | 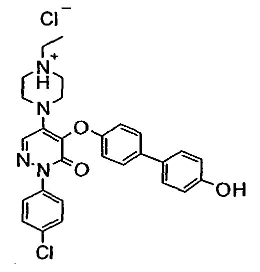 | DCI/NH₃: 502 (M+NH₄, 100%), 503 (M+H, 67%) HPLC: rt (%) = 6,69 (100) | 6a |
| **51** | 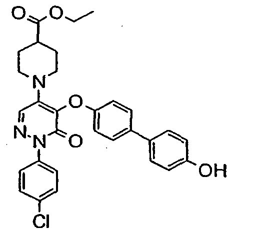 | DCI/NH₃: 563 (M+NH₄, 100%) HPLC: rt (%) = 9,69 (96,9) | 6a |
| **52** | 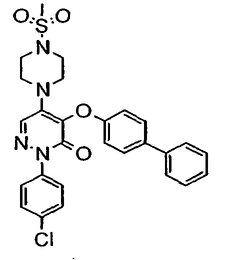 | ESI (CH₃CN/H₂O; Ammoniumacetat): 537 (100%) HPLC: rt (%) = 9,75 (98,0) | 4 |
| **53** | 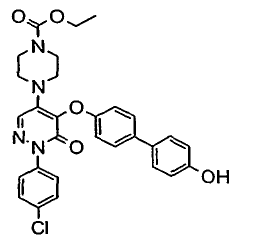 | ESI: 547 (M+H, 100%) HPLC: rt (%) = 9,08 (100) | 6a |
| **54** | 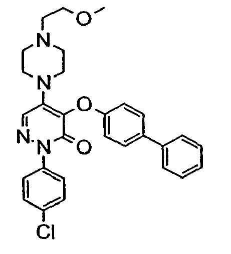 | ESI: 517 (100%) HPLC: rt (%)= 7,58 (97,0) | 6a |
| **55** | 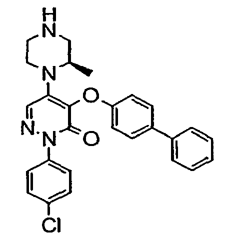 | DCI/NH₃: 473 (M+H, 100%) HPLC: rt (%) = 7,40 (92,4) | 7 |
| **56** | 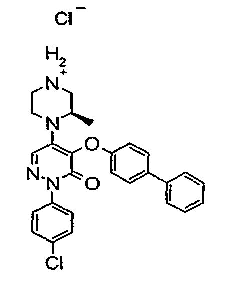 | DCI/NH₃: 473 (M+H) | 7 |
| **57** | 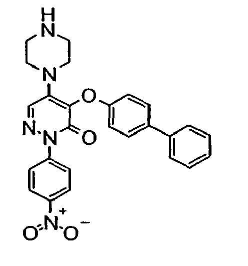 | DCI/NH₃: 470 (M+H, 100%) HPLC: rt (%) = 7,27 (100) | 7 |
| **58** | 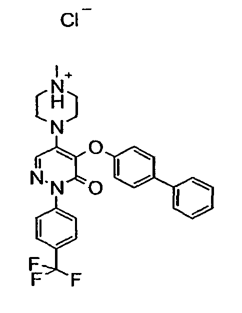 | HPLC: rt (%) = 7,65 (98,4) | 6a |
| **59** | 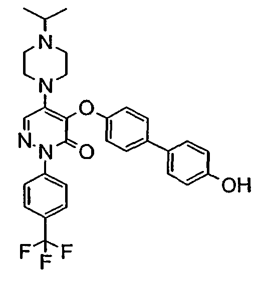 | ESI: 551 (M+H, 100%) HPLC: rt (%) = 6,96 (98,9) | 6a |
| **60** | 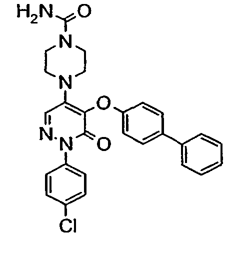 | ESI: 502 (M+H, 100%) HPLC: rt (%) = 8,51 (97,5) | 5 |
| **61** | 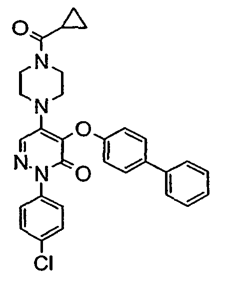 | ESI: 527 (M+H, 100%) HPLC: rt (%) = 9,65 (95,9) | 2 |
| **62** | 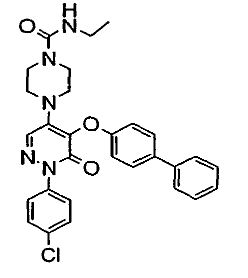 | ESI: 530 (M+H, 100%) HPLC: rt (%) = 9,22 (96,6) | 6a |
| **63** | 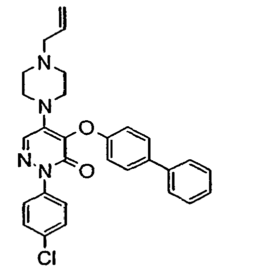 | ESI: 499 (M+H, 100%) HPLC: rt (%) = 7,66 (94,7) | 6a |
| **64** | 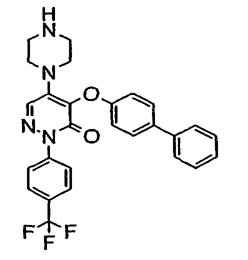 | ESI: 493 (M+H, 100%) HPLC: rt (%) = 7,64 (100) | 7 |
| **65** | 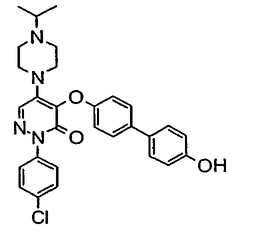 | ESI: 517 (M+H, 100%) R_{f}: 0.30 (Toluol/Acetonitril 4:1) | 6a |
| **66** | 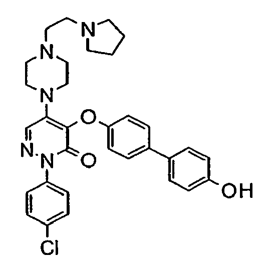 | DCI/NH₃: 572 (M+H, 100%) HPLC: rt (%) = 6,69 (100) | 6a |
| **67** | 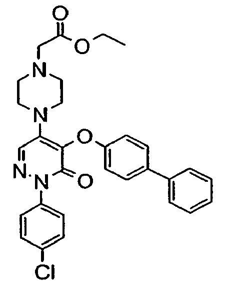 | DCI/NH₃: 545 (M+H, 100%) HPLC: rt (%) = 8,24 (99,3) | 6a |
| **68** | 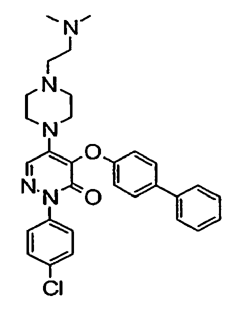 | DCI/NH₃: 530 (M+H, 100%) HPLC: rt (%) = 7,28 (73,6) | 6a |
| **69** | 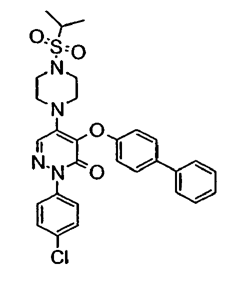 | DCI/NH3: 565 (M+H, 100%) HPLC: rt (%) = 10,12 (76,6) | 4 |
| **70** | 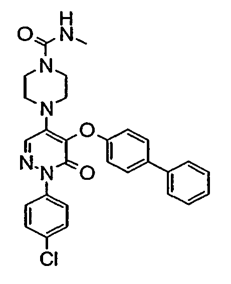 | ESI: 516 (M+H, 60%) HPLC: rt (%) = 8,90 (99,7) | 6a |
| **71** | 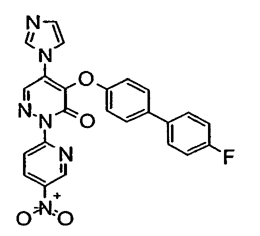 | 186°C | 16a |
| **72** | 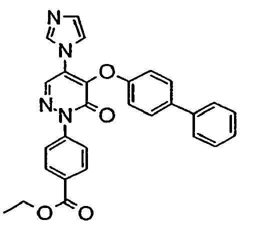 | 151°C | 16a |
| **73** | 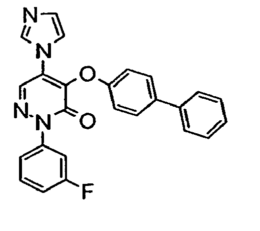 | 148°C | 16a |
| **74** | 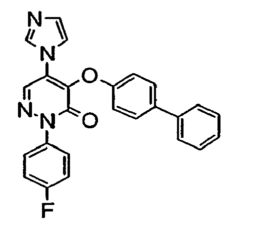 | 119°C | 16a |
| **75** | 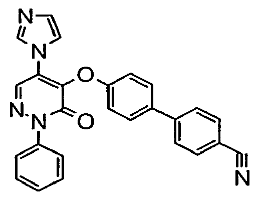 | | 16a |
| **76** | 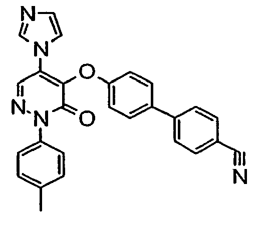 | 220°C | 16a |
| **77** | 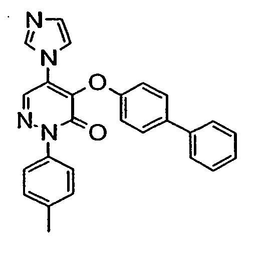 | 177°C | 16a |
| **78** | 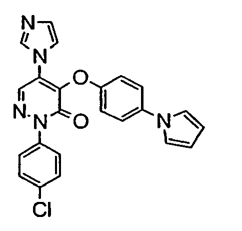 | 188°C | 16a |
| **79** | 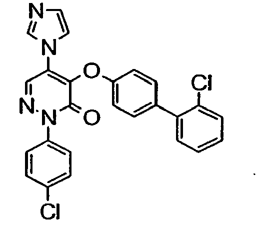 | | |
| **80** | 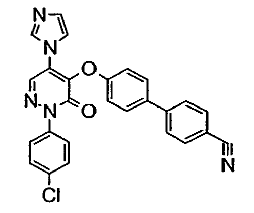 | 276°C | 16a |
| **81** | 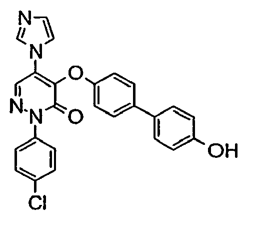 | 216°C ¹H-NMR (200 MHz, DMSO-d₆): δ = 6.7 (m, 2H), 7.2-7.3 (m, 3H), 7.4-7.8 (m, 9H), 8.25 (s,1H), 8.70 (s, 1H), 9.5 (s, 1H) | 16a |
| **82** | 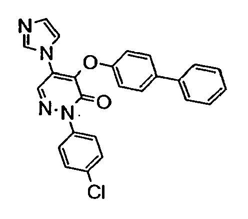 | 188°C ¹H-NNM (400 MHz, CDCl₃): δ = 7.15 (m, 2H, AA' eines AA'BB'-Systems), 7.2-7.6 (mehrere m, 11H), 7.65 (m, 2H, BB' eines AA'BB'-Systems), 8.1 (s, 1H), 8.2 (s, 1H) | 16c |
| **83** | 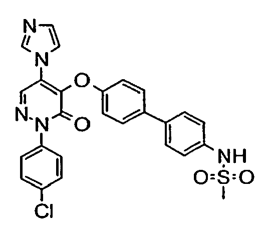 | 180°C | 16a |
| **84** | 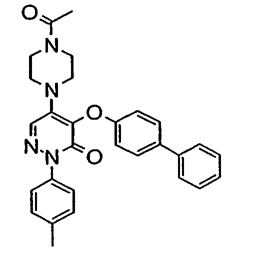 | MS (B): 481 (M+H) (100) HPLC: 8.77 (99) | 2 |
| **85** | 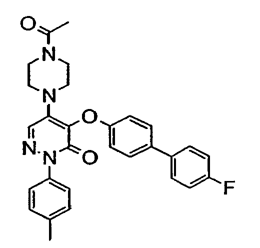 | MS (B): 499 (M+H) (100) HPLC: 8.84 (100) | 2 |
| **86** | 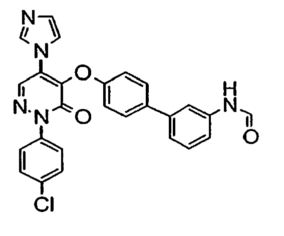 | MS (A): 484 (M+H) (100) HPLC: 7.45 (98) | 3 |
| **87** | 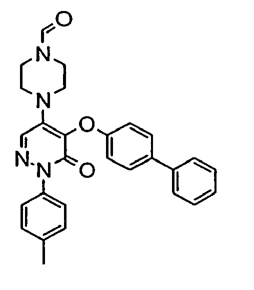 | MS (B): 467 (M+H) (100) HPLC: 8.73 (99) | 3 |
| **88** | 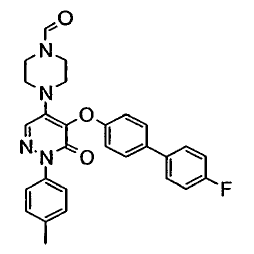 | MS (B): 485 (M+H) (100) HPLC: 8.80 (86) | 3 |
| **89** | 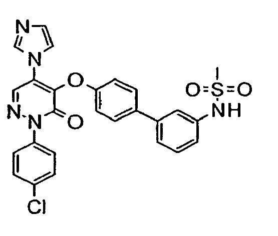 | MS (B): 534 (M+H) (100) HPLC: 7.83 (100) | 4 |
| **90** | 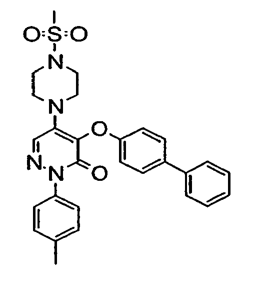 | MS (B): 517 (M+H) (100) HPLC: 9.38 (97) | 4 |
| **91** | 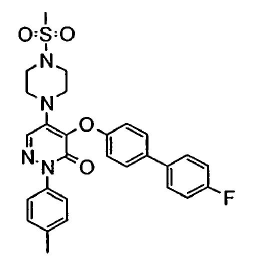 | MS (B): 535 (M+H) (100) HPLC: 9.44 (94) | 4 |
| **92** | 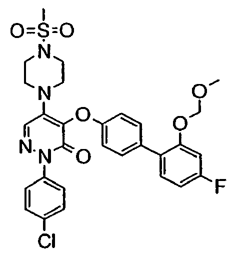 | MS (D): 615 (M+H) (100) HPLC: 9.75 (86) | 4 |
| **93** | 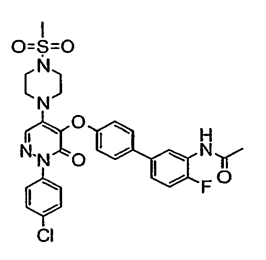 | MS (E): 612 (M+H) (100) HPLC: 8.47 (92) | 4 |
| **94** | 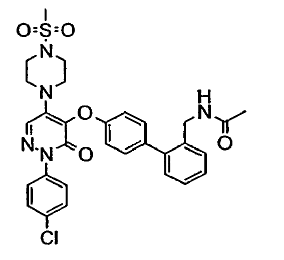 | MS (E): 608 (M+H) (100) HPLC: 8.30 (92) | 4 |
| **95** | 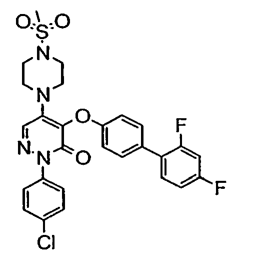 | MS (D): 573 (M+H) (100) HPLC: 9.75 (98) | 4 |
| **96** | 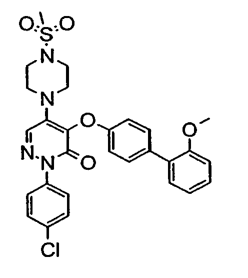 | MS (D): 567 (M+H) (100) HPLC: 9.62 (96) | 4 |
| **97** | 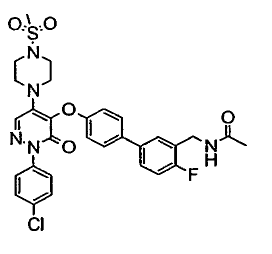 | MS (D): 626 (M+H) (100) HPLC: 8.34 (100) | 4 |
| **98** | 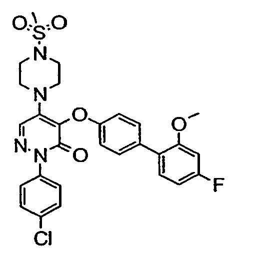 | MS (D): 585 (M+H) (100) HPLC: 9.71 (95) | 4 |
| **99** | 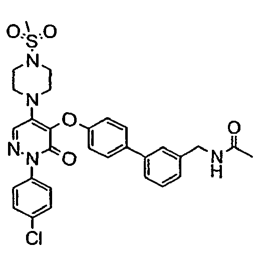 | MS (D): 608 (M+H) (100) HPLC: 8.19 (100) | 4 |
| **100** | 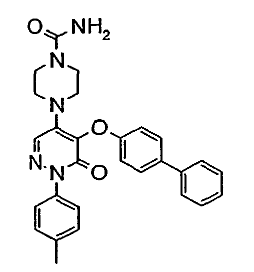 | MS (B): 482 (M+H) (100) HPLC: 8.21 (98) | 5 |
| **101** | 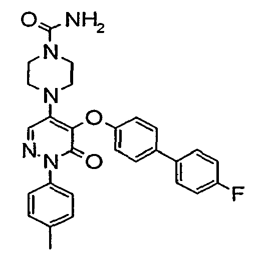 | MS (B): 500 (M+H) (100) HPLC: 8.28 (89) | 5 |
| **102** | 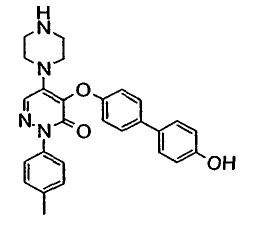 | MS (B): 455 (M+H) (100) HPLC: 4.98 (96) | 7 |
| **103** | 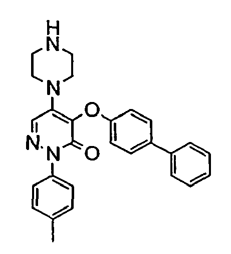 | MS (B): 439 (M+H) (100) HPLC: 6.89 (95) | 7 |
| **104** | 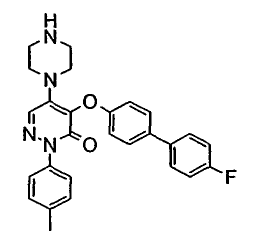 | MS (B): 457 (M+H) (100) HPLC: 7.12 (95) | 7 |
| **105** | 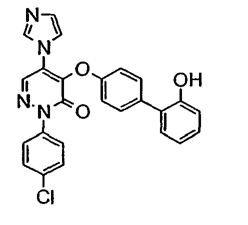 | MS (B): 457 (M+H) (100) HPLC: 7.90 (93) | 10 |
| **106** | 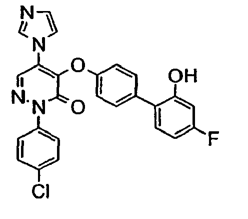 | MS (B): 475 (M+H) (100) HPLC: 3.78 (99) | 10 |
| **107** | 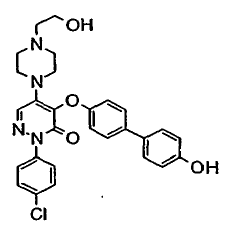 | MS (B): 519 (M+H) (100) HPLC: 6.48 (100) | 10 |
| **108** | 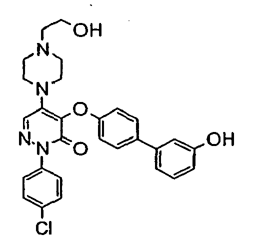 | MS (B): 519 (M+H) (100) HPLC: 6.53 (91) | 10 |
| **109** | 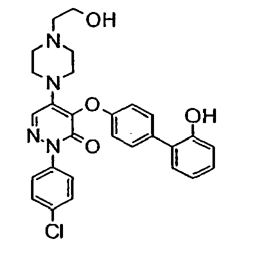 | MS (B): 519 (M+H) (100) HPLC: 6.65 (98) | 10 |
| **110** | 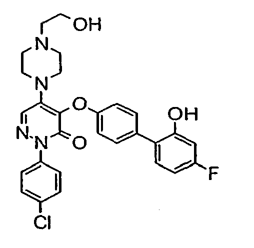 | MS (B): 537 (M+H) (100) HPLC: 6.84 (100) | 10 |
| **111** | 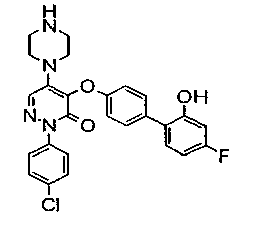 | MS (B): 493 (M+H) (100) HPLC: 6.86 (91) | 10 |
| **112** | 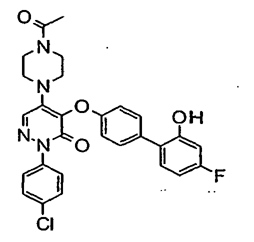 | MS (B): 535 (M+H) (100) HPLC: 8.45 (98) | 10 |
| **113** | 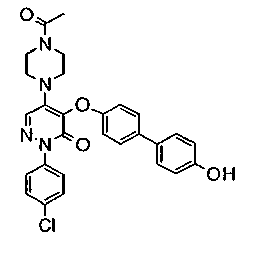 | MS (D): 517/519 (M+H) (100) HPLC: 7.89 (94) | 10 |
| **114** | 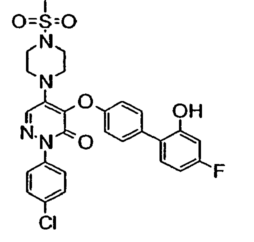 | MS (A): 571 (M+H) (100) HPLC: 9.01 (99) | 10 |
| **115** | 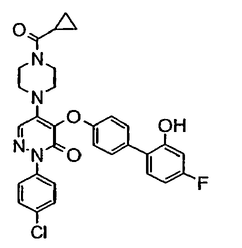 | MS (A): 561 (M+H) (100) HPLC: 8.97 (96) | 10 |
| **116** | 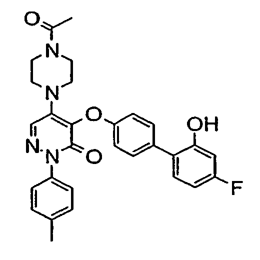 | MS (A): 515 (M+H) (100) HPLC: 8.18 (99) | 10 |
| **117** | 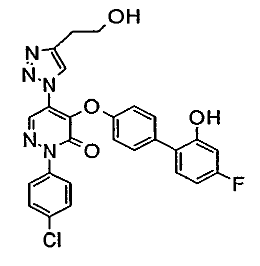 | MS (D): 520 (M+H) (100) HPLC: 8.72 (100) | 10 |
| **118** | 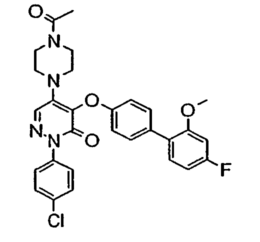 | MS (D): 549/551 (M+H) (100) HPLC: 9.31 (99) | 11 |
| **119** | 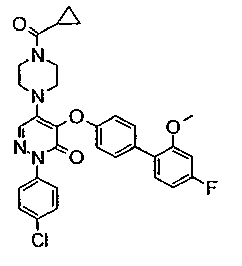 | MS (A): 575 (M+H) (100) HPLC: 9.72 (97) | 11 |
| **120** | 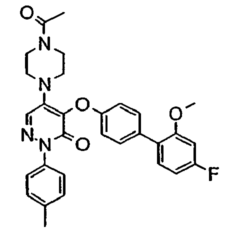 | MS (B): 529 (M+H) (100) HPLC: 8.89 (95) | 11 |
| **121** | 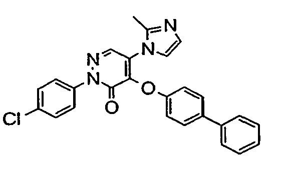 | MS (A): 455 (M+H) (100) HPLC: 7.94 (94) | 16a |
| **122** | 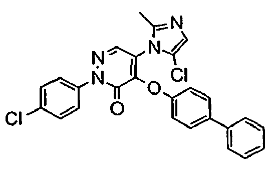 | MS (A): 489 (M+H) (100) HPLC: 10.15 (94) | 16a |
| **123** | 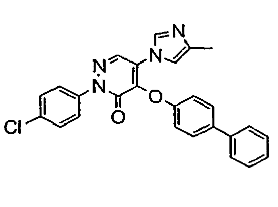 | MS (A): 455 (M+H) (100) HPLC: 8.15 (95) | 16a |
| **124** | 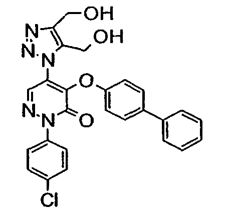 | MS (B): 502 (M+H) (100) HPLC: 8.69 (98) | 14 |
| **125** | 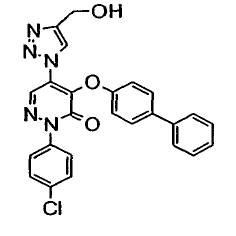 | MS (B): 472 (M+H) (100) HPLC: 9.33 (99) | 14 |
| **126** | 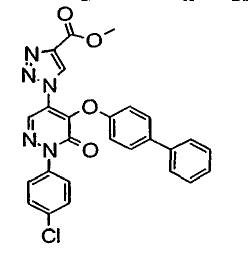 | MS (B): 500 (M+H) (100) HPLC: 10.20 (98) | 14 |
| **127** | 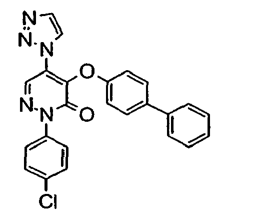 | MS (B): 442 (M+H) (100) HPLC: 10.12 (96) | 14 |
| **128** | 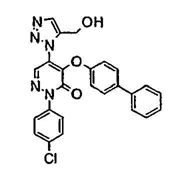 | MS (B): 472 (M+H) (100) HPLC: 9.20 (84) | 14 |
| **129** | 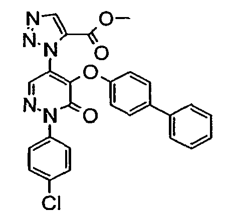 | MS (B): 500 (M+H) (100) HPLC: 10.21 (96) | 14 |
| **130** | 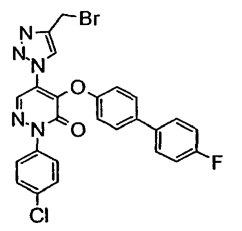 | MS (D): 552/554 (M+H) (100) HPLC: 10.60 (97) | 14 |
| **131** | 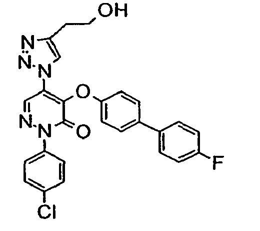 | MS (A): 504 (M+H) (100) HPLC: 9.37 (98) | 14 |
| **132** | 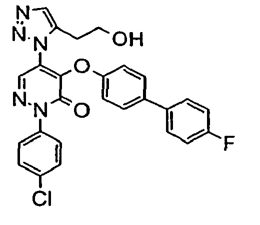 | MS (B): 504 (M+H) (100) HPLC: 9.16 (100) | 14 |
| **133** | 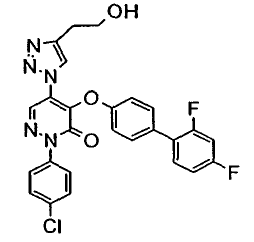 | MS (B): 522 (M+H) (100) HPLC: 9.78 (96) | |
| **134** | 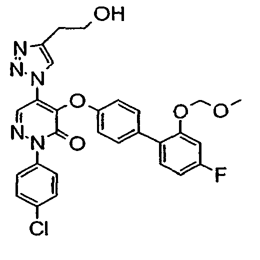 | MS (B): 564 (M+H) (100) HPLC: 9.94 (99) | 14 |
| **135** | 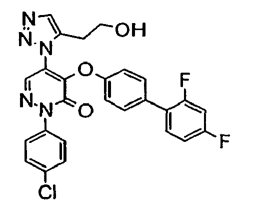 | MS (D): 522 (M+H) (100) HPLC: 9.22 (94) | 14 |
| **136** | 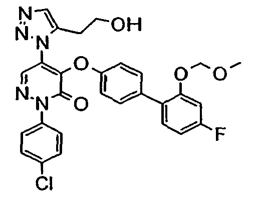 | MS (B): 563 (M⁺) (38) HPLC: 9.67 (99) | 14 |
| **137** | 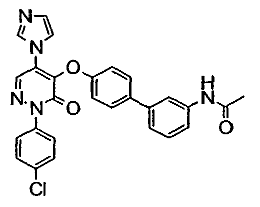 | MS (B): 49S (M+H) (100) HPLC: 7.58 (96) | |
| **138** | 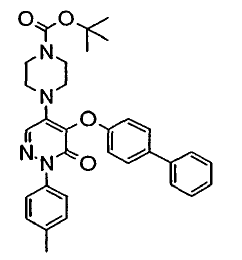 | MS (A): 539 (M+H) (100) HPLC: 10.89 (98) | 6 -a |
| **139** | 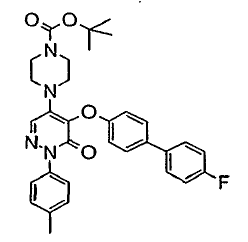 | MS (B): 557 (M+H) (100) | 6-a |
| **140** | 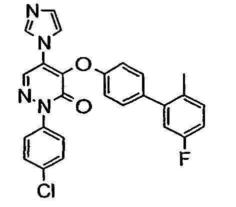 | MS (B): 473 (M+H) (100) HPLC: 9.25 (94) | 9-a |
| **141** | 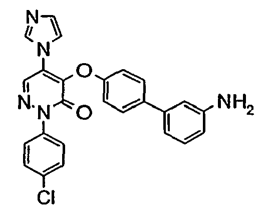 | MS (A): 473 (M+NH₄) (100), 456 (M+H) (52) HPLC: 6.64 (97) | 9-a |
| **142** | 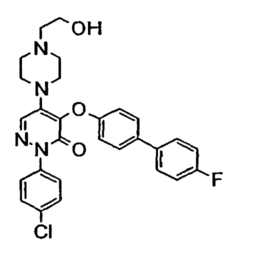 | MS (B): 521 (M+H) (100) HPLC: 7.45 (98) | 9-a |
| **143** | 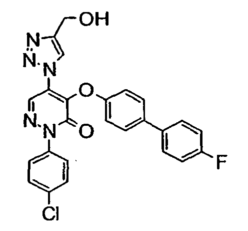 | MS (D): 490 (M+H) (100) HPLC: 9.36 (99) | 9-a |
| **144** | 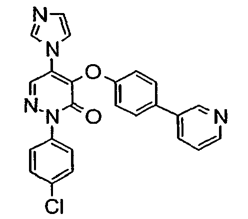 | MS (A): 442/444 (M+H) (100) HPLC: 6.75 (98) | 9-b |
| **145** | 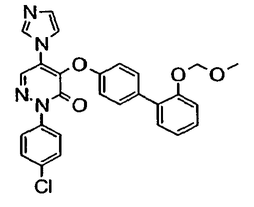 | MS (C): 500 (M) (100) HPLC: 8.80 (97) | 9-b |
| **146** | 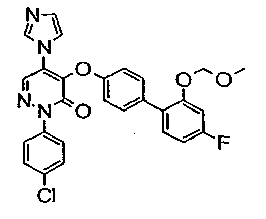 | MS (B): 541 (M+Na) (100), 519 (M+H) (74) HPLC: 8.95 (96) | 9-b |
| **147** | 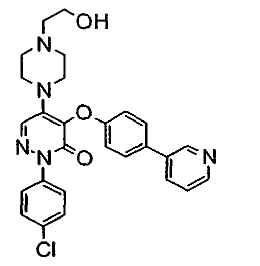 | MS (B): 504 (M+H) (100) HPLC: 5.20 (96) | 9-b |
| **148** | 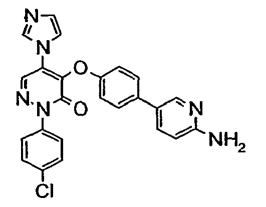 | MS (B): 457 (M+H) (100) HPLC: 5.93 (95) | 9-b |
| **149** | 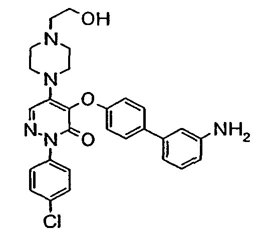 | MS (A): 518 (M+H) (100) HPLC: 5.55 (96) | 9-b |
| **150** | 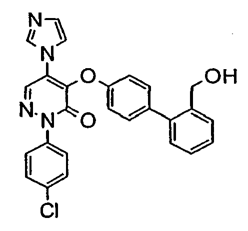 | MS (B): 471 (M+H) (100) HPLC: 7.71 (96) | 9-b |
| **151** | 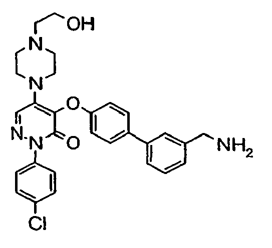 | MS (B): 532 (M+H) (100) HPLC: 5.32 (96) | 9-b |
| **152** | 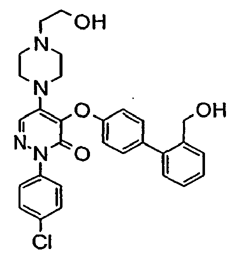 | MS (B): 533 (M+H) (100) HPLC: 6.68 (99) | 9-b |
| **153** | 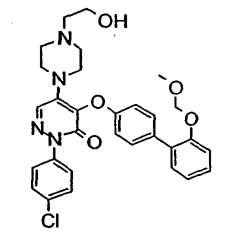 | MS (A): 563 (M+H) (100) HPLC: 7.30 (97) | 9-b |
| **154** | 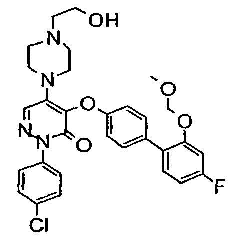 | MS (A): 581 (M+H) (100) HPLC: 7.51 (96) | 9-b |
| **155** | 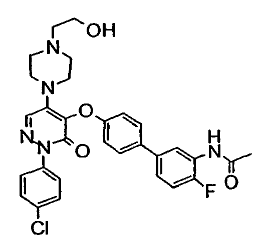 | MS (B): 578 (M+H) (100) HPLC: 6.51 (93) | 9-b |
| **156** | 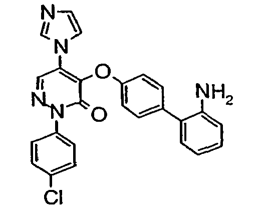 | MS (D): 456 (M+H) (100) HPLC: 3.83 (97) | 9-b |
| **157** | 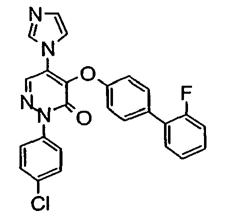 | MS (D): 459/461 (M+H) (100) HPLC: 4.47 (100) | 9-b |
| **158** | 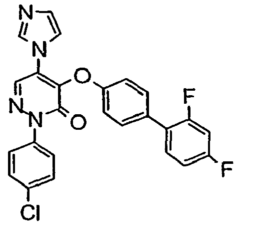 | MS (D): 477/479 (M+H) (100) HPLC: 4.53 (100) | 9-b |
| **159** | 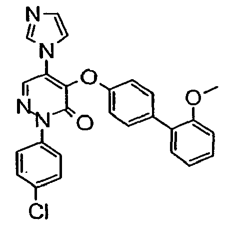 | MS (D): 471/473 (M+H) (100) HPLC: 4.45 (100) | 9-b |
| **160** | 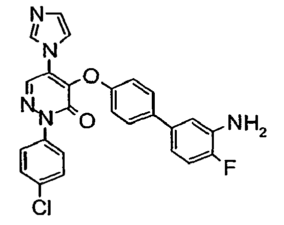 | MS (D): 474 (M+H) (100) HPLC: 4.05 (98) | 9-b |
| **161** | 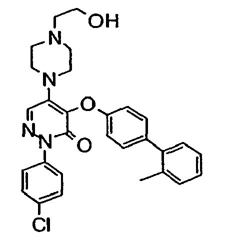 | MS (F): 517 (M+H) (100) | 9-b |
| **162** | 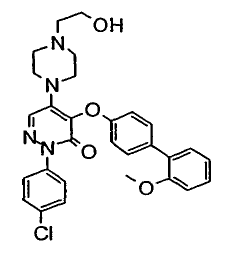 | MS (F): 533 (M+H) (100) | 9-b |
| **163** | 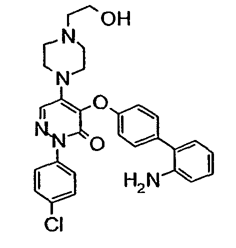 | MS (F): 518 (M+H) (100) | 9-b |
| **164** | 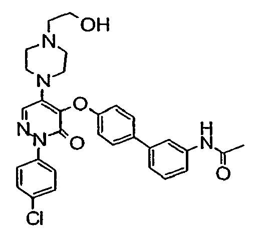 | MS (F): 560 (M+H) (100) | 9-b |
| **165** | 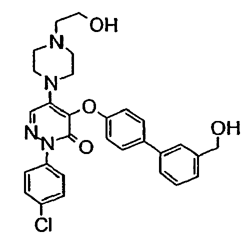 | MS (F): 533 (M+H) (100) | 9-b |
| **166** | 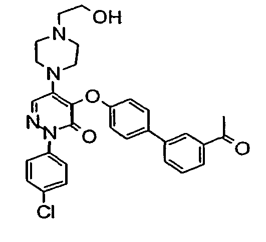 | MS (F): 545 (M+H) (100) | 9-b |
| **167** | 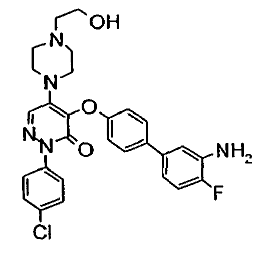 | MS (F): 536 (M+H) (100) | 9-b |
| **168** | 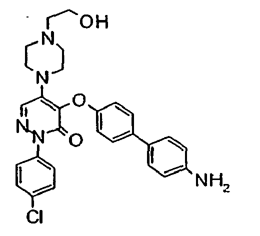 | MS (F): 518 (M+H) (100) | 9-b |
| **169** | 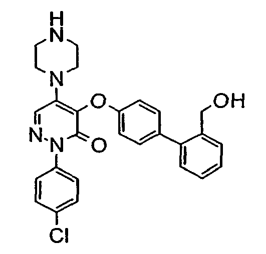 | MS (B): 489 (M+H) (100) HPLC: 6.60 (98) | 9-c |
| **170** | 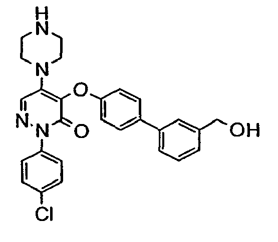 | MS (A): 489 (M+H) (100) HPLC: 6.48 (98) | 9-c |
| **171** | 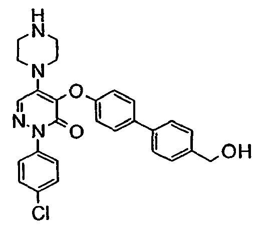 | MS (B): 489 (M+H) (100) HPLC: 6.37 (96) | 9-c |
| **172** | 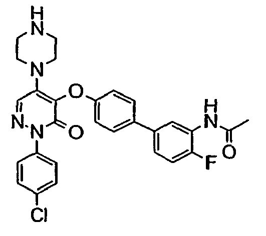 | MS (B): 534 (M+H) (100) HPLC: 6.55 (97) | 9-c |
| **173** | 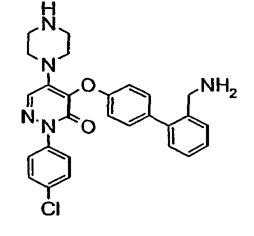 | MS (B): 488 (M+H) (100) HPLC: 6.33 (99) | 9-c |
| **174** | 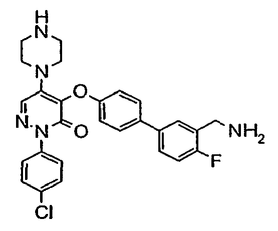 | MS (B): 506 (M+H) (100) HPLC: 5.39 (98) | 9-c |
| **175** | 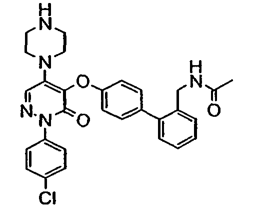 | MS (B): 530 (M+H) (100) HPLC: 6.54 (100) | 9-c |
| **176** | 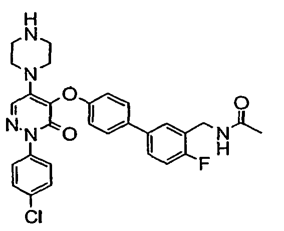 | MS (B): 548 (M+H) (100) HPLC: 6.55 (98) | 9-c |
| **177** | 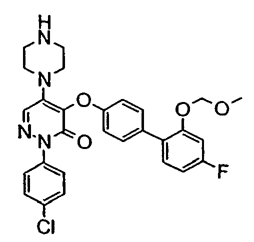 | MS (B): 537 (M+H) (100) HPLC: 7.46 (94) | 9-c |
| **178** | 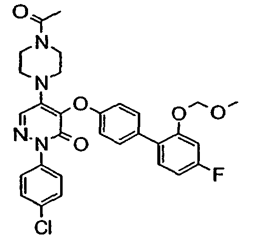 | MS (B): 579 (M+H) (100) HPLC: 9.21 (93) | 9-c -c |
| **179** | 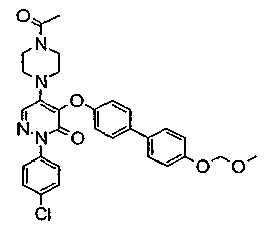 | MS (E): 561 (M+H) (100) HPLC: 8.91 (99) | 9-c |
| **180** | 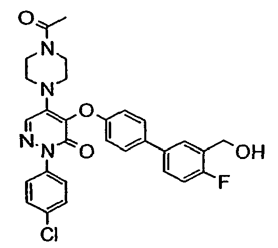 | MS (E): 549 (M+H) (100) HPLC: 8.04 (97) | 9-c |
| **181** | 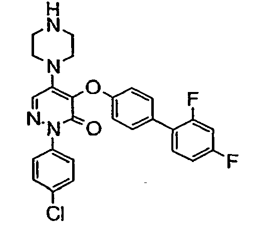 | MS (B): 495 (M+H) (100) HPLC: 7.47 (94) | 9-c |
| **182** | 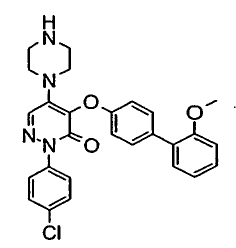 | MS (B): 489 (M+H) (100) HPLC: 7.25 (92) | 9-c |
| **183** | 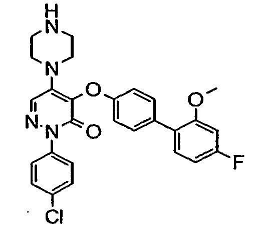 | MS (D): 507 (M+H) (100) HPLC: 7.40 (91) | 9-c |
| **184** | 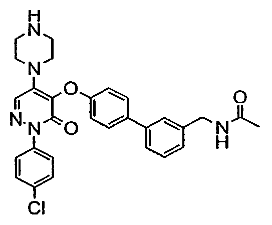 | MS (D): 530 (M+H) (100) HPLC: 6.37 (96) | 9-c |
| **185** | 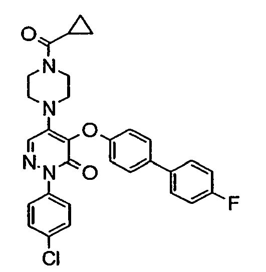 | MS (A): 545 (M+H) (100) HPLC: 9.67 (92) | 9-c |
| **186** | 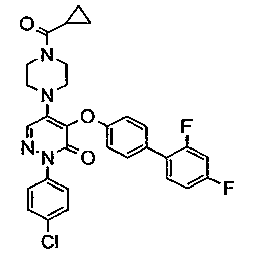 | MS (A): 563 (M+H) (100) HPLC: 9.77 (91) | 9-c |
| **187** | 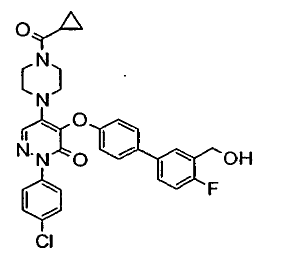 | MS (D): 575 (M+H) (100) HPLC: 8.57 (97) | 9-c |
| **188** | 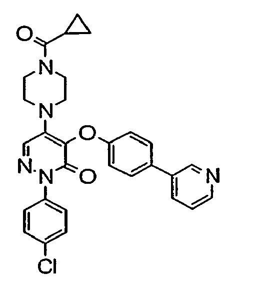 | MS (D): 528 (M+H) (100) HPLC: 6.55 (95) | 9-c |
| **189** | 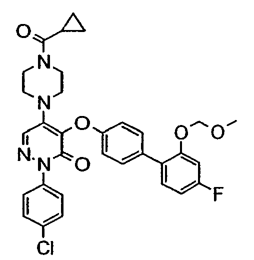 | MS (A): 605 (M+H) (100) HPLC: 9.74 (99) | 9-c |
| **190** | 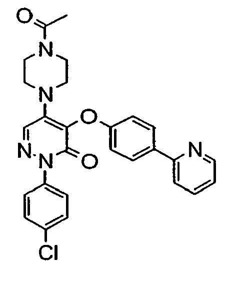 | MS (A): 502 (M+H) (100) HPLC: 6.16 (92) | 9-c |
| **191** | 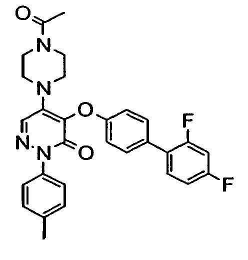 | MS (A): 517 (M+H) (100) HPLC: 8.93 (98) | 9-c |
| **192** | 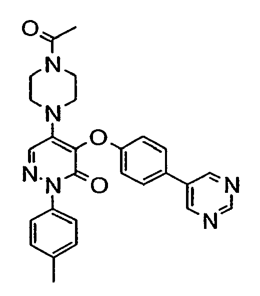 | MS (A): 483 (M+H) (100) HPLC: 6.79 (98) | 9-c |
| **193** | 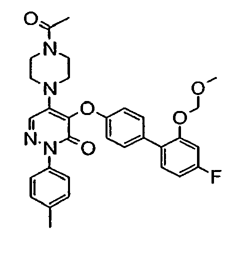 | MS (A): 559 (M+H) (100) HPLC: 8.92 (97) | 9-c |
| **194** | 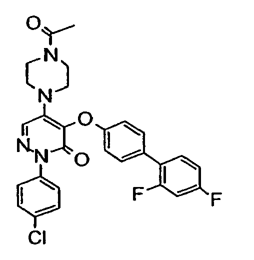 | MS (F): 537 (M+H) (100) HPLC: 4.59 (100) | 9-c |
| **195** | 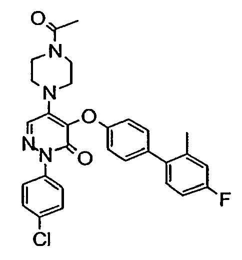 | MS (F): 533 (M+H) (100) HPLC: 4.69 (100) | 9-c |
| **196** | 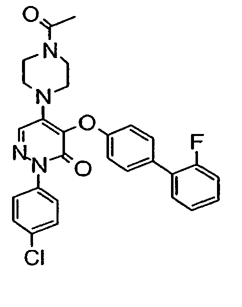 | MS (F): 519 (M+H) (100) HPLC: 4.51 (100) | 9-c -c |
| **197** | 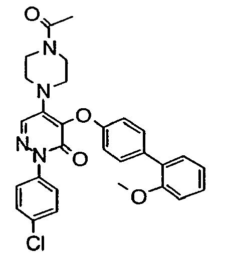 | MS (F): 531 (M+H) (100) HPLC: 4.50 (100) | 9-c |
| **198** | 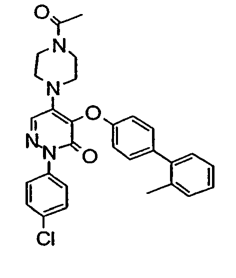 | MS (F): 515 (M+H) (100) HPLC: 4.64 (100) | 9-c |
| **199** | 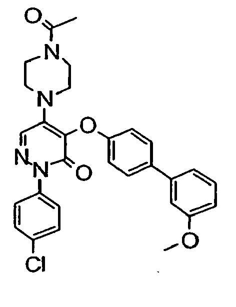 | MS (F): 531 (M+H) (100) HPLC: 4.47 (100) | 9-c |
| **200** | 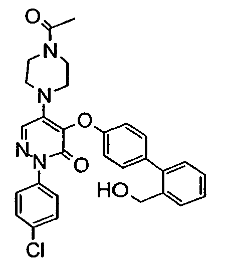 | MS (F): 531 (M+H) (100) HPLC: 3.99 (93) | 9-c |
| **201** | 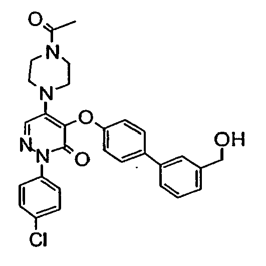 | MS (F): 531 (M+H) (100) HPLC: 3.92 (94) | 9-c |
| **202** | 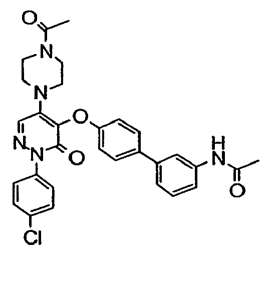 | MS (F): 558 (M+H) (100) HPLC: 3.90 (100) | 9-c |
| **203** | 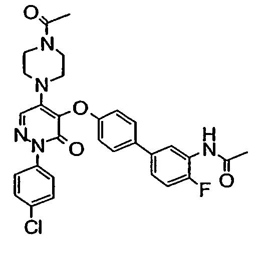 | MS (F): 576 (M+H) (100) HPLC: 3.94 (93) | 9-c |
| **204** | 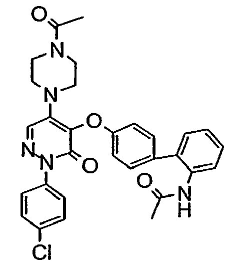 | MS (F): 558 (M+H) (100) HPLC: 3.84 (100) | 9-c |
| **205** | 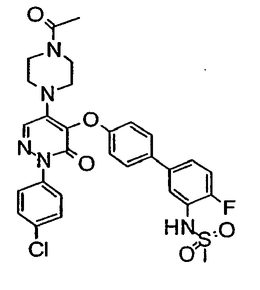 | MS (F): 612 (M+H) (100) HPLC: 4.01 (100) | 9-c |
| **206** | 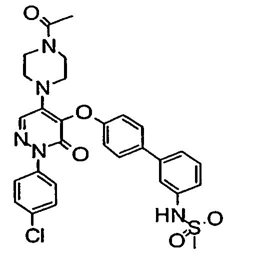 | MS (F): 594 (M+H) (100) HPLC: 4.02 (100) | 9-c |
| **207** | 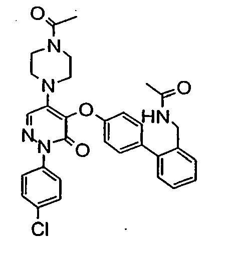 | MS (F): 572 (M+H) (100) HPLC: 3.87 (100) | 9-c |
| **208** | 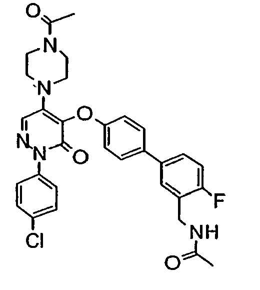 | MS (F): 590 (M+H) (100) HPLC: 3.90 (100) | 9-c |
| **209** | 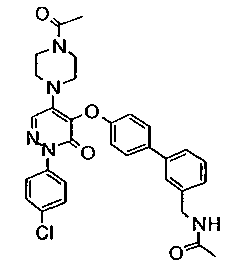 | MS (F): 572 (M+H) (100) HPLC: 3.84 (100) | 9-c |
| **210** | 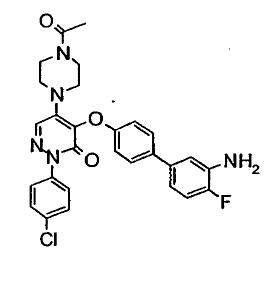 | MS (F): 534 (M+H) (100) HPLC: 4.00 (100) | 9-c |
| **211** | 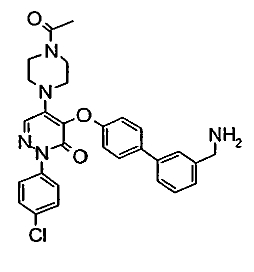 | MS (F): 530 (M+H) (100) HPLC: 3.27 (100) | 9-c |
| **212** | 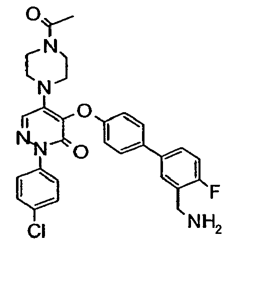 | MS (F): 548 (M+H) (100) HPLC: 3.28 (100) | 9-c |
| **213** | 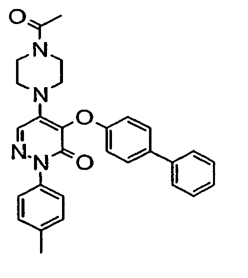 | MS (C): 481 (M+H) (100); HPLC: 8.77 (98) | 3 |
| **214** | 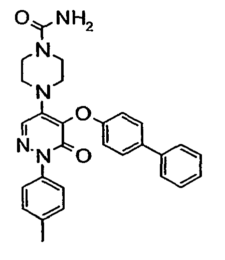 | MS (C): 482 (M+H) (100); HPLC: 8.22 (82) | 5 |
| **215** | 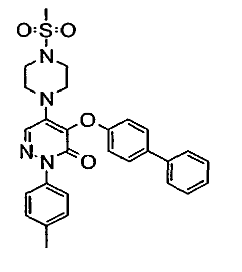 | MS (C): 517 (M+H) (100); HPLC: 9.38 (97) | 4 |
| **216** | 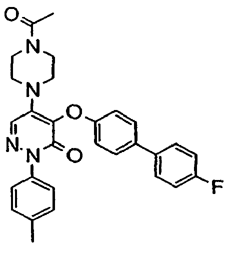 | MS (C): 499 (M+H) (100); HPLC: 8.84 (100) | 2 |
| **217** | 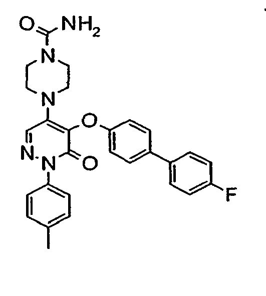 | MS (C): 500 (M+H) (100); HPLC: 8.28 (89) | 5 |
| **218** | 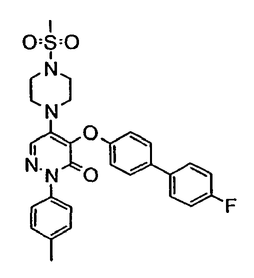 | MS (C): 535 (M+H) (100); HPLC: 9.44 (94) | 4 |
| **219** | 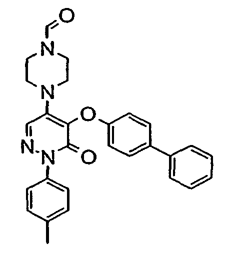 | MS (C): 467 (M+H) (100); HPLC: 8.73 (99) | 3 |
| **220** | 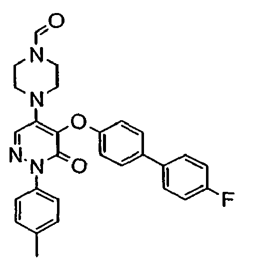 | MS (C): 485 (M+H) (100); HPLC: 8.80 (86) | 3 |
| **221** | 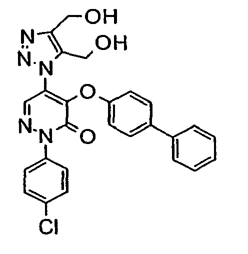 | MS (C): 558 (M+H) (100); HPLC: 10.32 (99) | 14 |
| **222** | 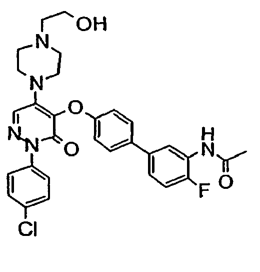 | | 1 |
| **223** | 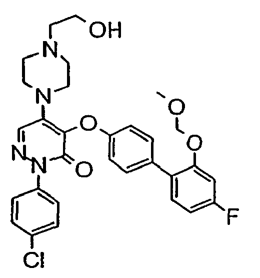 | MS (C): 582 (M+H) (100); HPLC: 7.39 (96) | 1 |

## Patentansprüche

1. Verbindungen der Formel (I) 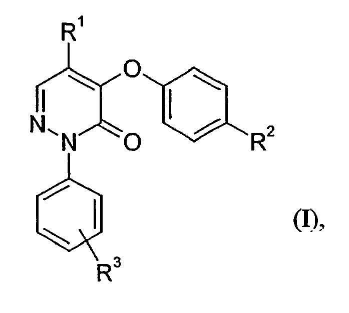 in welcher
R¹ für 5- bis 7-gliedriges, gesättigtes oder partiell ungesättigtes, über ein Ring-Stickstoffatom verknüpftes Heterocyclyl mit gegebenenfalls einem weiteren Heteroatom oder Heterokettenglied aus der Reihe N, O, S, SO oder SO₂ steht, das ein- oder zweifach, gleich oder verschieden, durch Substituenten ausgewählt aus der Gruppe Halogen, (C₁-C₆)-Alkyl, (C₂-C₆)-Alkenyl, (C₃-C₈)-Cycloalkyl, Hydroxy, Oxo, Carboxyl, (C₁-C₆)-Alkoxycarbonyl, (C₁-G₆)-All:anoyl, (C₃-C₈)-Cycloalkylcarbonyl, (C₁-C₆)-Alkylsulfonyl, Aminocarbonyl, 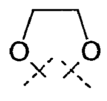 und (C₁-C₆)-Alkylaminocarbonyl substituiert sein kann,
wobei (C₁-C₆)-Alkyl und (C₁-C₆)-Alkanoyl ihrerseits jeweils durch Halogen, Hydroxy, (C₁-C₄)-Alkoxy, (C₁-C₄)-Alkoxycarbonyl, Amino, Mono- oder Di-(C₁-C₄)-alkylamino, (C₁-C₄)-Alkoxycarbonylamino oder 5- oder 6-gliedriges Heterocyclyl mit bis zu zwei Heteroatomen aus der Reihe N, O und/oder S substituiert sein können,
oder
R¹ für 5-gliedriges, über ein Ring-Stickstoffatom verknüpftes Heteroaryl mit bis zu zwei weiteren Ring-Stickstoffatomen steht, das ein- bis dreifach, gleich oder verschieden, durch Halogen, (C₁-C₆)-Alkoxycarbonyl oder (C₁-C₆)-Alkyl, welches seinerseits gegebenenfalls durch Hydroxy oder Halogen substituiert ist, substituiert sein kann,
R² für (C₆-C₁₀)-Aryl steht, das ein- oder zweifach, gleich oder verschieden, durch Substituenten ausgewählt aus der Gruppe Halogen, Nitro, Cyano, (C₁-C₆)-Alkyl, Trifluormethyl, (C₁-C₆)-Alkanoyl, (C₁-C₆)-Alkoxy, Hydroxy, (C₁-C₆)-Acyloxy, Amino, (C₁-C₆)-Acylamino, Mono- und Di-[(C₁C₆)-alkylsulfonyl]amino substituiert sein kann,
wobei (C₁-C₆)-Alkyl und (C₁-C₆)-Alkoxy ihrerseits jeweils durch Hydroxy, Amino, (C₁-C₄)-Alkoxy oder (C₁-C₄)-Acylamino substituiert sein können,
oder
R² für 5- oder 6-gliedriges Heteroaryl mit bis zu zwei Ring-Stickstoffatomen steht, das durch Amino, Hydroxy, Halogen, (C₁-C₆)-Alkyl oder (C₁-C₆)-Alkoxy substituiert sein kann,
und
R³ für Wasserstoff, Halogen, (C₁-C₆)-Alkyl, Trifluormethyl, Nitro, Cyano, Carboxyl oder (C₁-C₆)-Alkoxycarbonyl steht
und ihre Salze, Solvate und Solvate der Salze.

2. Verbindungen der Formel (I) nach Anspruch 1,
in welcher
R¹ für eine Gruppe der Formel 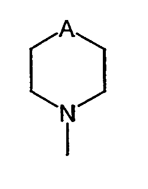 steht, worin
A für CR⁴R⁵, O, S, NR⁶ oder -CH₂NR⁶- steht, wobei
R⁴ und R⁵ unabhängig voneinander für Wasserstoff, (C₁-C₄)-Alkyl, das durch Hydroxy substituiert sein kann, Hydroxy, Fluor, Carboxyl oder (C₁-C₄)-Alkoxycarbonyl stehen oder gemeinsam mit dem Kohlenstoffatom, an das sie gebunden sind, eine Carbonylgruppe bilden,
und
R⁶ für Wasserstoff, (C₂-C₄)-Alkenyl, (C₃-C₆)-Cycloalkyl, (C₁-C₄)-Alkoxycarbonyl, Formyl, Acetyl, (C₃-C₆)-Cycloalkylcarbonyl, (C₁-C₄)-Alkylsulfonyl, Aminocarbonyl, (C₁-C₄)-Alkylaminocarbonyl oder für (C₁-C₄)-Alkyl steht, welches seinerseits durch Hydroxy, Methoxy, Ethoxy, (C₁-C₄)-Alkoxycarbonyl, Amino, Dimethylamino, Diethylamino, Pyrrolidino, Piperidino oder Morpholino substituiert sein kann,
oder
R¹ für 5-gliedriges, über ein Ring-Stickstoffatom verknüpftes Heteroaryl mit bis zu zwei weiteren Ring-Stickstoffatomen steht, das ein- oder zweifach, gleich oder verschieden, durch Fluor, Chlor, (C₁-C₄)-Alkoxycarbonyl oder (C₁-C₄)-Alkyl, welches seinerseits gegebenenfalls durch Hydroxy substituiert ist, substituiert sein kann,
R² für Phenyl steht, das ein- oder zweifach, gleich oder verschieden, durch Substituenten ausgewählt aus der Gruppe Fluor, Chlor, Cyano, (C₁-C₄)-Alkyl, Trifluormethyl, Formyl, Acetyl, (C₁-C₄)-Alkoxy, Hydroxy, Acetoxy, Pivaloyloxy, Amino, Formylamino, Acetylamino und Methylsulfonylamino substituiert sein kann,
wobei (C₁-C₄)-Alkyl und (C₁-C₄)-Alkoxy ihrerseits jeweils durch Hydroxy, Amino, Methoxy, Ethoxy oder Acetylamino substituiert sein können,
oder
R² für Pyrrolyl, Pyridyl oder Pyrimidinyl steht, die jeweils durch Amino, Fluor, Chlor, Methyl, Ethyl, Methoxy oder Ethoxy substituiert sein können,
und
R³ für Wasserstoff, Fluor, Chlor, Brom, Methyl, Ethyl, Trifluormethyl, Nitro oder Cyano steht
und ihre Salze, Solvate und Solvate der Salze.

3. Verbindungen der Formel (I) nach Anspruch 1,
in welcher
R¹ für über ein Ring-Stickstoffatom angebundenes Imidazolyl oder für über ein Ring-Stickstoffatom angebundenes Piperazinyl, das am zweiten Ring-Stickstoffatom durch Methyl, Ethyl, 2-Hydroxyethyl, 2-Methoxyethyl, Acetyl, tert.-Butoxycarbonyl oder Methylsulfonyl substituiert sein kann, steht,
R² für Phenyl, das in 4-Position zur Anknüpfstelle an den Phenylring durch Fluor oder Hydroxy substituiert sein kann, steht,
und
R³ sich in 4-Position zur Anknüpfstelle desPyridazinonrings befindet und für Wasserstoff, Fluor, Chlor, Methyl oder Trifluormethyl steht
und ihre Salze, Solvate und Solvate der Salze.

4. Verbindungen der Formel (I) nach Anspruch 1 mit den folgenden Strukturen: 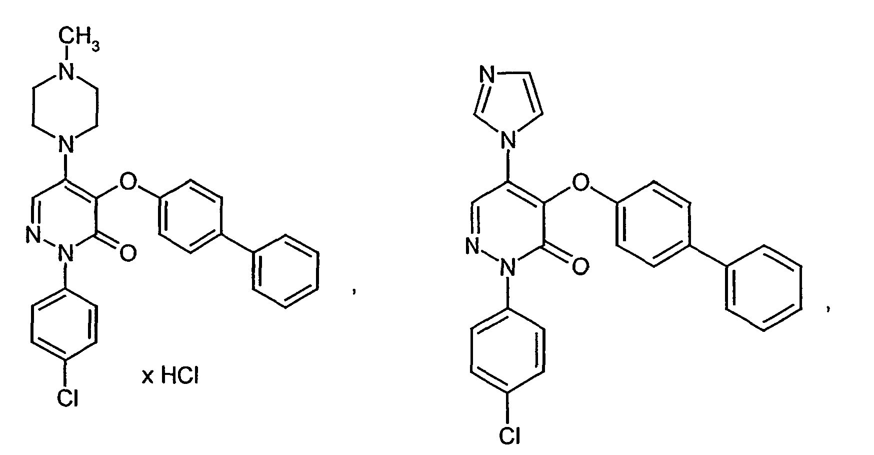 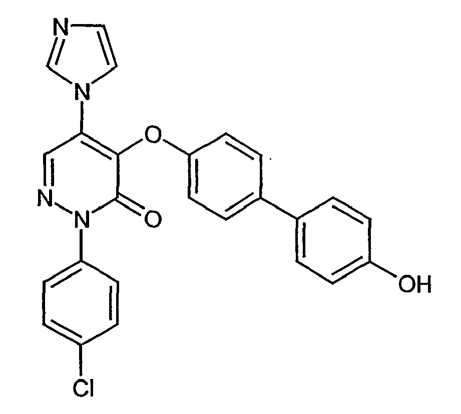 und ihre Salze, Solvate und Solvate der Salze.

5. Verfahren zur Herstellung der Verbindungen der Formel (I), wie in Anspruch 1 definiert, **dadurch gekennzeichnet, dass** man Verbindungen der Formel (II) 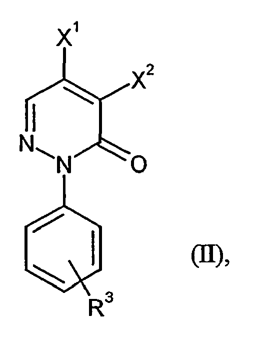 in welcher
R³ die in Anspruch 1 angegebene Bedeutung hat, und
X¹ und X² jeweils für Halogen, vorzugsweise für Brom oder Chlor stehen,
zunächst mit einer Verbindung der Formel (III)
R¹-H (III),
in welcher R¹ die in Anspruch 1 angegebene Bedeutung hat,
in Verbindungen der Formel (IV) 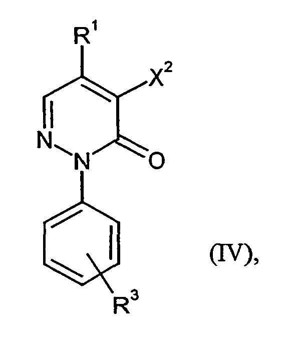 in welcher R¹, R³ und X² jeweils die oben angegebene Bedeutung haben,
überführt und diese dann mit einer Verbindung der Formel (V) - 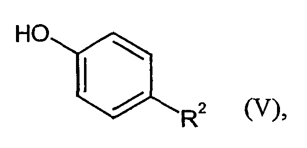 in welcher R² die in Anspruch 1 angegebene Bedeutung hat,
umsetzt.

6. Verbindungen der Formel (I), wie in Anspruch 1 definiert, zur Prophylaxe und/oder Behandlung von Erkrankungen.

7. Arzneimittel, enthaltend mindestens eine Verbindung der Formel (I), wie in Anspruch 1 definiert, und mindestens einen weiteren Hilfsstoff.

8. Arzneimittel, enthaltend mindestens eine Verbindung der Formel (I), wie in Anspruch 1 definiert, und mindestens einen weiteren Wirkstoff.

9. Verwendung von Verbindungen der Formel (I), wie in Anspruch 1 definiert, zur Herstellung von Arzneimitteln zur Prophylaxe und/oder Behandlung von fibrotischen Erkrankungen.

## Claims

1. Compounds of the formula (I) 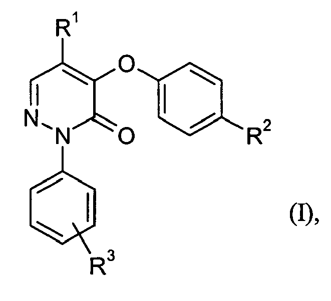 in which
R¹ is 5- to 7-membered, saturated or partially unsaturated heterocyclyl which is linked via a ring nitrogen atom and optionally has a further heteroatom or hetero chain member from the series N, O, S, SO or SO₂, and which may be substituted once or twice, identically or differently, by substitutents selected from the group of halogen, (C₁-C₆)-alkyl, (C₂-C₆)-alkenyl, (C₃-C₈)-cycloalkyl, hydroxy, oxo, carboxyl, (C₁-C₆)-alkoxycarbonyl, (C₁-C₆)-alkanoyl, (C₃-C₈)-cycloalkylcarbonyl, (C₁-C₆)-alkylsulfonyl, aminocarbonyl, 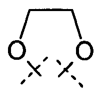 and (C₁-C₆)-alkylaminocarbonyl,
where (C₁-C₆)-alkyl and (C₁-C₆)-alkanoyl in turn may each be substituted by halogen, hydroxy, (C₁-C₄)-alkoxy, (C₁-C₄)-alkoxycarbonyl, amino, mono- or di-(C₁-C₄)-alkylamino, (C₁-C₄)-alkoxycarbonylamino or 5- or 6-membered heterocyclyl having up to two heteroatoms from the series N, O and/or S,
or
R¹ is 5-membered heteroaryl which is linked via a ring nitrogen atom and has up to two further ring nitrogen atoms, and which may be substituted once to three times, identically or differently, by halogen, (C₁-C₆)-alkoxycarbonyl or (C₁-C₆)-alkyl which is in turn optionally substituted by hydroxy or halogen,
R² is (C₆-C₁₀)-aryl which may be substituted once or twice, identically or differently, by substituents selected from the group of halogen, nitro, cyano, (C₁-C₆)-alkyl, trifluoromethyl, (C₁-C₆)-alkanoyl, (C₁-C₆)-alkoxy, hydroxy, (C₁-C₆)-acyloxy, amino, (C₁-C₆)-acylamino, mono- and di-[(C₁-C₆)-alkylsulfonyl]amino,
where (C₁-C₆)-alkyl and (C₁-C₆)-alkoxy in turn may each be substituted by hydroxy, amino, (C₁-C₄)-alkoxy or (C₁-C₄)-acylamino,
or
R² is 5- or 6-membered heteroaryl which has up to two ring nitrogen atoms and which may be substituted by amino, hydroxy, halogen, (C₁-C₆)-alkyl or (C₁-C₆)-alkoxy,
and
R³ is hydrogen, halogen, (C₁-C₆)-alkyl, trifluoromethyl, nitro, cyano, carboxyl or (C₁-C₆)-alkoxycarbonyl,
and the salts, solvates and solvates of the salts thereof.

2. Compounds of the formula (I) according to Claim 1,
in which
R¹ is a group of the formula 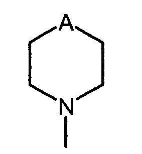 in which
A is CR⁴R⁵, O, S, NR⁶ or -CH₂NR⁶-, where
R⁴ and R⁵ are independently of one another hydrogen, (C₁-C₄)-alkyl, which may be substituted by hydroxy, or hydroxy, fluorine, carboxyl or (C₁-C₄)-alkoxycarbonyl, or together with the carbon atom to which they are bonded form a carbonyl group,
and
R⁶ is hydrogen, (C₂-C₄)-alkenyl, (C₃-C₆)-cycloalkyl, (C₁-C₄)-alkoxycarbonyl, formyl, acetyl, (C₃-C₆)-cycloalkylcarbonyl, (C₁-C₄)-alkylsulfonyl, aminocarbonyl, (C₁-C₄)-alkylaminocarbonyl or is (C₁-C₄)-alkyl which in turn may be substituted by hydroxy, methoxy, ethoxy, (C₁-C₄)-alkoxycarbonyl, amino, dimethylamino, diethylamino, pyrrolidino, piperidino or morpholino,
or
R¹ is 5-membered heteroaryl which is linked via a ring nitrogen atom and has up to two further ring nitrogen atoms and which may be substituted once or twice, identically or differently, by fluorine, chlorine, (C₁-C₄)-alkoxycarbonyl or (C₁-C₄)-alkyl which in turn is optionally substituted by hydroxy,
R² is phenyl which may be substituted once or twice, identically or differently, by substituents selected from the group of fluorine, chlorine, cyano, (C₁-C₄)-alkyl, trifluoromethyl, formyl, acetyl, (C₁-C₄)-alkoxy, hydroxy, acetoxy, pivaloyloxy, amino, formylamino, acetylamino and methylsulfonylamino,
where (C₁-C₄)-alkyl and (C₁-C₄)-alkoxy in turn may each be substituted by hydroxy, amino, methoxy, ethoxy or acetylamino,
or
R² is pyrrolyl, pyridyl or pyrimidinyl, each of which may be substituted by amino, fluorine, chlorine, methyl, ethyl, methoxy or ethoxy,
and
R³ is hydrogen, fluorine, chlorine, bromine, methyl, ethyl, trifluoromethyl, nitro or cyano,
and the salts, solvates and solvates of the salts thereof.

3. Compounds of the formula (I) according to Claim 1,
in which
R¹ is imidazolyl which is attached via a ring nitrogen atom or is piperazinyl which is attached via a ring nitrogen atom and which may be substituted on the second ring nitrogen atom by methyl, ethyl, 2-hydroxyethyl, 2-methoxyethyl, acetyl, tert-butoxycarbonyl or methylsulfonyl,
R² is phenyl which may be substituted by fluorine or hydroxy in position 4 relative to the linkage point on the phenyl ring,
and
R³ is located in position 4 relative to the linkage point of the pyridazinone ring and is hydrogen, fluorine, chlorine, methyl or trifluoromethyl,
and the salts, solvates and solvates of the salts thereof.

4. Compound of the formula (I) according to Claim 1 with the following structures: 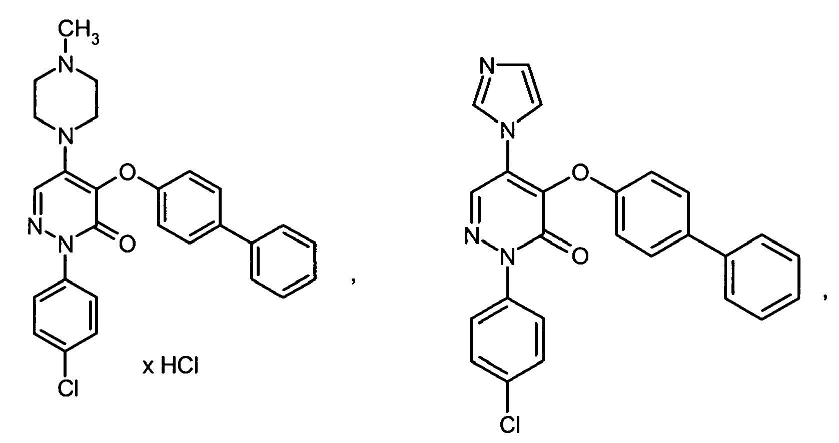 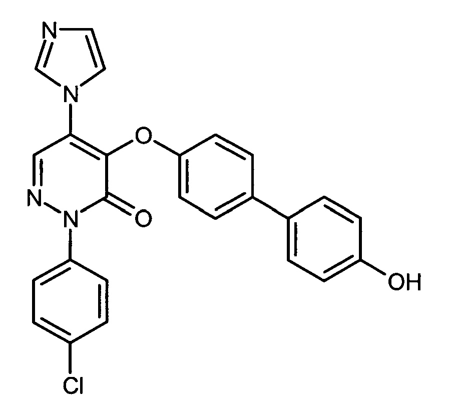 and the salts, solvates and solvates of the salts thereof.

5. Process for preparing the compounds of the formula (I) as defined in Claim 1, **characterized in that** first compounds of the formula (II) 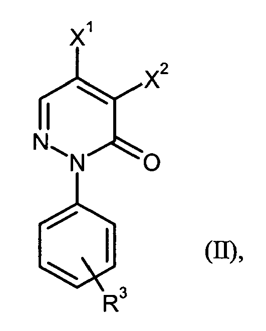 in which
R³ has the meaning indicated in claim 1, and
X¹ and X² are each halogen, preferably bromine or chlorine,
are converted with a compound of the formula (III)
R¹-H (III),
in which R¹ has the meaning indicated in claim 1,
into compounds of the formula (IV) 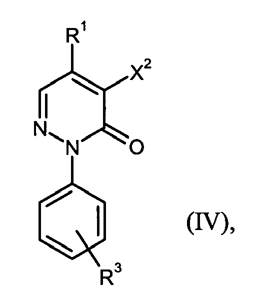 in which R¹, R³ and X² each have the meaning indicated above,
and the latter are then reacted with a compound of the formula (V) 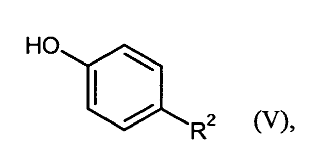 in which R² has the meaning indicated in claim 1.

6. Compounds of the formula (I) as defined in Claim 1 for the prophylaxis and/or treatment of disorders.

7. Medicament comprising at least one compound of the formula (I) as defined in Claim 1, and at least one further excipient.

8. Medicament comprising at least one compound of the formula (I) as defined in Claim 1, and at least one further active ingredient.

9. Use of compounds of the formula (I) as defined in Claim 1 for producing medicaments for the prophylaxis and/or treatment of fibrotic disorders.

## Revendications

1. Composés de formule (I) 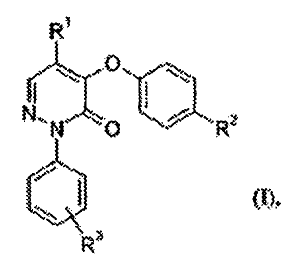 dans laquelle
R¹ représente un reste hétérocyclyle de 5 à 7 chaînons, saturé ou partiellement insaturé, lié par l'intermédiaire d'un atome d'azote du noyau, ayant éventuellement un autre hétéroatome ou hétérogroupe de la série N, O, S, SO ou SO₂, qui peut être substitué une ou deux fois identiques ou différentes par des substituants choisis dans le groupe halogène, alkyle en C₁ à C₆, alcényle en C₂ à C₆, cycloalkyle en C₃ à C₈, hydroxy, oxo, carboxyle, (alkoxy en C₁ à C₆)carbonyle, alcanoyle en C₁ à C₆, (cycloalkyle en C₃ à C₈) carbonyle, alkylsulfonyle en C₁ à C₆, aminocarbonyle, 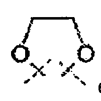 et (alkyle en C₁ à C₆) aminocarbonyle, les substituants alkyle en C₁ à C₆ et alcanoyle en C₁ à C₆ pouvant eux-mêmes être substitués chacun par un radical halogéno, hydroxy, alkoxy en C₁ à C₄, (alkoxy en C₁ à C₄)carbonyle, amino, mono- ou di- (alkyle en C₁ à C₄)amino, (alkoxy en C₁ à C₄)carbonylamino ou hétérocyclyle pentagonal ou hexagonal ayant jusqu'à deux hétéroatomes de la série O, N et/ou S,
ou bien
R¹ représente un reste hétéroaryle pentagonal, lié par l'intermédiaire d'un atome d'azote du noyau, qui peut être substitué une à trois fois identiques ou différentes par un radical halogéno, (alkoxy en C₁ à C₆) carbonyle ou alkyle en C₁ à C₆ qui est lui-même éventuellement substitué par un radical hydroxy ou halogéno,
R² est un radical aryle en C₆ à C₁₀ qui peut être substitué une ou deux fois identiques ou différentes par des substituants choisis dans le groupe halogéno, nitro, cyano, alkyle en C₁ à C₆, trifluorométhyle, alcanoyle en C₁ à C₆, alkoxy en C₁ à C₆, hydroxy, acyloxy en C₁ à C₆, amino, acylamino en C₁ à C₆, mono- ou di-[(alkyle en C₁ à C₆)sulfonyl]amino,
les substituants alkyle en C₁ à C₆ et alkoxy en C₁ à C₆ pouvant eux-mêmes être substitués par un radical hydroxy, amino, alkoxy en C₁ à C₄ ou acylamino en C₁ à C₄,
ou bien
R² représente un reste hétéroaryle pentagonal ou hexagonal ayant jusqu'à deux atomes d'azote dans le noyau, qui peut être substitué par un radicval amino, hydroxy, halogéno, alkyle en C₁ à C₆ ou alkoxy en C₁ à C₆,
et
R³ représente l'hydrogène, un halogène, un reste alkyle en C₁ à C₆, trifluorométhyle, nitro, cyano, carboxyle ou (alkoxy en C₁ à C₆) carbonyle,
et leurs sels, leurs produits de solvatation et les produits de solvatation des sels.

2. Composés de formule (I) suivant la revendication 1,
formule dans laquelle
R¹ est un groupe de formule 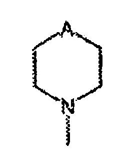
où
A représente CR⁴R⁵, O, S, NR⁶ ou -CH₂NR⁶-,
R⁴ et R⁵ représentent indépendamment l'un de l'autre l'hydrogène, un reste alkyle en C₁ à C₄ qui peut être substitué par un radical hydroxy, un reste hydroxy, le fluor, un reste carboxyle ou (alkoxy en C₁ à C₄) carbonyle, ou forment un groupe carbonyle conjointement avec l'atome de carbone auquel ils sont liés,
et
R⁶ représente l'hydrogène, un reste alcényle en C₂ à C₄, cycloalkyle en C₃ à C₆, (alkoxy en C₁ à C₄)carbonyle, formyle, acétyle, (cycloalkyle en C₃ à C₆) carbonyle, alkylsulfonyle en C₁ à C₄, aminocarbonyle, (alkyle en C₁ à C₄)aminocarbonyle, ou un reste alkyle en C₁ à C₄ qui peut lui-même être substitué par un radical hydroxy, méthoxy, éthoxy, (alkoxy en C₁ à C₄)carbonyle, amino, diméthylamino, diéthylamino, pyrrolidino, pipéridino ou morpholino,
ou bien
R¹ représente un reste hétéroaryle pentagonal, lié par l'intermédiaire d'un atome d'azote du noyau,
ayant jusqu'à deux autres atomes d'azote dans le noyau, qui peut être substitué une ou deux fois identiques ou différentes par du fluor, du chlore, un radical (alkoxy en C₁ à C₄) carbonyle ou un radical alkyle en C₁ à C₄ qui peut lui-même éventuellement être substitué par un radical hydroxy,
R² représente un reste phényle qui peut être substitué une ou deux fois identiques ou différentes par des substituants choisis dans le groupe fluor, chlore, cyano, alkyle en C₁ à C₄, trifluorométhyle, formyle, acétyle, alkoxy en C₁ à C₄, hydroxy, acétoxy, pivaloyloxy, amino, formylamino, acétylamino et méthylsulfonylamino,
les substituants alkyle en C₁ à C₄ et alkoxy en C₁ à C₄ pouvant eux-mêmes être substitués par un radical hydroxy, amino, méthoxy, éthoxy ou acétylamino,
ou bien
R² représente un reste pyrrolyle, pyridyle ou pyrimidinyle, chacun pouvant être substitué par un radical amino, fluoro, chloro, méthyle, éthyle, méthoxy ou éthoxy
et
R³ représente l'hydrogène, le fluor, le chlore, le brome, un reste méthyle, éthyle, trifluorométhyle, nitro ou cyano,
et leurs sels, leurs produits de solvatation et les produits de solvatation des sels.

3. Composés de formule (I) suivant la revendication 1,
formule dans laquelle
R¹ représente un reste imidazolyle lié par l'intermédiaire d'un atome d'azote du noyau ou un reste pipérazinyle lié par l'intermédiaire d'un atome d'azote du noyau, qui peut être substitué sur le second atome d'azote du noyau par un radical méthyle, éthyle, 2-hydroxyéthyle, 2-méthoxyéthyle, acétyle, tertiobutoxycarbonyle ou méthylsulfonyle,
R² représente un reste phényle qui peut être substitué en position 4 par rapport au site de rattachement au noyau phényle par un radical fluoro ou hydroxy,
et
R³ se trouve en position 4 par rapport au site de rattachement du noyau pyridazinone et représente l'hydrogène, le fluor, le chlore, un reste méthyle ou trifluorométhyle
et leurs sels, leurs produits de solvatation et les produits de solvatation des sels.

4. Composés de formule (I) suivant la revendication 1, ayant les structures suivantes : 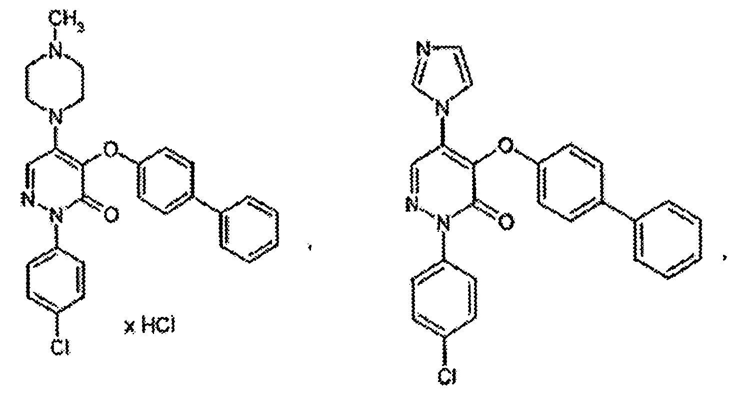 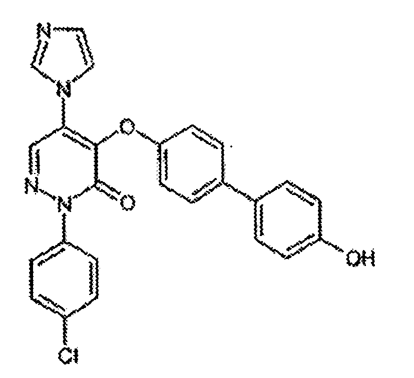 et leurs sels, leurs produits de solvatation et les produits de solvatation des sels.

5. Procédé de production des composés de formule (I) telle que définie dans la revendication 1, **caractérisé en ce qu'**on transforme tout d'abord des composés de formule (II) 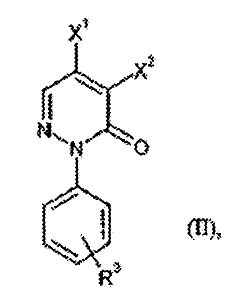 dans laquelle
R³ a la définition indiquée dans la revendication 1 et
x¹ et x² représentent chacun un halogène, avantageusement le brome ou le chlore,
par réaction avec un composé de formule (III)
R¹-H (III)
dans laquelle
R¹ a la définition indiquée dans la revendication 1 en composés de formule (IV) 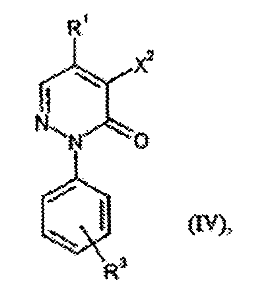 dans laquelle
R¹, R³ et X² ont chacun la définition indiquée ci-dessus,
qu'on fait ensuite réagir avec un composé de formule (V) 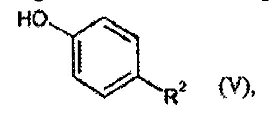 dans laquelle
R² a la définition indiquée dans la revendication 1.

6. Composés de formule (I) telle que définie dans la revendication 1, destinés à la prophylaxie et/ou au traitement de maladies.

7. Médicament contenant au moins un composé de formule (I) telle que définie dans la revendication 1, et au moins une autre substance auxiliaire.

8. Médicament contenant au moins un composé de formule (I) telle que définie dans la revendication 1 et au moins une autre substance active.

9. Utilisation de composés de formule (I) telle que définie dans la revendication 1 pour la préparation de médicaments destinés à la prophylaxie et/ou au traitement de fibroses.
